# EUROPEAN PATENT SPECIFICATION

(11) **EP 1 737 958 B1**
(45) Date of publication and mention of the grant of the patent: **29.10.2008**
(21) Application number: 05744134.7
(22) Date of filing: 25.03.2005
(51) Int. Cl.: C12N 15/12, C12N 15/13, C07K 14/705, C07K 16/28, A61K 39/00, A61P 37/00

(54) **COMPOSITIONS AND METHODS FOR INDUCING ANTI-TUMOR IMMUNITY**
ZUSAMMENSETZUNGEN UND VERFAHREN ZUR INDUKTION EINER ANTITUMORIMMUNITÄT
COMPOSITIONS ET TECHNIQUES INDUISANT UNE IMMUNITE ANTITUMORALE

(30) Priority: 26.03.2004 US 556633 P
(43) Date of publication of application: 03.01.2007
(73) Proprietor: Washington Research Foundation, Seattle, WA 98102 (US)
(72) Inventor: HELLSTROM, Karl Erik, Seattle, WA 98105 (US); HELLSTROM, Ingegerd, Seattle, WA 98105 (US); YANG, Yi, Lynnwood, WA 98036 (US)
(74) Representative: Naylor, Kathryn May
(86) International application number: PCT/US2005/010195
(87) International publication number: WO 2005/097997

(56) References cited:
- US-A1- 2003 219 436
- SCHOLLER N ET AL: "CUTTING EDGE: CD83 REGULATES THE DEVELOPMENT OF CELLULAR IMMUNITY" JOURNAL OF IMMUNOLOGY, THE WILLIAMS AND WILKINS CO. BALTIMORE, US, vol. 168, no. 6, 15 March 2002 (2002-03-15), pages 2599-2602, XP001134923 ISSN: 0022-1767 cited in the application
- SCHOLLER N ET AL: "CD83 REGULATES THE DEVELOPMENT OF CYTOTOXIC LYMPHOCYTES" FASEB JOURNAL (FEDERATION OF AMERICAN SOCIETIES FOR EXPERIMENTAL BIOLOGY), BETHESDA, US, vol. 16, no. 4, 20 March 2002 (2002-03-20), pages A710-A711, XP008012706 ISSN: 0892-6638
- HELLSTROM K E ET AL: "THERAPEUTIC VACCINATION WITH TUMOR CELLS THAT ENGAGE CD137" JOURNAL OF MOLECULAR MEDICINE, SPRINGER VERLAG, DE, vol. 81, no. 2, February 2003 (2003-02), pages 71-86, XP008036323 ISSN: 0946-2716
- MELERO I ET AL: "Monoclonal antibodies against the 4-1BB T-cell activation molecule eradicate established tumors" NATURE MEDICINE, NATURE PUBLISHING, CO, US, vol. 3, no. 6, June 1997 (1997-06), pages 682-685, XP002104261 ISSN: 1078-8956 cited in the application
- YE ZHENGMAO ET AL: "Gene therapy for cancer using single-chain Fv fragments specific for 4-1BB" NATURE MEDICINE, NATURE PUBLISHING, CO, US, vol. 8, no. 4, April 2002 (2002-04), pages 343-348, XP002316896 ISSN: 1078-8956 cited in the application
- YANG SHILIN ET AL: "Melanoma cells transfected to express CD83 induce antitumor immunity that can be increased by also engaging CD137." PROCEEDINGS OF THE NATIONAL ACADEMY OF SCIENCES OF THE UNITED STATES OF AMERICA. 6 APR 2004, vol. 101, no. 14, 6 April 2004 (2004-04-06), pages 4990-4995, XP002342740 ISSN: 0027-8424

## Description

### TECHNICAL FIELD

This invention relates to compositions containing tumor cells expressing a cell surface CD83 polypeptide for inducing anti-tumor immunity. The compositions of the invention also include a cell surface expressed antibody that binds specifically to an immune receptor such as CD137. The invention also relates to recombinant expression constructs that express a cell surface CD83 polypeptide and/or a cell surface expressed antibody that binds specifically to an immune receptor, and to constructs that express at least one tumor antigen.

### BACKGROUND OF THE INVENTION

Cancer includes a broad range of diseases, affecting approximately one in four individuals worldwide. The severity of the adverse impact of cancer cannot be understated, influencing medical policy and procedure as well as society generally. As known in the immunological arts, certain compositions, according to a variety of formulations, may be usefully employed for the purpose of inducing a desired immune response in a host. The immune system has been characterized as distinguishing foreign (heterologous or "non-self') agents from familiar (autologous or "self") components, such that foreign agents are recognized as such and elicit immune responses while "self" components are ignored or tolerated. Immune responses have traditionally been characterized as either humoral responses, in which antibodies specific for antigens are produced by differentiated B lymphocytes known as plasma cells, or cell mediated responses, in which various types ofT lymphocytes act to eliminate antigens by a number of mechanisms. For example, CD4+ helper T cells that are capable of recognizing specific antigens may respond by releasing soluble mediators such as cytokines to recruit additional cells of the immune system to participate in an immune response. Also, CD8+ cytotoxic T cells that are also capable of specific antigen recognition may respond by binding to and destroying or damaging an antigen-bearing cell or particle.

Several strategies for eliciting specific immune responses through the administration of a composition to a host include (1) immunization with heat-killed or live, attenuated infectious pathogens such as viruses, bacteria, or certain eukaryotic pathogens; (2) immunization with a non-virulent infective agent capable of directing the expression of genetic material encoding the antigen(s) to which an immune response is desired; and (3) immunization with subunit vaccines that contain isolated immunogens (such as proteins) from a particular pathogen in order to induce immunity against the pathogen (*see, e.g.,* Liu, Nature Medicine 4(5 suppl.):515 (1998)). Each of these approaches is compromised by certain trade-offs between safety and efficacy. Moreover, certain types of desirable immunity for which none of these approaches has been particularly effective include the development of compositions effective in protecting a host immunologically against cancer, autoimmune disease, human immunodeficiency viruses, or other clinical conditions *(see, e.g.,* Hahne et al., Science 274:1363-66 (1996); Dong et al., Nat. Med. 8:793 -300 (2002)).

CD83 is a marker of activated human dendritic cells (DC) (Banchereau et al., Ann. Rev. Immunol. 18:767-811 (2000)). In humans, its ligands are primarily expressed on resting monocytes and a subpopulation of activated T cells (Scholler et al., J. Immunol. 166:3865-72 (2001)). Transfection of cells from the human B cell line T51 to express surface CD83 increases their ability to stimulate proliferation of allogeneic peripheral blood mononuclear cells, including CD8+ cytotoxic T lymphocytes (CTL). This effect is abolished in the presence of a soluble CD83 fusion protein with an immunoglobulin "tail" (Scholler et al., J. Immunol. 168:2599-602 (2002)).

In mice, the ligands for CD83 are primarily expressed on B cells (Cramer et al., Int. Immunol. 12:1347-51 (2000); *see* Fujimoto et al., Cell 108:755-67 (2002)), and soluble fusion proteins that express the extracellular part of mouse CD83 inhibit antigen-specific T cell proliferation and IL-2 secretion in cultures ofmouse spleen (*id.*)*.* Cells from the M2 clone of a mouse melanoma K1735 cell line that were transfected to express human CD83 were rejected by syngeneic mice, some of which also rejected a subsequent transplant of non-transfected wild type (WT) cells from the M2 clone (U.S. Patent Application No. 2003/0219436 and Scholler et al., J. Immunol. 168:2599.602 (2002)); (*see also* Estin et al., J. Natl. Cancer Inst. 81:445-48 (1989)). In contrast, injection of a soluble CD83-mouse immunoglobulin fusion protein facilitated the outgrowth of transplanted cells from the P815 mastocytoma and caused a decrease in a tumor-directed CTL response (Scholler et al., J. Immunol. 168:2599-2602 (2002)). The molecular form, cellular context, and mode of presentation of CD83 to the immune system thus appear to have significance to immunotherapy, but specific and effective modalities for beneficially employing CD83 remain elusive.

Hellstrom et al. J. Mol Med (2003) 81:71-86 describes an approach to transfect tumour cells 3 to express the CD137 ligand.

Thus, while a number of human tumor associated antigens have been defined and structurally characterized, a need persists for an increased understanding of the molecular pathways that are required for induction and maintenance of an immune response, and the clinical response of cancer patients when immunization with such tumor antigens has been attempted has often been disappointing. Accordingly, a need exists to develop compositions that are directed to inducing immune responses to tumor cells. The present invention provides compositions and methods for inducing anti-tumor cell immunity, as well as other related advantages.

### SUMMARY OF THE INVENTION

The invention provides methods and compositions for inducing anti-tumor immunity in a host. In one embodiment, the invention is directed to a composition for inducing anti-tumor immunity comprising a tumor cell that expresses (i) a cell surface CD83 polypeptide,

or a portion thereof; and (ii) a cell surface form of an antibody, or antigen-binding fragment thereof, that binds specifically to CD137. In a certain embodiment, the tumor cell is transfected with (i) a recombinant expression construct comprising at least one first promoter operatively linked to a polynucleotide encoding a cell surface CD83 polypeptide and at least one second promoter operatively linked to a polynucleotide that encodes a cell surface form of an antibody, or antigen-binding fragment thereof, that binds specifically to a CD 137 polypeptide or the tumor cell is transfected with (ii) a first recombinant expression construct that comprises at least one first promoter operatively linked to a polynucleotide encoding a cell surface CD83 polypeptide and a second recombinant expression construct comprising at least one second promoter operatively linked to a polynucleotide encoding a cell surface form of an antibody, or antigen-binding fragment thereof, that binds specifically to a CD137 polypeptide.

In another embodiment a composition for inducing anti-tumor immunity comprises a first tumor cell that expresses (i) a cell surface CD83 polypeptide, or a portion thereof; and (ii) a second tumor cell that expresses a cell surface form of an antibody, or antigen-binding fragment thereof, that binds specifically to CD137. In a particular embodiment, the first tumor cell is transfected with a first recombinant expression construct comprising at least one first promoter operatively linked to a polynucleotide encoding a cell surface CD83 polypeptide, and the second tumor cell is transfected with a second recombinant expression construct comprising at least one second promoter operatively linked to a polynucleotide encoding a cell surface form of an antibody, or antigen-binding fragment thereof, that binds specifically to a CD137 polypeptide.

In one embodiment, the invention provides a composition for inducing anti-tumor immunity comprising (i) a recombinant expression construct comprising at least one first promoter operatively linked to a polynucleotide encoding a cell surface CD83 polypeptide and at least one second promoter operatively linked to a polynucleotide that encodes a cell surface form of an antibody, or antigen-binding fragment thereof, that binds specifically to a CD137 polypeptide or (ii) a first recombinant expression construct that comprises at least one first promoter operatively linked to a polynucleotide encoding a cell surface CD83 polypeptide and a second recombinant expression construct comprising at least one second promoter operatively linked to a polynucleotide encoding a cell surface form of an antibody, or antigen-binding fragment thereof, that binds specifically to a CD 137 polypeptide. In another embodiment, the composition comprises a recombinant expression vector comprises at least one promoter operatively linked to a polynucleotide sequence encoding at least one tumor antigen. In a certain embodiment the tumor antigen is expressed by a tumor cell in a biological sample that is obtained from a subject having a malignant condition.

The invention also provides a composition for inducing anti-tumor immunity comprising (i) a first recombinant expression construct that comprises at least one promoter operatively linked to a polynucleotide encoding a cell surface CD83 polypeptide, or a portion thereof, and (ii) a second recombinant expression construct comprising at least one second promoter operatively linked to a polynucleotide encoding at least one tumor antigen. In a certain embodiment the tumor antigen is expressed by a tumor cell in a biological sample that is obtained from a subject having a malignant condition.

In a particular embodiment of the invention, the antibody that specifically binds to CD137 of the above described compositions is a single chain Fv antibody. In certain other embodiments, the antigen-binding fragment that specifically binds to CD137 is an Fab, an Fab', an (Fab')₂, and an Fv.

The invention also provides a method for inducing anti-tumor immunity in a subject comprising administering to a subject any one of the aforementioned compositions and related embodiments and a pharmaceutically acceptable carrier.

In one embodiment, the invention provides a composition for inducing anti-tumor immunity comprising at least one composition comprising (i) a first tumor cell that expresses a cell surface CD83 polypeptide, or a portion thereof; (ii) a second tumor cell that expresses on its surface an antibody, or an antigen-binding fragment thereof, that specifically binds to a CD137 polypeptide; and (iii) a pharmaceutically acceptable carrier. In one embodiment, the first and second tumor cells are obtained from a biological sample obtained from the host. In a certain embodiment, the ability of each of the first and second tumor cells to divide in the host is inhibited or substantially impaired. In a particular embodiment, the antibody that specifically binds to CD137 is a single chain Fv antibody, and in certain other embodiments, the antigen-binding fragment that specifically binds to CD137 is an Fab, an Fab', an (Fab')₂, or an Fv.

The invention also provides the use of a composition in the manufacture of a medicament for treating a subject having a malignant condition comprising (a) isolating from the subject at least one tumor cell; (b) introducing into the tumor cell a composition for inducing anti-tumor immunity; (c) inhibiting or substantially impairing an ability of the tumor cell to divide to obtain a modified tumor cell; and (d) administering to the subject the modified tumor cell and a pharmaceutically acceptable carrier, thereby inducing or enhancing an immune response to the malignant condition in the subject. In certain embodiments, the malignant condition is melanoma, carcinoma, sarcoma, lymphoma, or leukemia. In one embodiment, the composition for inducing anti-tumor immunity comprises a recombinant expression construct comprising at least one first promoter operatively linked to a polynucleotide encoding a cell surface CD83 polypeptide and at least one second promoter operatively linked to a polynucleotide that encodes a cell surface form of an antibody, or antigen-binding fragment thereof, that binds specifically to a CD137 polypeptide or (ii) a first recombinant expression construct that comprises at least one first promoter operatively linked to a polynucleotide encoding a cell surface CD83 polypeptide and a second recombinant expression construct comprising at least one second promoter operatively linked to a polynucleotide encoding a cell surface form of an antibody, or antigen-binding fragment thereof, that binds specifically to a CD137 polypeptide, or a portion thereof. In another embodiment, the composition comprises a recombinant expression vector comprises at least one promoter operatively linked to a polynucleotide sequence encoding at least one tumor antigen. In another embodiment, the composition comprises a first recombinant expression construct that comprises at least one promoter operatively linked to a polynucleotide encoding a cell surface CD83 polypeptide, sequence, or a portion thereof, and (ii) a second recombinant expression construct comprising at least one second promoter operatively linked to a polynucleotide encoding at least one tumor antigen. In another embodiment, the method comprises an anti-immunosuppression step. In a particular embodiment, the anti-immunosuppression step comprises administering to the subject an agent that inhibits an immunosuppressive effect of an immunosuppressive molecule selected from the group consisting of a TGF-beta polypeptide, a CTLA4 polypeptide, a glucocorticoid-induced tumor necrosis factor receptor, and Fas ligand. In another embodiment the agent is a chemotherapeutic agent, an antibody or antigen-binding fragment thereof that specifically binds to a TGF-beta polypeptide, an antibody or antigen-binding fragment thereof that specifically binds to a CTLA4 polypeptide, or an antibody or antigen-binding fragment thereof that specifically binds to a glucocorticoid-induced tumor necrosis factor receptor.

In another embodiment, the invention provides a recombinant expression construct that comprises at least one first promoter operatively linked to a polynucleotide encoding a cell surface CD83 polypeptide, and at least one second promoter operatively linked to a polynucleotide that encodes a cell surface form of an antibody or antigen-binding fragment thereof that binds specifically to a CD137 polypeptide. In a certain embodiment, the antibody is a single chain Fv antibody, and in another embodiment, the antigen-binding fragment is an Fab, an Fab', an (Fab')₂, or an Fv. The invention also provides a host cell comprising the recombinant expression construct.

These and other embodiments of the present invention will become apparent upon reference to the following detailed description and attached drawings.

### BRIEF DESCRIPTION OF DRAWINGS

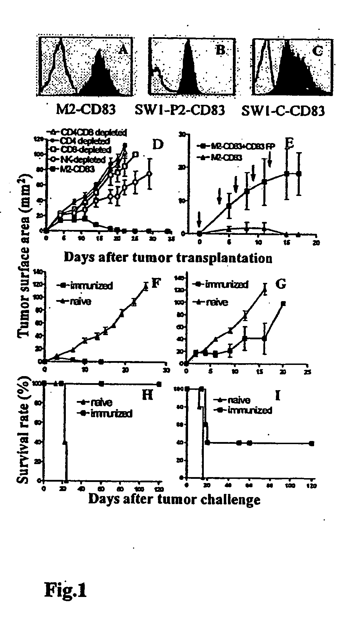

Figure 1A-1C present flow cytometry data showing expression of CD83 on surfaces of transfected (shaded) versus wild type (open area) cells. Figure 1D shows the growth of M2-CD83 cells in mice depleted of the indicated cell populations. Figure 1E shows the outgrowth of M2-CD83 cells in mice injected intraperitoneally with mCD83 fusion protein (CD83 FP), 100 µg/mouse, at the time of tumor transplantation and repeated as indicated; the control group was injected with PBS. Figure 1F and 1H show regression of M2-WT cells, and Figure 1G and 1I show regression of Ag104 cells in mice immunized by live M2-CD83 cells (5 mice/group).

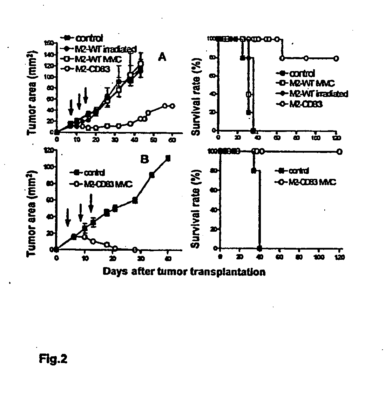

Figure 2 illustrates the therapeutic efficacy against subcutaneously established M2-WT tumors by immunization with live (Figure 2A) or MMC-treated (Figure 2B) M2-CD83 cells as indicated. The left part of Figure 2A and Figure 2B indicates tumor growth and the right part of each panel illustrates survival (5 mice/group).

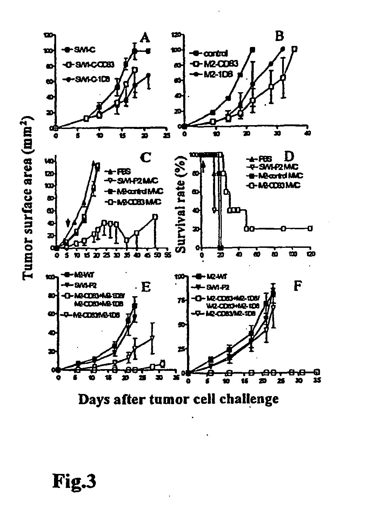

Figure 3 demonstrates tumor formation by SW1-C-CD83, SW1-C-1 D8, and SW1-C cells transplanted into naïve mice (10⁶ cells/mouse) (Figure 3A). Figure 3B illustrates tumor formation by SW1-C cells in mice (10⁶ cells/mouse) that had been immunized four times with MMC-treated M2-CD83 or M2-1D8 cells (2x10⁶ cells/mouse). Figure 3C indicates tumor formation by SW1-P2 cells (1 0⁶/mouse) transplanted into mice, and Figure 3D shows survival of mice that had been immunized 3 times, 7 days apart, with MMC-treated M2-CD83 cells (2×10⁶/mouse), and re-imrnunized as indicated by the arrow. Inhibited outgrowth of SW1-C and SW1-P2 cells is shown in Figure 3E and Figure 3F, respectively, in mice immunized twice, one week apart, by a mixture of M2-CD83+M2-1D8 cells (10⁶ cells of each cell type/mouse) (5 mice/group).

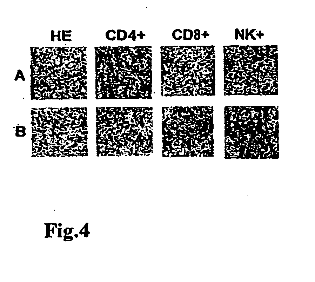

Figure 4 presents immunostained sections of M2-WT (Figure 4A) and M2-CD83 tumors (Figure 4B) harvested 6 days after transplantation into naive mice. Original magnification is 64x. Cells were counterstained with HE (hematoxylin and eosin). CD4+ cells were detected with an anti-mouse CD4 monoclonal antibody GK1.5; CD8+ cells were detected with anti-mouse CD8 monoclonal antibody 53-6.7; and NK cells were detected with rabbit anti-mouse NK antisera.

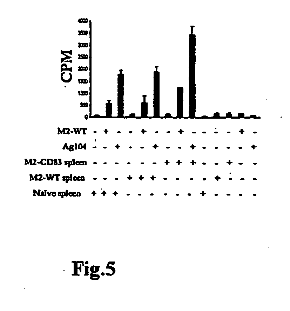

Figure 5 illustrates proliferation of splenocytes from mice that were either naive or immunized 3 times, 7 days apart, with MMC-treated M2-WT or M2-CD83 cells (2×10⁶ cells/mouse). The spleen cells were co-cultured with MMC-treated M2-WT or Ag104-WT cells for 3 days.

### DETAILED DESCRIPTION OF THE INVENTION

The invention relates to the unexpected discovery that rejection of tumor cells in a host may be enhanced or accelerated by administering tumor cells that express a cell surface CD83 polypeptide in combination with tumor cells that express a cell surface antibody, or antigen-binding fragment thereof, that binds specifically to CD137. The present invention is directed to compositions and methods for inducing anti-tumor immunity related to the use of a CD83 polypeptide and an antibody that binds specifically to an immune cell receptor expressed on a cell surface.

The invention relates to the observation that a tumor cell that expresses a cell surface form of a CD83 polypeptide can induce tum or immunity in a syngeneic host, such as a mammalian subject. For example, and as described in detail herein, when a murine CD83 polypeptide was expressed on the cell surface of an M2 tumor cell, the tumor cells were rejected more rapidly and in all recipient mice, compared to animals that were immunized with a cell that expressed a xenogeneic (human) CD83 polypeptide.

Certain tumor cell lines may exhibit low immunogenicity when transplanted into certain hosts (that is, are less likely to be rejected by the host), for example, the cell lines designated SW1-C or SW1-P2, as known in the art and described herein. Surprisingly, however, outgrowth of both SW1-C and SW1-P2 could be delayed in mice that were immunized with a distinct tumor (mitomycin C (MMC)-treated cell line M2) that had been genetically altered to express either a cell surface CD83 polypeptide or a cell surface expressed anti-CD 137 scFv. Moreover, tumor cell outgrowth could be prevented in most mice that were concomitantly immunized with a first population of tumor cells genetically modified to express a cell surface CD83 polypeptide *and* with a second population of tumor cells genetically modified to express an anti-CD137 antibody on the cell surface. Without wishing to be bound by theory, the increased apparent immunotherapeutic effectiveness observed following concomitant administration of both tumor cell populations, may be the result of distinct mechanisms of immune system engagement by each genetically altered tumor cell population. The increased immunotherapeutic effectiveness may be additive, or the immunotherapeutic effectiveness may be synergistic, providing an induced immune response that results in more (e.g., increased in a statistically significant manner) than the sum of the immunotherapeutic effectiveness induced by a cell surface CD83 or by a cell surface anti-CD137 antibody when each is administered alone.

Compositions of the present invention according to certain embodiments may include a tumor cell that comprises a cell surface form of a CD83 polypeptide, or a portion thereof. In certain embodiments, the composition relates to a tumor cell that expresses a cell surface form of a CD83 polypeptide and a cell surface form of an antibody that specifically binds to a receptor on immune cells, and in certain embodiments the CD83 polypeptide and the cell surface antibody may be expressed on different tumor cells. In certain preferred embodiments, the immune cell receptor to which the antibody specifically binds is CD137 (4-1BB). The compositions of the present invention can be used as immunotherapeutics for inducing tumor immunity in a subject having a malignant condition.

In one embodiment of the invention, a composition for inducing anti-tumor immunity comprises a tumor cell that expresses a cell surface CD83 polypeptide, or a portion thereof. In a certain embodiment, the invention provides a composition for inducing anti-tumor immunity comprising a tumor cell that expresses a cell surface CD83 polypeptide and a cell surface form of an antibody or antigen-binding fragment thereof that binds specifically to an immune cell receptor such as CD137. In one embodiment of the invention, both the CD83 polypeptide and the anti-CD137 antibody are expressed on the cell surface of the same tumor cell. In another embodiment, the CD83 polypeptide and the anti-CD137 antibody are each expressed on a separate cell (that is, for example, a CD83 polypeptide is expressed on a first tumor cell and the anti-CD137 antibody is expressed on a second tumor cell).

According to non-limiting theory, inducing an immune response by presenting to a host immune system (i) at least one tumor antigen (or tumor-associated antigen), for example, a tumor antigen (or tumor-associated antigen) expressed on or in a tumor cell obtained from a subject, or alternatively, an exogenously derived tumor antigen (or tumor-associated antigen) that is introduced into a cell or a host according to methods known in the art and described herein either as an isolated polypeptide or as the product of a recombinant expression construct that is delivered to a cell or host, in combination with (ii) a cell surface CD83 polypeptide, may stimulate or enhance the immune response in a manner that would not be accomplished by presentation of the tumor antigen alone. According to non-limiting theory, an immune response to tumor antigens (or tumor-associated antigens) induced according to the invention described herein, may be stimulated by interaction between CD83 and its ligand(s) on B cells, and may also include stimulation of monocytes that express a CD83 ligand. In the context of such CD83-mediated immunostimulation, a tumor antigen (or tumor-associated antigen) provided on a tumor cell, as an isolated polypeptide, and/or expressed from a recombinant expression construct introduced into a cell, may be processed according to currently understood mechanisms of processing and presentation by Major Histocompatibility Complex (MHC) molecules. One or more of these peptides may, for example, stimulate T helper lymphocytes that control antibody secreting cells and the generation of cytotoxic T lymphocytes. Such induction of an immune response may be further enhanced or stimulated by additionally administering at least one cell surface expressed anti-immune receptor antibody, such as an anti-CD137 antibody (for example, a single chain Fv fused to a transmembrane domain and a cytoplasmic domain), subsequently or simultaneously, and in any order. Such triggering via an additional immune receptor may further stimulate an ongoing antigen-specific immune response mechanism or may stimulate or enhance an immune response by a mechanism that involves a different subset of immune cells.

A tumor cell may be isolated or obtained from a biological sample from a biological source or a subject. A biological sample may be provided by obtaining a blood sample, biopsy specimen, tissue explant, organ culture, biological fluid or any other tissue or cell preparation from a subject or a biological source. A biological fluid that is a biological sample may include blood, serum, serosal fluid, plasma, lymph, urine, cerebrospinal fluid, saliva, a mucosal secretion, a vaginal secretion, ascites fluid, pleural fluid, pericardial fluid, peritoneal fluid, abdominal fluid, culture medium, conditioned culture medium or lavage fluid. The subject or biological source may be a human or non-human animal, a primary cell culture or culture adapted cell line including but not limited to genetically engineered cell lines that may contain chromosomally integrated or episomal recombinant nucleic acid sequences, immortalized or immortalizable cell lines, somatic cell hybrid cell lines, differentiated or differentiatable cell lines, transformed cell lines, and the like. In certain preferred embodiments of the invention, the subject or biological source may be suspected of having or being at risk for having a malignant condition as provided herein, and in certain other embodiments the subj ect or biological source may be known to be free of a risk or presence of such disease.

Tumor cells that are to be transfected to express a cell surface CD83 polypeptide and/or an anti-immune cell receptor antibody for use in the present invention may be obtained from a subject or biological source having a malignant condition, and to whom the transfected tumor cells can be administered to induce an anti-tumor immune response (*i.e.,* delivery of autologous tumor cells). Alternatively, tumor cells may be isolated from another subject or biological source having a malignant condition and which tumor cells express one or more of the same tumor antigens (that is the tumor cells are antigenically related) as are expressed on the tumor cells from the subject to be treated (*i.e.,* delivery of allogeneic tumor cells). Transfection and delivery of allogeneic tumor cells may be useful when autologous tumor cells express one or more factors such as Fas ligand that can suppress an immune response. In certain embodiments, a tumor cell from a subject having one type of malignant condition may be used in the compositions as described herein to induce an anti-tumor immune response in a subject having the same malignant condition, and in certain other embodiments, the immune response is induced in a subject having a different type of malignant condition. By way of example, a sarcoma tumor cell that expresses one or more tumor antigens or tumor associated antigens that are also expressed on a carcinoma tumor cell, ma.y be transfected to express a cell surface CD83 polypeptide and an immune cell receptor antibody. Such a doubly transfected cell may then be delivered to a subject having a carcinoma to induce anti-tumor immunity in the recipient.

The present invention provides compositions and methods for immunotherapy of a malignant condition. A malignant condition such as cancer includes carcinomas, and thus may include but need not be limited to melanoma, mesothelioma, ovarian carcinoma, pancreatic carcinoma, and non-small cell lung carcinoma, breast, stomach, or colon carcinoma, or another form of cancer, including any of the various carcinomas such as squamous cell carcinomas and adenocarcinomas, and also including sarcomas and hematologic malignancies (*e.g.,* leukemias, lymphomas, myelomas, etc.). Classification of these and other malignant conditions is known to those having familiarity with the art. A subject or host having a malignant condition may exhibit sequelae or exhibit clinical symptoms of the condition or di sease, and the presence of a tumor may be known, and/or its location is, or can be, identified visually, by palpability, or by any of a number of art accepted imaging and/or other diagnostic techniques, or may be identified upon surgical inspection. The present invention also contemplates that a subject may not manifest clinical symptoms of a malignant condition or may have a small or not yet detectable tumor mass, but the presence of a malignant condition may be identified by a particular diagnostic method, for example, a method that detects cellular changes indicating pre-malignancy or malignancy (such as a PAP smear or a tissue biopsy) or a diagnostic method that detects tumor associated or tumor specific antigens in a biological sample. Such a subject may have a tumor that is graded as an early stage malignant condition according to classification methods known in the medical arts.

A method of screening for the presence of a malignant condition may detect one or more tumor associated markers in a biological sample from a subject. Currently a number of soluble tumor associated antigens are detectable in samples of readily obtained biological fluids. These include, but need not be limited to, CEA, CA 125, sialyl TN, SCC, TPS, mesothelin related antigen, and PLAP, (see *e.g.,* Bast et al., 1983 N. Eng. J. Med. 309:883; Lloyd et al., 1997 Int. J. Canc. 71:842; Sarandakou et al., 1997 Acta Oncol. 36:755; Sarandakou et al., 1998 Eur. J. Gynaecol. Oncol. 19:73; Meier et al., 1997 Anticancer Res. 17(4B):2945; Kudoh et al., 1999 Gynecol. Obstet. Invest. 47:52; Ind et al., 1997 Br. J. Obstet. Gynaecol. 104:1024; Bell et al. 1998 Br. J. Obstet. Gynaecol. 105:1136; Cioffi et al., 1997 Tumori 83:594; Meier et al. 1997 Anticancer Res. 17(4B):2949; Meier et al., 1997 Anticanc. Res. 17(4B):3019; Greiner et al., Cancer Res. 62:6944-51 (2002); WO 00/50900), and may further include any known marker, the presence of which in a biological sample may be correlated with the presence of at least one malignant condition as provided herein. The presence of a malignant condition in a subject may be detected by any of a variety of methods known in the art for detecting expressed tumor associated antigens (such as immunoassay methods) or for detecting nucleic acid molecules that encode a tumor associated marker according to methods known to a skilled artisan (polymerase chain reaction, hybridization and the like).

Tumor cells may be isolated from a biological sample according to methods known in the art and described herein. The term "isolated" means that the material is removed from its original environment (*e.g*., the natural environment if it is naturally occurring). A tumor cell may be isolated from a tumor or from a biological sample such as blood, serum, ascites fluid, lung lavage, or from any other biological sample described herein. The tumor cell may be isolated from a biological sample or separated from normal cells and molecules in a biological sample according to one or more properties of the tumor cell, such as its size, shape, or by virtue of expression of one or more tumor associated antigens. Isolation methods with which a skilled artisan will be familiar include size differentiation centrifugation and chromatography, filtration, flow cytometry, immunological methods using anti-tumor associated antigen (or tumor antigen) antibodies and/or antibodies that specifically bind to one or more cell markers, such as immunohistochemistry, affinity isolation methods (for example, an anti-tumor associated antigen (or tumor antigen) antibody attached to a solid support such as a particle or bead that binds a tumor cell expressing the tumor associated antigen (or tumor antigen)), and the like. Tumor cells may also be propagated and isolated from tissue explants, organ cultures, or cell cultures.

The invention provides compositions for inducing anti-tumor immunity in a subject or host capable of mounting an immune response. As will be known to persons having ordinary skill in the art, an immune response may be any active alteration of the immune status of a host, which may include any alteration in the structure or function of one or more tissues, organs, cells, or molecules that participate in maintenance and/or regulation of host immune status. An immune response may include a stimulation, enhancement, or recruitment of one or more types of immune cells including but not limited to dendritic cells, T cells, B cells, NK cells or other lymphocytes, mast cells, macrophages, other immunologically active cells ofhematopoeitic origin, etc. An immune response may also include the stimulation or increased production of soluble mediators, such as cytokines, chemokines, chemokine binding proteins, and the like, that alter or modulate (increase or decrease in a statistically significant manner), a cellular immune response or signaling pathway.

An immune response may include a cellular response, such as a T cell response that is an alteration (modulation, *e.g*., statistically significant enhancement, stimulation, activation, impairment, or inhibition) of cellular function that is a T cell function. A T cell response may include generation, proliferation or expansion, or stimulation of a particular type ofT cell, or subset ofT cells, for example, CD4+, CD8+, or natural killer (NK) cells. Such T cell subsets may be identified by detecting one or more cell receptors or cell surface molecules *(e.g*., CD or cluster of differentiation molecules) using methods described herein and known in the art, such as flow cytometry, immunohistochemistry, and other immunoassays. A T cell response may also include altered expression (statistically significant increase or decrease) of a cellular factor, such as a soluble mediator (*e.g.,* a cytokine, lymphokine, cytokine binding protein, or interleukin) that influences the growth and differentiation of other cells. An immune response may also include enhancing or stimulating a humoral immune response, that is, generation, activation, or expansion of B cells, including maturation into plasma cells, which are specialized cells of the immune system capable of making antibodies. Inducing an immune response therefore may elicit an increase or enhancement in the titer of one or more antibodies that can be detected using any one of a variety of known immunoassays.

Immune responses may often be regarded, for instance, as including discrimination between self and non-self structures by the cells and tissues of a host's immune system at the molecular and cellular levels, but the invention should not be so limited. For example, immune responses may also include immune system state changes that result from immune recognition of self molecules, cells or tissues, as may accompany any number of normal conditions such as typical regulation of immune system components, or as may be present in pathological conditions such as inappropriate autoimmune responses observed in autoimmune and degenerative diseases. As another example, in addition to induction by up-regulation of particular immune system activities (such as antibody and/or cytokine production, or activation of cell mediated immunity), immune responses may also include suppression, attenuation, or any other down-regulation of detectable immunity, which may be the consequence of the antigen selected, the route of antigen administration, specific tolerance induction, or other factors. In certain preferred embodiments, an immune response that is induced according to the present disclosure generates CD4+ and CD8+ T cells that can facilitate directly or indirectly the death, or loss of the ability to divide or propogate, of a tumor cell, rather than generating CD4+CD25+ regulatory T cells, which regulatory T cells in normal animals are engaged in maintaining immunological self-tolerance (*see* Shimizu et al., Nat. Immunol. 3:135-42 (2002)).

Induction of an immune response by the compositions of the present invention or presence of an immune response induced by these compositions may be determined by detecting any of a variety of well known immunological parameters *(see, e.g*., Platsoucas et al., Anticancer Res. 23:1969-96 (2003); Lyerly, Semin. Oncol. 30(3 Supple 8):9-16 (2003); Takaoka et al., Cancer Sci. 94:405-11 (2003); Nagorsen et al., Crit. Rev. Immunol. 22:449-62 (2002)). Induction of an immune response may therefore be established by any of a number of well known assays, including immunological assays, with which those having ordinary skill in the art will be readily familiar. As described above, such assays include, but need not be limited to, in vivo or in vitro determination of soluble immunoglobulins or antibodies; soluble mediators such as cytokines, lymphokines, chemokines, hormones, growth factors and the like as well as other soluble small peptide, carbohydrate, nucleotide and/or lipid mediators; cellular activation state changes as determined by altered functional or structural properties of cells of the immune system, for example cell proliferation, altered motility, altered intracellular cation gradient or concentration (such as calcium); phosphorylation or dephosphorylation of cellular polypeptides; induction of specialized activities such as specific gene expression or cytolytic behavior; cellular differentiation by cells of the immune system, including altered surface antigen expression profiles, or the onset of apoptosis (programmed cell death); or any other criterion by which the presence of an immune response may be detected. For example, cell surface markers that distinguish immune cell types may be detected by specific antibodies, for example as described herein, antibodies that bind to CD4+, CD8+, or NK cells. Other markers and cellular components that can be detected include but are not limited to interferon γ (IFN-γ), tumor necrosis factor (TNF), CD19, and CD45. Procedures for performing these and similar assays are widely known and may be found, for example in Lefkovits (Immunology Methods Manual: The Comprehensive Sourcebook of Techniques, 1998; *see also* Current Protocols in Immunology; *see also, e.g.,* Weir's Handbook of Experimental Immunology, 1996 Blackwell Scientific, Boston, MA; Mishell and Shigii (eds.) Selected Methods in Cellular Immunology, 1979 Freeman Publishing, San Francisco, CA; Green and Reed, 1998 Science 281:1309 and references cited therein; *see also* Li et al., J. lmmunol. 153:421-28 (1994).

### CD83

As discussed above, CD83 is a marker of activated human dendritic cells (DC) (Banchereau et al., *supra*). In humans, its ligands are primarily expressed on resting monocytes and a subpopulation of activated T cells (Scholler et al. (2001), *supra*). In mice, the ligands for CD83 are primarily expressed on B cells (Cramer et al., Int. Immunol. 12:1347-51 (2000)).

The CD83 polypeptides of the present invention include polypeptides having amino acid sequences that are identical or similar to sequences known in the art. For example by way of illustration and not limitation, a CD83 polypeptide may be of human origin and comprise an amino acid sequence set forth in SEQ ID NO:2 or SEQ ID NO:4 (*see* GenBank Acc. Nos. NP_004224 and AAH30830) encoded by a polynucleotide having a nucleotide sequence set forth in either SEQ ID NO:1 or SEQ ID NO:3 (GenBank Accession No. NM_004233 and BC_030830, respectively) (*see also* U.S. Patent Application No. 2003/0219436, SEQ ID NO:4 therein). The human CD83 polypeptide is an approximately 45 kDa type 1 membrane glycoprotein member of the immunoglobulin superfamily (Zhou et al, J. Immunol. 149:735 (1992)). The polypeptide has a single extracellular V-type Ig-like domain, a transmembrane region, and a 40 amino acid cytoplasmic domain (*id.,* Scholler et al., J. Immunol. 166:3865-72 (2001)).

In certain embodiments, the CD83 polypeptide is a mouse CD83 polypeptide comprising the amino acid sequence set forth in SEQ ID NO:6 or SEQ ID NO:8 *(see* GenBank Acc. Nos. CAB63843 and NP_033986) encoded by a polynucleotide having a nucleotide sequence set forth in either SEQ ID NO:5 or SEQ ID NO:7 (GenBank Accession Nos. AJ245551 and NM_009856, respectively). In other embodiments, the CD83 polypeptide is a rat CD83 polypeptide comprising the amino acid sequence set forth in SEQ ID NO:9 (GenBank Accession No. XP_341510) encoded by a polynucleotide having a sequence set forth in SEQ ID NO:10 (GenBank Accession No. XM_341509).

Variants of these CD83 polypeptides are also contemplated for use according to the instant invention and may contain one or more substitutions, deletions, additions and/or insertions. Such CD83 polypeptide variants have at least 70% similarity (preferably a 70% sequence identity), 80% similarity (preferably a 80% sequence identity), 85% similarity (preferably a 85% sequence identity), and more preferably 90% similarity (more preferably a 90% sequence identity) to the reported polypeptides, more preferably 95% similarity (more preferably a 95% sequence identity), and still more preferably a 98% similarity (still more preferably a 98% sequence identity) to the reported polypeptides. As known in the art "similarity" between two polypeptides is determined by comparing the amino acid sequence and conserved amino acid substitutes thereto of the polypeptide to the sequence of a second polypeptide. Fragments or portions of the nucleic acid molecules encoding polypeptides of the present invention may be used to synthesize full-length polynucleotides. As used herein, "% identity" refers to the percentage of identical amino acids situated at corresponding amino acid residue positions in a sequence when two or more polypeptide are aligned and their sequences analyzed using a gapped BLAST algorithm (*e.g.,* Altschul et al., Nucleic Acids Res. 25:3389 (1997)), which weights sequence gaps and sequence mismatches according to the default weightings provided by the National Institutes of Health/ NCBI database (Bethesda, MD; *see* Intemet:>www.ncbi.nlm.nih.gov/cgi-bin/BLAST/nph-newblast).

A CD83 polypeptide variant of the present invention preferably has similar function to a wild type CD83 polypeptide, including the ability to bind to a carbohydrate epitope on the CD83 ligand, which epitope depends on sialic acid residues, (*see* Scholler et al., J. Immunol. (2001) *supra*). Determination of the ability of a CD83 variant polypeptide to bind to the CD83 ligand may be performed using techniques for detecting binding according to any number of assays known in the art (*see also id*.). Assays or techniques that assess the function of a CD83 polypeptide variant may also be used. For example, a human B cell line, such as the T51 cell line, may be transfected with a polynucleotide that encodes a cell surface CD83 polypeptide variant, and the functions and properties of the transfected cell line determined. A cell line such as the T51 cell line so transfected may have an ability to stimulate proliferation of allogeneic PBMC, including CD8+ cytotoxic T lymphocytes (CTL), similar to that exhibited by the B cell line transfected with the CD83 wildtype polypeptide (*see* Scholler et al., J. Immunol. 168:2599-2602, (2002)). A CD83 polypeptide variant that exhibits the ability to stimulate proliferation of immune cells, or to exhibit to a substantially similar extent one or more other functions that are characteristic of the wildtype CD83 polypeptide, exhibits preferably at least 65% of the ability of the wildtype CD83 polypeptide, more preferably 70-80% of the wildtype CD83 polypeptide, and still more preferably 80-90%, and even still more preferably 90-100% of the ability exhibited by the wildtype CD83 polypeptide as determined by the particular technique.

The integrity of the secondary or tertiary structure of a CD83 polypeptide variant may be determined by retention of the specificity and affinity of the CD83 polypeptide variant for the CD83 ligand and by determining the effect of the CD83 polypeptide variant on the proliferation of CD8+ cells as described above. In addition, whether the secondary and tertiary structures of the CD83 polypeptide variant are maintained (*i.e.,* remain substantially similar to the secondary and tertiary structure of wildtype CD83) may be determined by evaluating the ability of the variant to bind to one or more antibodies that specifically bind to the wildtype CD83 polypeptide in a conformation-dependent manner. Preparation of anti-CD83 antibodies may be performed by methods described herein and known in the art, and immunoassays using these antibodies may be performed by any number of immunoassays described herein and known in the art, including immunoblotting, ELISA, immunoprecipitation, immunohistochemistry, and the like.

A CD83 polypeptide variant is regarded as specific or capable of specifically binding if it binds a desired target molecule such as a CD83 ligand as provided herein, with a Kₐ of greater than or equal to about 10⁴ M⁻¹, preferably of greater than or equal to about 105 M⁻¹, more preferably of greater than or equal to about 10⁶ M⁻¹ and still more preferably of greater than or equal to about 10⁷ M⁻¹, and still more preferably of greater than or equal to about 10⁸ M⁻¹. Affinity of a CD83 polypeptide or variant thereof for its cognate ligand is also commonly expressed as a dissociation constant K_{D}, and a CD83 polypeptide or variant thereof specifically binds to a ligand if it binds with a K_{D} of less than or equal to 10⁻⁴ M, more preferably less than or equal to about 10⁻⁵ M, more preferably less than or equal to about 10⁻⁶ M, still more preferably less than or equal to 10⁻⁷ M, and still more preferably less than or equal to 10⁻⁸ M. Affinities of CD83 polypeptides and variants thereof according to the present invention can be readily determined using conventional techniques, for example those described by Scatchard et al., Ann. N. Y. Acad. Sci. 51:660 (1949) or by surface plasmon resonance (BIAcore, Biosensor, Piscataway, NJ) *(see, e.g.,* Wolff et al., Cancer Res. 53:2560-2565 (1993)).

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine, and valine; glycine, and alanine; asparagine and glutamine; and serine, threonine, phenylalanine, and tyrosine. Other groups of amino acids that may represent conservative changes include (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Amino acids may be classified according to the nature of their side groups. Amino acids with nonpolar alkyl side groups include glycine, alanine, valine, leucine, and isoleucine. Serine and threonine have hydroxyl groups on their side chains, and because hydroxyl groups are polar and capable of hydrogen bonding, these amino acids are hydrophilic. Sulfur groups may be found in methionine and cysteine. Carboxylic acid groups are part of the side chain of aspartic acid and glutamic acid, which because of the acidity of the carboxylic acid group, the amino acids are not only polar but can become negatively charged in solution. Glutamine and asparagine are similar to glutamic acid and aspartic acid except the side chains contain amide groups. Lysine, arginine, and histidine have one or more amino groups in their side chains, which can accept protons, and thus these amino acids act as bases. Aromatic groups may be found on the side chains of phenylalanine, tyrosine, and tryptophan. Tyrosine is polar because of its hydroxyl group, but tryptophan and phenylalanine are non-polar. A variant may also, or alternatively, contain nonconservative changes.

A CD83 variant with at least one substitution, addition, insertion, or deletion may be made according to mutagenesis methods described herein and known in the art. Such modifications in a polynucleotide sequence that encodes a CD83 variant or derivative may be introduced using standard mutagenesis techniques, such as oligonucleotide-directed site-specific mutagenesis. Alterations of the native amino acid sequence may be accomplished by any of a number of conventional methods. Mutations can be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion. Alternatively, as described herein in detail and known in the art, oligonucleotide-directed site-specific mutagenesis procedures can be employed to provide an altered encoding polynucleotide wherein predetermined codons can be altered by substitution, deletion, or insertion. A CD83 polypeptide for use in the present invention may be a portion or fragment of a full-length CD83 polypeptide or a truncated CD83 polypeptide as described herein. A truncated CD83 polypeptide as described in greater detail herein may be any CD83 polypeptide molecule that comprises less than a full-length version of the CD83 polypeptide. As used herein "deletion" has its common meaning as understood by those familiar with the art, and may refer to molecules that lack one or more of a portion of a sequence from either terminus or from a non-terminal region, relative to a corresponding full length molecule, for example, as in the case of truncated molecules provided herein.

A portion or fragment or a truncated form of a CD83 polypeptide may have any number of amino acids fewer than about 175, 170, 160, 155, 150, 149,148,147, 146, 145, 144, 143, 142, 141, 140, 139, 138, 137, 135,130, 125, 120, 115, 100, 95, 90, 85, 80, 75, 70, 65, 60 or 55 amino acids. In certain embodiments a truncated CD83 polypeptide comprises a portion of a CD83 polypeptide that is the extracellular domain of the CD83 polypeptide. The portion of a CD83 polypeptide that is the extracellular domain may also include one, two, three, four, five, six or more residues of the transmembrane region of the CD83 polypeptide. A portion of a CD83 polypeptide may also include the extracellular domain and the transmembrane domain, which may further include 1, 2, 3, 4, 5, or more residues of the intracellular cytoplasmic portion of CD83. The CD83 extracellular domain may be detected by a variety of methods, such as immunological methods, using techniques by which intracellular epitopes of the CD83 polypeptide are not detected. For example, detecting a CD83 polypeptide expressed on the surface of a cell according to methods for detecting an extracellular domain of the CD83 polypeptide preferably excludes any step that permeabilizes the cell expressing the CD83 polypeptide. Given the CD83 amino acid sequences provided herein and known in the art, antibodies that bind to epitopes on the extracellular domain, which may be used to identify the extracellular domain of CD83, may be prepared according to methods described herein and known in the art to artisans skilled in the antibody arts.

A CD83 polypeptide extracellular domain may also include a portion of a CD83 polypeptide that is susceptible to cleavage by one or more proteases that naturally occur in the extracellular environment, such as in a tissue, organ, or cell culture, or that is exogenously added to a cell culture or to a mixture of cells expressing a CD83 polypeptide on the cell surface. The extracellular domain of the human CD83 polypeptide (SEQ ID NO:2) comprises amino acids located at position 1 through about position 144 of SEQ ID NO:2. In alternative embodiments, the extracellular domain comprises amino acids located at positions 1-140, 1-141, 1-142, or 1-143 of SEQ ID NO:2, and in certain other embodiments the extracellular domain comprises amino acids located at positions 1-145, 1-146, 1-147, or 1-148 of SEQ ID NO:2. The extracellular domain of mouse CD83 polypeptide comprises amino acids located at position 1 through about position 134 of SEQ ID NO:6. In alternative embodiments, an extracellular domain comprises amino acids located at positions 1-130, 1-131, 1-132, or 1-133 of SEQ ID NO:6, and in certain other embodiments the extracellular domain comprises amino acids located at positions 1-135, 1-136, 1-137, or 1-138 of SEQ ID NO:6. In certain embodiments, a portion of a CD83 polypeptide may comprise the extracellular domain and the transmembrane region of a CD83 polypeptide.

In one embodiment, the CD83 polypeptide is a cell surface CD83 fusion polypeptide. The CD83 polypeptide or a portion thereof is fused to a second polypeptide such that the CD83 polypeptide component (or a portion thereof) of the CD83 fusion polypeptide is extracellular, but the cell surface fusion protein is associated with a cell. For instance, the polypeptide to which the CD83 polypeptide is fused may be found as a component of the cell (plasma) membrane and may optionally extend into the cytoplasm. Alternatively, the fusion protein may be associated with the cell surface via any of a number of known molecular tethering structures such as posttranslational modifications described herein, or affinity domains such as a receptor, counterreceptor, or ligand domains capable of interaction with a cell surface structure (*e.g*., an FcR) or the like. Preferably, the CD83 polypeptide is not involved in the mechanism of attachment to or retention on the surface of the cell in which it is expressed. A CD83 polypeptide or portion thereof may be fused to at least one immunoglobulin constant region polypeptide. The portion of the CD83 polypeptide that is fused to another polypeptide is preferably the extracellular domain of the CD83 polypeptide as described herein. The CD83 fusion polypeptide may also include the CD83 transmembrane domain. In alternative embodiments, a transmembrane domain is derived from any one of numerous transgenic domains that are known in the art as useful for cell surface expression of a polypeptide, for example, the transmembrane domain of CD80 or CTLA4.

### Anti-Immune Cell Receptor Antibodies

The present invention also relates to the surprising discovery that induction of an immune response may be enhanced, increased, or improved by stimulating a second molecule that is involved in the immune response to tumor cells and tumor antigens expressed thereon, in addition to immune stimulation via a CD3 8-mediated mechanism. As a non-limiting example, a composition for inducing anti-tumor immunity may thus comprise a tumor cell that expresses a cell surface CD83 polypeptide and a tumor cell that expresses an antibody or antigen-binding fragment thereof that binds specifically to a receptor on an immune cell, for example, an antibody or antibody fragment that specifically binds to CD137 (4-1BB).

CD137 is a co-stimulatory receptor expressed on activated T cells that can amplify or enhance T cell immunity. Synonyms for CD137 that are known in the art include 4-1BB, member 9 of the tumor necrosis factor receptor superfamily, ILA, CDw137, and MGC2172. Co-stimulatory signals have been defined as signals that occur as a result of ligation of membrane bound molecules that enhance, synergize with, or modify signals provided when a T cell receptor engages a peptide-MHC complex. CD137 is a member of the tumor necrosis factor receptor (TNFR) family and is expressed on activated CD8+ and CD4+ T cells (*see, e.g.,* Hurtado et al., J. Immunol. 158:2600 (1997); Takahashi et al., J. Immunol. 162:5037 (1999); Diehl et al., J. Immunol. 168:3755-62 (2002)). A monoclonal antibody (1D8) that specifically binds to murine CD 137 reportedly engages an anti-tumor response more effectively than the natural ligand of CD137 (4-1BBL) (*see, e.g.,* Ye et al., Nature Medicine 8:343-48 (2002)). A melanoma tumor cell line K1735 that was transfected to express a single chain Fv fragment of 1D8 (1D8 scFv) on the cell surface induced a strong type 1 T-helper cell (Th1) response for which CD4+ but not CD8+ T cells are needed, and which may also involve natural killer (NK) cells (*id.*)*.* Other members of the TNFR family, for example OX40 (CD134) and CD27, also may be involved in generating a T cell response. Antibodies or antigen-binding fragments thereof that specifically bind such an immune cell receptor, or the cognate ligands of the TNFR family member may be expressed as cell surface forms for use in the present invention.

Another example of an immune cell receptor that may be usefully recognized by a cell surface antibody or antigen-binding fragment thereof expressed on a tumor cell according to the present invention is CD40 (*see, e.g.,* Kumura et al., Cancer Gene Ther. 10: 833-39 (2003); Coughlin et al., Cancer Biol. Ther. 2:466-70 (2003)). CD40 is a membrane differentiation antigen that is constitutively expressed on B cells and is also expressed by a variety of other cell types including dendritic cells, follicular dendritic cells, monocytes, macrophages, mast cells, fibroblasts, and endothelial cells (*see, e.g.,* Cayabyab et al., J. Immunol. 152:1523-31 (1994); Grewal et al., Annu. Rev. Immunol. 16:111-35 (1998)). CD4O enables antigen-presenting cells to process and present antigen effectively to T cells (*see, e.g.,* French et al., Nat. Med. 5:548-53 (1999)). A cytotoxic T cell response mediated by an expansion of CD8+ T cells occurs when the CD40 ligand CD154 triggers CD40 (*see, e.g.,* French et al., *supra*; Di ehl et al., Nat. Med. 5:774-79 (1999); Schoenberger et al., Nature 393:480-83 (1998)). An antibody that specifically binds to CD40 is also able to contribute to the generation of antigen-presenting cells that are capable of priming cytotoxic T lymphocytes, which may increase tumor immunity (Todryk et al., J. Immunol. Methods 248:139-47 (2001)).

CD137 polypeptides include polypeptides having amino acid sequences that are identical or similar to CD137 sequences known in the art. For example by way of illustration and not limitation, a CD137 polypeptide may be of human origin and comprise an amino acid sequence set forth in SEQ ID NO:12 or SEQ ID NO:14 encoded by a polynucleotide having a nucleotide sequence set forth in either SEQ ID NO:11 or SEQ ID NO:13 *(see* GenBank Accession No. BC006196 and NM_001561, respectively; *see* GenBank Acc. Nos. AAH06196 and NP_001552 also providing the amino acid sequence). Alternatively, a CD 137 polypeptide may be of mouse origin and comprise an amino acid sequence set forth in SEQ ID NO:16 encoded by a polynucleotide having a nucleotide sequence set forth in either SEQ ID NO:15 *(see* GenBank Accession No. NM_011612; *see also* GenBank Accession No. NP_035742, also providing the amino acid sequence).

As described herein, CD137 represents a preferred immune cell receptor that may be recognized by an antibody or antigen-binding fragment thereof, such as a recombinantly expressed antibody on a tumor cell surface. However, while anti-CD137 antibodies are described as a representative example, the invention also contemplates antibodies specific for other immune cell receptors. In one embodiment of the invention, a single chain Fv antibody that specifically binds to a CD137 polypeptide is capable ofbeing expressed by a host cell such that it localizes to the surface of the host cell. In particular embodiments, the host cell is a tumor cell. Antibodies that bind specifically to a CD137 antigen are known in the art and also can be readily generated using methods described herein and known in the art (*see, e.g.,* Melero et al., Nature Med. 3:682-85 (1998)). One anti-CD137 antibody that may be useful for practicing the present invention is designated 1D8 and is described above *(see e.g., id*.; Ye et al., Nature Med., supra). A cell surface anti-CD 137 scFv antibody may be constructed by operatively linking a polynucleotide that encodes the anti-CD137 scFv antibody to a polynucleotide that encodes a polypeptide that comprises a transmembrane portion (or domain) and a cytoplasmic portion (or domain) such that the anti-CD137 scFv antibody will be expressed on the cell surface. In certain embodiments, the transmembrane domain and the cytoplasmic domain of a CD80 polypeptide (CD80/B7.1, *see, e.g.,* Freeman et al., 1989 J. Immunol. 43:2714; Freeman et al., 1991 J. Exp. Med. 174:625; see also *e.g.,* GenBank Acc. Nos. U33208, I683379) is fused to an anti-CD137 scFv antibody. Such cell surface expressed scFv antibodies maybe constructed using methods known in the art and described herein *(see, e.g.,* Hayden et al., Tissue Antigens 48:242-54 (1996); Winberg et al., Immunol. Rev. 153:209-23 (1996)).

Antibodies that are specific for a CD137 polypeptide are known in the art (*see, e.g.,* Melero et al., Nature Med. 3:682-85 (1997)) and can be readily generated as monoclonal antibodies or as polyclonal antisera, or may be produced as genetically engineered immunoglobulins (Ig) that are designed to have desirable properties using methods well known in the art. One antibody described herein and known in the art is 1D8, which is a monoclonal antibody that binds to murine CD137 (*see, e.g.,* Shuford et al, J. Exp. Med. 186:47-55 (1997); Melero et al., Nature Med. 3:682-85 (1997)). Forexample, by way of illustration and not limitation, antibodies contemplated by the present invention may include recombinant IgGs, chimeric fusion proteins having immunoglobulin derived sequences, or "humanized" antibodies (see, *e.g.,* U.S. Patent Nos. 5,693,762; 5,585,089; 4,816,567; 5,225,539; 5,530,101; and references cited therein) that may all be used according to the invention. Antibodies and antigen-binding fragments thereof that specifically bind to any of the polypeptides of interest discussed herein, including other immune cell receptors, a CD83 polypeptide, and one or more tumor antigens (or tumor associated antigens), may also be readily prepared using methods described herein and known in the art.

The term "antibodies" includes polyclonal antibodies, monoclonal antibodies, and antigen-binding fragments thereof such as F(ab')₂, Fab', Fab fragments, and Fv fragments as well as any naturally occurring or recombinantly produced binding partners, which are molecules that specifically bind a polypeptide of interest. Antibodies are defined to be "immunospecific" or specifically binding if they bind to an antigen or polypeptide of interest with a Kₐ of greater than or equal to about 10⁴ M⁻¹, preferably of greater than or equal to about 10⁵ M⁻¹, more preferably of greater than or equal to about 10⁶ M⁻¹, and still more preferably of greater than or equal to about 10⁷ M⁻¹, and still more preferably of greater than or equal to about 10⁸ M⁻¹. Affinities of binding partners or antibodies can be readily determined using conventional techniques, for example those described by Scatchard et al., Ann. N.Y. Acad. Sci. 51:660 (1949) or by surface plasmon resonance (BIAcore, Biosensor, Piscataway, NJ). *See, e.g.,* Wolff et al., Cancer Res. 53:2560-65 (1993). Affinity of an antibody for its cognate antigen is also commonly expressed as a dissociation constant K_{D}, and, for example, an anti-CD137 antibody specifically binds to a CD 137 polypeptide if it binds with a K_{D} of less than or equal to 10⁻⁴ M, more preferably less than or equal to about 10⁻⁵ M, more preferably less than or equal to about 10⁻⁶ M, still more preferably less than or equal to 10⁻⁷ M, and still more preferably less than or equal to 10⁻⁸ M.

Antibodies may generally be prepared by any of a variety of techniques known to those of ordinary skill in the art *(see, e.g.,* Harlow and Lane, Antibodies: A Laboratory Manual, Cold Spring Harbor Laboratory, 1988). Preparation of the immunogen for injection into animals may include covalent coupling of the polypeptide of interest (or variant or fragment thereof), to another immunogenic protein, for example, a carrier protein such as keyhole limpet hemocyanin (KLH) or bovine serum albumin (BSA). In addition, the peptide, polypeptide, or polypeptide-expressing cells to be used as immunogen may be emulsified in an adjuvant. *See, e.g.,* Harlow et al., *supra*. In one such technique, an immunogen comprising a CD 137 polypeptide or peptide thereof, (or another polypeptide of interest), for example, a purified CD 137 polypeptide or fragment thereof or a cell having a CD137 polypeptide on its surface is initially injected into a suitable animal (*e.g*., a mouse, rat, rabbit, sheep, hamster, chicken, and goat), preferably according to a predetermined schedule incorporating one or more booster immunizations. The animals may be bled periodically and the titer (an indicator of concentration) of the specific antibodies of interest can be determined. Polyclonal antibodies specific for the CD137 polypeptide (or other polypeptide of interest) may then be purified from such antisera, for example, by affinity chromatography using the polypeptide antigen, protein A, or an antibody specific for a constant region domain epitope coupled to a solid support.

Monoclonal antibodies specific for polypeptides, variants or derivatives thereof, described herein thereof may be prepared, for example, using the technique of Kohler and Milstein (Eur. J. Immunol. 6:511-519 (1976)), and improvements thereto. Briefly, these methods involve the preparation of immortal cell lines capable ofproducing antibodies having the desired specificity (*e*.*g*., reactivity with a CD137 or another polypeptide of interest). Lymphoid cells that include antibody-forming cells, typically spleen cells, are obtained from an immunized animal and may then be immortalized by, for example, fusion with a drug-sensitized myeloma (*e*.*g*., plasmacytoma) cell fusion partner, preferably one that is syngeneic with the immunized animal and that optionally has other desirable properties (*e*.*g*., inability to express endogenous Ig gene products). For example, the spleen cells and myeloma cells may be combined with a membrane fusion promoting agent such as polyethylene glycol or a nonionic detergent for a few minutes, and then plated at low density on a selective medium that supports the growth of hybrid cells but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred. Hybridomas that generate monoclonal antibodies that specifically bind to human and mouse CD137 polypeptides are contemplated by the present invention.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Alternatively, the nucleotide sequences encoding the immunoglobulin light chain and heavy chain variable regions may be cloned and introduced into other cell lines that naturally or with manipulation produce a higher concentration of antibody than the original hybridoma cell. Contaminants may be removed from the ascites fluid or from culture supernatants from the antibodies by conventional techniques, such as chromatography, gel filtration, precipitation, extraction, and the like. For example, antibodies may be purified by affinity chromatography using an appropriate ligand selected based on particular properties of the monoclonal antibody (*e.g*., heavy or light chain isotype, binding specificity, etc.). Examples of a suitable ligand, immobilized on a solid support, include Protein A, Protein G, an anti-constant region (light chain or heavy chain) antibody, an anti-idiotype antibody, and the antigen, such as a CD137 polypeptide, which may be accomplished according to standard techniques.

Within certain embodiments, the use of antigen-binding fragments of antibodies may be preferred. Such fragments include Fab fragments or F(ab')₂ fragments, which may be prepared by proteolytic digestion with papain or pepsin, respectively. The antigen binding fragments may be separated from the Fc fragments by affinity chromatography, for example, using immobilized protein A or protein G, or immobilized polypeptide to which the antibody specifically binds, or a suitable variant or fragment thereof. Those having ordinary skill in the art can routinely and without undue experimentation determine what is a suitable variant or fragment based on characterization of affinity purified antibodies obtained, for example, using immunodetection methods as provided herein. A method to generate an Fab' fragment includes mild reduction of disulfide bonds in a F(ab')₂ fragment followed by alkylation. *See, e.g.,* Weir, Handbook of Experimental Immunology, 1986, Blackwell Scientific, Boston.

Antibodies also may be produced as genetically engineered immunoglobulins (Ig) or Ig fragments designed to have desirable properties (*see generally* Antibody Engineering, Methods and Protocols, Lo, ed., (Human Press 2004)). For example, by way of illustration and not limitation, antibodies may include a recombinant IgG that is a chimeric fusion protein having at least one variable (V) region domain from a first mammalian species and at least one constant region domain from a second, distinct mammalian species. Most commonly, a chimeric antibody has murine variable region sequences and human constant region sequences. Such a murine/human chimeric immunoglobulin may be "humanized" by grafting the complementarity determining regions (CDRs) derived from a murine antibody, which confer binding specificity for an antigen, into human-derived V region framework regions and human-derived constant regions. Immunoglobulins (antibodies) may be further engineered, for example, the V regions may be mutated to increase the binding affinity (an in vitro procedure that mimics in vivo affinity maturation) *(see, e.g.,* Mountain et al., Biotechmol. Genet. Eng. Rev. 10:1-142 (1992); Glaser et al., J. Immunol. 149:3903-13 (1992); Barbas et al., Proc. Natl. Acad. Sci. USA 91:3809-13 (1994); U.S. Patent No. 5,792,456), or mutations may be introduced into a constant region domain to alter (increase or decrease in a statistically significant manner) an effector function of the constant region *(see, e.g.,* Duncan et al., Nature 332:563-64 (1988); Morgan et al., Immunology 86:319-24 (1995); Eghtedarzedeh-Kondri et al., Biotechniques 23:830-34 (1997); Wright et al., Trends Biotechnol. 15:26-32 (1997); Sensel et al., Mol. Immunol. 34:1019-29 (1997)).

According to certain embodiments, non-human, human, or humanized heavy chain and light chain variable regions of any of the above described immunoglobulin molecules may be constructed as single chain Fv (sFv) polypeptide fragments (single chain antibodies). *See, e.g.,* Bird et al., 1988 Science 242:423-426; Huston et al., 1988 Proc. Natl. Acad. Sci. USA 85:5879-83. In certain embodiments of the present invention, the anti-CD137 scFv antibody comprises at least one immunoglobulin variable region polypeptide, which may be a light chain or a heavy chain variable region polypeptide, and in certain embodiments the fusion protein comprises at least one such light chain V-region and one such heavy chain V-region and at least one linker peptide that is fused to each of the V-regions. Construction of such binding domains, for example single chain Fv domains, is well known in the art and has been described, for example, in U.S. Patent Nos. 5,892,019 and references cited therein. Selection and assembly of single-chain variable regions and of linker polypeptides that may be fused to each of a heavy chain-derived and a light chain-derived V region (*e.g*., to generate a binding domain that comprises a single-chain Fv polypeptide) are also known to the art and described herein and, for example, in U.S. Patent Nos. 5,869,620, U.S. 4,704,692 and U.S. 4,946,778.

In certain embodiments all or a portion of an immunoglobulin sequence that is derived from a non-human source may be "humanized" according to recognized procedures for generating humanized antibodies, *i.e*., immunoglobulin sequences into which human immunoglobulin sequences are introduced to reduce the degree to which a human immune system would perceive such proteins as foreign (see, *e.g*., U.S. Patent Nos. 5,693,762; 5,585,089; 4,816,567; 5,225,539; 5,530,101; and references cited therein; *see also* Popkov et al, J. Mol. Biol. 325:325-35 (2003); Rader et al., J. Biol. Chem. 275:18668-76 (2000)).

Multifunctional fusion proteins having specific binding affinities for preselected antigens by virtue of immunoglobulin V-region domains encoded by DNA sequences linked in-frame to sequences encoding various effector proteins are known in the art, for example, as disclosed in EP-B1-0318554, U.S. Patent No. 5,132,405, U.S. Patent No. 5,091,513 and U.S. Patent No. 5,476,786. Such effector proteins include polypeptide domains that may be used to detect binding of the fusion protein by any of a variety of techniques with which those skilled in the art will be familiar, including but not limited to a biotin mimetic sequence *(see, e.g.,* Luo et al., 1998 J. Biotechnol. 65:225 and references cited therein), direct covalent modification with a detectable labeling moiety, non-covalent binding to a specific labeled reporter molecule, enzymatic modification of a detectable substrate or immobilization (covalent or non-covalent) on a solid-phase support.

Single chain antibodies for use in the present invention may also be generated and selected by a method such as phage display (*see, e.g.,* U.S. Patent No. 5,223,409; Schlebusch et al., 1997 Hybridoma 16:47; and references cited therein; *see also* Andris-Widhopf et al., J. Immunol. Methods 242:159-81 (2000)). Briefly, in this method, DNA sequences are inserted into the gene III or gene VIII gene of a filamentous phage, such as M13. Several vectors with multicloning sites have been developed for insertion (McLafferty et al., Gene 128:29-36,1993; Scott and Smith, Science 249:386-390, 1990; Smith and Scott, Methods Enzymol. 217:228-257, 1993). The inserted DNA sequences may be randomly generated or may be variants of a known binding domain for binding to a CD 137 polypeptide (or other polypeptide of interest). Single chain antibodies may readily be generated using this method. The peptide encoded by the inserted sequence is displayed on the surface of the bacteriophage. Bacteriophage expressing a binding domain for a CD137 polypeptide are selected by binding to an immobilized CD 137 polypeptide, for example a recombinant polypeptide prepared using methods well known in the art and nucleic acid coding sequences as disclosed as described herein. The DNA sequence of the insert in the binding phage is then determined. Once the predicted amino acid sequence of the binding peptide is known, sufficient peptide for use herein as an antibody specific for a human CD137 polypeptide may be made either by recombinant means or synthetically. Recombinant means are used when the antibody is produced as a fusion protein. The peptide may also be generated as a tandem array of two or more similar or dissimilar peptides, in order to maximize affinity or binding.

In addition, an antibody of the present invention may be a human monoclonal antibody. Human monoclonal antibodies may be generated by any number of techniques with which those having ordinary skill in the art will be familiar. Such methods include, but are not limited to, Epstein Barr Virus (EBV) transformation of human peripheral blood cells (e.g., containing B lymphocytes), *in vitro* immunization of human B cells, fusion of spleen cells from immunized transgenic mice carrying inserted human immunoglobulin genes, isolation from human immunoglobulin V region phage libraries, or other procedures as known in the art and based on the disclosure herein. For example, human monoclonal antibodies may be obtained from transgenic mice that have been engineered to produce specific human antibodies in response to antigenic challenge (see, e.g., Green et al., Nature Genet. 7:13, 1994; Lonberg et al., Nature 368:856 (1994); Taylor et al., Int. Immun. 6:579 (1994); U.S. Patent No. 5,877,397; Bruggemann et al., Curr. Opin. Biotechnol. 8:455-58 (1997); Jakobovits et al., Ann. N. Y. Acad. Sci. 764:525-35 (1995)). Lymphoid cells of the immunized transgenic mice can be used to produce human antibody-secreting hybridomas according to the methods described herein. Polyclonal sera containing human antibodies may also be obtained from the blood of these immunized animals.

Another method for generating specific monoclonal antibodies includes immortalizing human peripheral blood cells by EBV transformation. *See, e.g.,* U.S. Patent No. 4,464,456. Such an immortalized B cell line (or lymphoblastoid cell line) producing a monoclonal antibody that specifically binds to a polypeptide of interest described herein (or a variant or fragment thereof) can be identified by immunodetection methods as provided herein, for example, an ELISA, and then isolated by standard cloning techniques. The stability of the lymphoblastoid cell line producing an anti-TGF-beta binding protein antibody may be improved by fusing the transformed cell line with a murine myeloma to produce a mouse-human hybrid cell line according to methods known in the art (*see, e.g.,* Glasky et al., Hybridoma 8:377-89 (1989)). Still another method to generate human monoclonal antibodies is *in vitro* immunization, which includes priming human splenic B cells with antigen, followed by fusion of primed B cells with a heterohybrid fusion partner. *See, e.g.,* Boerner et al., J. Immunol. 147:86-95 (1991).

In certain embodiments, a B cell that is producing an antibody that specifically binds to a polypeptide of interest is selected and the light chain and heavy chain variable regions are cloned from the B cell according to molecular biology techniques known in the art (WO 92/02551; US patent 5,627,052; Babcook et al., Proc. Natl. Acad. Sci. USA 93:7843-48 (1996)) and described herein. Preferably B cells from an immunized animal are isolated from the spleen, lymph node, or peripheral blood sample by selecting a cell that is producing a specific antibody. B cells may also be isolated from humans, for example, from a peripheral blood sample. Methods for detecting single B cells that are producing an antibody with the desired specificity are well known in the art, for example, by plaque formation, fluorescence-activated cell sorting, in vitro stimulation followed by detection of specific antibody, and the like. Methods for selection of specific antibody producing B cells include, for example, preparing a single cell suspension of B cells in soft agar that contains the polypeptide of interest or a peptide fragment thereof. Binding of the specific antibody produced by the B cell to the antigen results in the formation of a complex, which may be visible as an immunoprecipitate. After the B cells producing the specific antibody are selected, the specific antibody genes may be cloned by isolating and amplifying DNA or mRNA according to methods known in the art and described herein.

To detect an antibody specific for any polypeptide described herein, including a CD 13 7 polypeptide, a variety of assay formats are known to those of ordinary skill in the art, including but not limited to enzyme linked immunosorbent assay (ELISA), radioimmunoassay (RIA), immunofluorimetry, immunoprecipitation, equilibrium dialysis, immunodiffusion and other techniques. *See, e.g.,* Harlow and Lane, Antibodies: A Laboratory Manual, Cold Spring Harbor Laboratory, 1988; Weir, D.M., Handbook of Experimental Immunology, 1986, Blackwell Scientific, Boston. For example, the assay may be performed in a Western blot format, wherein a protein preparation is submitted to gel electrophoresis, transferred to a suitable membrane and allowed to react with the sample containing an antibody. The presence of the antibody on the membrane may then be detected using a suitable detection reagent, as is well known in the art and described below.

### Tumor Antigen

In certain embodiments as described herein, at least one tumor antigen or tumor-associated antigen may be expressed on a tumor cell that is present within compositions disclosed herein for inducing an anti-tumor immune response, which compositions also include a cell surface CD83 polypeptide and a cell surface expressed antibody that specifically binds to an immune cell receptor. In alternative embodiments, a recombinant expression vector (*e.g*., a recombinant expression construct) comprising a polynucleotide sequence that encodes at least one tumor antigen or tumor-associated antigen, or a portion or fragment thereof, is present in a composition for inducing an anti-tumor immunity according to methods described herein. In certain related embodiments, a recombinant expression vector may comprise a polynucleotide sequence that encodes two, three or more tumor antigens and/or tumor-associated antigens. In another alternative embodiment, the expression product(s) (*e.g*., isolated tumor antigen and/or tumor-associated antigen) is delivered to a host according to methods described herein such as by a peptide delivery system.

Antigenic markers such as serologically defined markers known as tumor associated antigens, which are either uniquely expressed by cancer cells or are present at markedly higher levels (*e.g*., elevated in a statistically significant manner) in subjects having a malignant condition relative to appropriate controls, are contemplated for use in certain related embodiments. Tumor-associated antigens may include, for example, cellular oncogene-encoded products or aberrantly expressed proto-oncogene-encoded products (*e.g*., products encoded by the neu, ros, trk, and kit genes), or mutated forms of growth factor receptor or receptor-like cell surface molecules (*e.g*., surface receptor encoded by the c-erb B gene). Other tumor-associated antigens include molecules that may be directly involved in transformation events, or molecules that may not be directly involved in oncogenic transformation events but are expressed by tumor cells (*e.g*., carcinoembryonic antigen, CA-125, melonoma associated antigens, etc.) (*see, e.g.,* U.S. Patent No. 6,699,475; Jager et al., Int. J. Cancer 106:817-20 (2003); Kennedy et al., Int. Rev. Immunol. 22:141-72 (2003); Scanlan et al. Cancer Immun. 4:1 (2004)).

Genes that encode cellular tumor associated antigens include cellular oncogenes and proto-oncogenes that are aberrantly expressed. In general, cellular oncogenes encode products that are directly relevant to the transformation of the cell, and because of this, these antigens are particularly preferred targets for immunotherapy. An example is the tumorigenic *neu* gene that encodes a cell surface molecule involved in oncogenic transformation. Other examples include the *ros*, *kit*, and *trk* genes. The products of proto-oncogenes (the normal genes which are mutated to form oncogenes) may be aberrantly expressed (*e.g*., overexpressed), and this aberrant expression can be related to cellular transformation. Thus, the product encoded by proto-oncogenes can be targeted for immune therapy. Some oncogenes encode growth factor receptor molecules or growth factor receptor-like molecules that are expressed on the tumor cell surface. An example is the cell surface receptor encoded by the *c-erbB* gene. Other tumor-associated antigens may or may not be directly involved in malignant transformation. These antigens, however, are expressed by certain tumor cells and may therefore provide effective targets for immunotherapy. Some examples are carcinoembryonic antigen (CEA), CA 125 (associated with ovarian carcinoma), and melanoma specific antigens.

In ovarian and other carcinomas, for example, tumor associated antigens are detectable in samples of readily obtained biological fluids such as serum or mucosal secretions. One such marker is CA125, a carcinoma associated antigen that is also shed into the bloodstream, where it is detectable in serum (*e*.*g*., Bast et al., 1983 N. Eng. J. Med. 309:883; Lloyd et al., 1997 Int. J. Canc. 71:842). CA125 levels in serum and other biological fluids have been measured along with levels of other markers, for example, carcinoembryonic antigen (CEA), squamous cell carcinoma antigen (SCC), tissue polypeptide specific antigen (TPS), sialyl TN mucin (STN), and placental alkaline phosphatase (PLAP), in efforts to provide diagnostic and/or prognostic profiles of ovarian and other carcinomas (*e*.*g*., Sarandakou et al., 1997 Acta Oncol. 36:755; Sarandakou et al., 1998 Eur. J. Gynaecol. Oncol. 19:73; Meier et al., 1997 Anticancer Res. 17(4B):2945; Kudoh et al., 1999 Gynecol. Obstet. Invest. 47:52; Ind et al., 1997 Br. J. Obstet. Gynaecol. 104:1024; Bell et al. 1998 Br. J. Obstet. Gynaecol. 105:1136; Cioffi et al., 1997 Tumori 83:594; Meier et al. 1997 Anticancer Res. 17(4B):2949; Meier et al., 1997 Anticancer Res. 17(4B):3019). Elevated serum CA125 may also accompany neuroblastoma (*e.g*., Hirokawa et al., 1998 Surg. Today 28:349), while elevated CEA and SCC, among others, may accompany colorectal cancer (Gebauer et al., 1997 Anticancer Res. 17(4B):2939).

The tumor associated antigen, mesothelin, defined by reactivity with monoclonal antibody K-1, is present on a majority of squamous cell carcinomas including epithelial ovarian, cervical, and esophageal tumors, and on mesotheliomas (Chang et al., 1992 Cancer Res. 52:181; Chang et al., 1992 Int. J. Cancer 50:373; Chang et al.,1992 Int. J. Cancer 51:548; Chang et al., 1996 Proc. Natl. Acad. Sci. USA 93:136; Chowdhury et al., 1998 Proc. Natl. Acad. Sci. USA 95:669). Using MAb K-1, mesothelin is detectable only as a cell-associated tumor marker and has not been found in soluble form in serum from ovarian cancer patients, or in medium conditioned by OVCAR-3 cells (Chang et al., 1992 Int. J. Cancer 50:373). Structurally related human mesothelin polypeptides, however, also include tumor-associated antigen polypeptides such as the distinct mesothelin related antigen (MRA) polypeptide, which is detectable as a naturally occurring soluble antigen in biological fluids from patients having malignancies (*see* WO 00/50900).

A tumor antigen may include a cell surface molecule. Tumor antigens of known structure and having a known or described function, include the following cell surface receptors: HER1 (*e.g*., GenBank Accession Nos. U48722, SEG_HEGFREXS, KO3193), HER2 (Yoshino et al., 1994 J. Immunol. 152:2393; Disis et al., 1994 Canc. Res. 54:16; see also, *e.g*., (GenBank Acc. Nos. X03363, M17730, SEG_HUMHER20), HER3 (*e.g.,* GenBank Acc. Nos. U29339, M34309), HER4 (Plowman et al., 1993 *Nature* 366:473; see also *e.g*., GenBank Acc. Nos. L07868, T64105), epidermal growth factor receptor (EGFR) (*e.g*., GenBank Acc. Nos. U48722, SEG_HEGFREXS, KO3193), vascular endothelial cell growth factor (*e.g*., GenBank No. M32977), vascular endothelial cell growth factor receptor (*e.g*., GenBank Acc. Nos. AF022375, 1680143, U48801, X62568), insulin-like growth factor-I (*e.g*., GenBank Acc. Nos. X00173, X56774, X56773, X06043, *see also* European Patent No. GB 2241703), insulin-like growth factor-II (*e.g.,* GenBank Acc. Nos. X03562, X00910, SEG_HUMGFIA, SEG_HUMGFI2, M17863, M17862), transferrin receptor (Trowbridge and Omary, 1981 Proc. Nat. Acad. USA 78:3039; see also *e.g.,* GenBank Acc. Nos. X0106O, M11507), estrogen receptor (*e.g.,* GenBank Acc. Nos. M38651, X03635, X99101, U47678, M12674), progesterone receptor (*e.g*., GenBank Acc. Nos. X51730, X69068, M15716), follicle stimulating hormone receptor (FSH-R) (*e.g*., GenBank Acc. Nos. Z34260, M65085), retinoic acid receptor (*e.g.,* GenBank Acc. Nos. L12060, M60909, X77664, X57280, X07282, X06538), MUC-1 (Barnes et al., 1989 Proc. Nat. Acad. Sci. USA 86:7159; see also *e.g*., GenBank Acc. Nos. SEG_MUSMUCIO, M65132, M64928) NY-ESO-1 (*e.g*., GenBank Acc. Nos. AJ003149, U87459), NA 17-A (*e*.*g*., European Patent No. WO 96/40039), Melan-A/MART-1 (Kawakami et al., 1994 Proc. Nat. Acad. Sci. USA 91:3515; see also *e.g.,* GenBank Acc. Nos. U06654, U06452), tyrosinase (Topalian et al., 1994 Proc. Nat. Acad. Sci. USA 91:9461; see also *e.g*., GenBank Acc. Nos. M26729, SEG_HUMTYR0, *see also* Weber et al., J. Clin. Invest (1998) 102:1258), Gp-100 (Kawakami et al., 1994 Proc. Nat. Acad. Sci. USA 91:3515; see also *e.g.,* GenBank Acc. No. S730O3, *see also* European Patent No. EP 668350; Adema et al., 1994 J. Biol. Chem. 269:20126), MAGE (van den Bruggen et al., 1991 Science 254:1643; see also *e.g,* GenBank Acc. Nos. U93163, AF064589, U66083, D32077, D32076, D32075, U10694, U10693, U10691, U10690, U10689, U10688, U10687, U10686, U10685, L18877, U10340, U10339, L18920, U03735, M77481), BAGE (*e.g.,* GenBank Acc. No. U19180, *see also* U.S. Patent Nos. 5,683,886 and 5,571,711), GAGE (*e.g.,* GenBank Acc. Nos. AF055475, AF055474, AF055473, U19147, U19146, U19145, U19144, U19143, U19142), any of the CTA class of receptors including in particular HOM-MEL-40 antigen encoded by the SSX2 gene (*e.g*., GenBank Acc. Nos. X86175, U90842, U90841, X86174), carcinoembyonic antigen (CEA, Gold and Freedman, 1985 J. Exp. Med. 121:439; *see also e.g.,* GenBank Acc. Nos. SEG_HUMCEA, M59710, M59255, M29540), and PyLT (*e.g*., GenBank Acc. Nos. J02289, J02038); p97 (melanotransferrin) (Brown et al., J. Immunol. 127:53 9-46 (1981); Rose et al., Proc. Natl. Acad. Sci. USA 83:1261-61 (1986)).

Additional tumor associated antigens include prostate surface antigen (PSA) (e.g., U.S. Patent No. 6,677,157; 6,673,545); β-human chorionic gonadotropin (β-HCG) (McManus et al., Cancer Res. 36:3476-81 (1976); Yoshimura et al., Cancer 73:2745-52 (1994); Yamaguchi et al., Br. J. Cancer 60:3 82-84 (1989): Marcillac et al., (1992); Alfthan et al., Cancer Res. 52:4628-33 (1992); Tormey et al., Cancer 39:2391-96 (1977); Tormey et al., Cancer 35:1095-1100 (1975)); glycosyltransferase β-1, 4-N-acetylgalactosaminyltransferase (GalNAc) (Hoon et al., Int. J. Cancer 43:857-62 (1989); Ando et al., Int. J. Cancer 40:12-17 (1987); Tsuchida et al., J. Natl. Cancer 78:45-54 (1987); Tsuchida et al., J. Natl. Cancer 78:55-60 (1987); Irie et al., in M. Torisu et al., eds., Basic Mechanisms and Clinical Treatment of Tumor Metastasis, 371-84 (Academic Press, Tokyo 1985)); MUC18 (Lehmann et al., Proc. Natl. Acad. Sci. USA 86:9891-95 (1989); Lehmann et al., Cancer Res. 47:841-45 (1987)); melanoma antigen gp75 (Vijayasardahi et al., J. Exp. Med. 171:1375-80 (1990); GenBank Accession Nos. X51455); human cytokeratin 8 (Pittman et al., (1993); GenBank Accession Nos. XM_092267; NM_002273); high molecular weight melanoma antigen (Natali et al., Cancer 59:55-63 (1987); keratin 19 (Datta et al., J. Clin. Oncol. 12:475-82 (1994); GenBank Accession Nos. NM_002276; BC007628). *See also* Pantel et al., Onkologie 18:394-401 (1995); Pelkey et al., Clin. Chem. 42:1369-81 (1996); Noguchi et al., Cancer 74:1595-1600 (1994); Schmitz-Drager et al., World J. Urol. 14:190-96 (1996).

Tumor antigens of interest include antigens regarded in the art as "cancer/testis" (CT) antigens that are immunogenic in subjects having a malignant condition (*see* Scanlan et al., Cancer Immun. 4:1 (2004) and references therein). CT antigens include at least 19 different families of antigens that contain one or more members and that are capable of inducing an immune response, including but not limited to MAGEA (CT1); BAGE (CT2); MAGEB (CT3); GAGE (CT4); SSX (CT5); NY-ESO-1 (CT6); MAGEC (CT7); SYCP1(C8); SPANXB1 (CT11.2); NA88 (CT18); CTAGE (CT21); SPA17 (CT22); OY-TES-1 (CT23); CAGE (CT26); HOM-TES-85 (CT28); HCA661 (CT30); NY-SAR-35 (CT38); FATE (CT43); and TPTE (CT44), some of which are described above (*id.*)*.*

Also contemplated by the invention are variants, derivatives, and fragments of a tumor antigen (or tumor-associated antigen). Such tumor antigen (or tumor-associated antigen) variants may contain one or more substitutions, deletions, additions and/or insertions. A tumor antigen variant has at least 70% similarity (preferably 70% sequence identity), 80% similarity (preferably 80% sequence identity), 85% similarity (preferably 85% sequence identity), and more preferably 90% similarity (more preferably 90% sequence identity) to the wildtype tumor antigen, more preferably 95% similarity (more preferably 95% sequence identity), and still more preferably a 98% similarity (still more preferably 98% sequence identity) to the wildtype tumor antigen (or tumor-associated antigen). As known in the art and described herein, "similarity" between two polypeptides is determined by comparing the amino acid sequence and conserved amino acid substitutions thereto of a first polypeptide to the sequence of a second polypeptide. Fragments or portions of the nucleic acid molecules encoding a tumor antigen (or tumor-associated antigen) of the present disclosure may be used to synthesize full-length polynucleotides of the present invention. As used herein, "% identity" refers to the percentage of identical amino acids situated at corresponding amino acid residue sequence positions when two or more polypeptide are aligned and their sequences analyzed using a gapped BLAST algorithm (*e.g*., Altschul et al., NucleicAcids Res. 25:3389 (1997)), which weights sequence gaps and sequence mismatches according to the default weightings provided by the National Institutes ofHealth/ NCBI database (Bethesda, MD; *see* [Internet: www.ncbi.nlm.nih.gov/cgi-bin/BLAST/nph-newblast].

### Cell Surface Polypeptides

A cell surface polypeptide, such as a cell surface CD83 polypeptide, and a cell surface expressed anti-immune receptor antibody, such as a cell surface form of an antibody or antigen-binding fragment thereof that specifically binds to an immune cell receptor, for instance an anti-CD137 scFv antibody, that localizes to the cell surface, may do so by virtue of having naturally present or artificially introduced structural features that target the polypeptide to a cell membrane, and in particular to a host cell plasma membrane, according to known membrane localization polypeptide motifs that may be naturally present or artificially introduced into the nucleic acid sequences encoding such a cell surface polypeptide (*e.g*., Nelson et al. 2001 Trends Cell Biol. 11:483; Ammon et al., 2002 Arch. Physiol. Biochem. 110:137; Kasai et al., 2001 J. Cell Sci. 114:3115; Watson et al., 2001 Am. J. Physiol. Cell Physiol. 281:C215; Chatterjee et al., 2000 J. Biol. Chem. 275:24013). A cell membrane as used herein may be any cellular membrane, and typically refers to membranes that are in contact with cytosolic components, including especially the plasma membrane and also including intracellular membrane bounded compartments such as intracellular vesicles, endosomes, lysosomes, receptosomes, ER-Golgi constituents and other organelles.

In other preferred embodiments, for example, recombinant expression constructs as provided herein that encode a cell surface polypeptide (including those that encode a cell surface anti-immune receptor antibody or a cell surface tumor antigen(or tumor-associated antigen)) also encode polypeptide sequences that direct the protein to be incorporated into a heterologous plasma membrane component, or to associate with a specific cytoplasmic or membrane component such as the cytoplasmic or transmembrane domains of a transmembrane cell surface receptor, or to be directed to a particular subcellular location by any of a variety of known intracellular protein sorting mechanisms with which those skilled in the art will be familiar. These and related embodiments are encompassed by the instant compositions and methods directed to targeting a polypeptide of interest to a predefined intracellular, membrane, or extracellular location.

Structural features that target a polypeptide to a cell membrane include by way of illustration and not limitation, secretory signal sequences, leader sequences, plasma membrane anchor domain polypeptides such as hydrophobic transmembrane domains (*e.g*., Heuck et al., 2002 Cell Biochem. Biophys. 36:89; Sadlish et al., 2002 Biochem J. 364:777; Phoenix et al., 2002 Mol. Membr. Biol. 19:1; Minke et al., 2002 Physiol. Rev. 82:429) or glycosylphosphatidylinositol attachment sites ("glypiation" sites, *e*.*g*., Chatterjee et al., 2001 Cell Mol. Life Sci. 58:1969; Hooper, 2001 Proteomics 1:748; Spiro, 2002 Glycobiol. 12:43R), cell surface receptor binding domains, extracellular matrix binding domains, or any other structural feature that causes the polypeptide, which may be synthesized as a fusion protein, to localize to the cell surface. Particularly preferred are polypeptides and fusion proteins that comprise a plasma membrane anchor domain that includes a transmembrane polypeptide domain, typically comprising a membrane spanning domain that includes a hydrophobic region capable of energetically favorable interaction with the phospholipid fatty acyl tails that form the interior of the plasma membrane bilayer. Such features are well known to those of ordinary skill in the art, who will further be familiar with methods for introducing nucleic acid sequences encoding these features into the subject expression constructs by genetic engineering and with routine testing of such constructs to verify cell surface localization of the product.

Accordingly, constructs encoding the cell surface polypeptides, cell surface forms of anti-immune receptor antibodies, and tumor antigens may include sequences that encode such polypeptides that are secreted, or that are not secreted, or that are targeted for localization to specific subcellular compartments (including membranes) within the cell. Nucleic acid sequences encoding peptides that direct intracellular sorting of newly synthesized polypeptides to secretory pathways or to residence in particular intracellular compartments are known and are within the scope of the present invention.

Thus, for example, nucleic acid recombinant expression constructs that encode a cell surface polypeptide (*e.g*., a cell surface CD83 polypeptide), an anti-immune receptor antibody or antigen-binding fragment thereof (*e.g*., a cell surface form of anti-CD137 scFv), and/or a tumor antigen (or tumor-associated antigen) may contain sequences encoding peptides that direct the encoded polypeptides to be incorporated into the plasma membrane, to be secreted from a cell via the classical ER-Golgi secretory pathway, to associate with a specific cytoplasmic component including the cytoplasmic domain of a transmembrane cell surface receptor, or to be directed to a particular subcellular location by a known intracellular protein sorting mechanism with which those skilled in the art will be familiar. Such intracellular protein sorting peptide sequences may also be present in ligands or nucleic acid binding domains that are provided by the present invention.

According to certain related embodiments, a plasma membrane anchor domain polypeptide comprises such a transmembrane domain polypeptide and also comprises a cytoplasmic tail polypeptide, which refers to a region or portion of the polypeptide sequence that contacts the cytoplasmic face of the plasma membrane and/or is in contact with the cytosol or other cytoplasmic components. A large number of cytoplasmic tail polypeptides are known that comprise the intracellular portions of plasma membrane transmembrane proteins. Discrete functions have been identified for many such polypeptides, including biological signal transduction (*e.g*., activation or inhibition of protein kinases, protein phosphatases, G-proteins, cyclic nucleotides and other second messengers, ion channels, secretory pathways, etc.), biologically active mediator release, stable or dynamic association with one or more cytoskeletal components, cellular differentiation, cellular activation, mitogenesis, cytostasis, apoptosis and the like (*e.g*., Maher et al., 2002 Immunol. Cell Biol. 80:131; E1 Far et al., 2002 Biochem J. 365:329; Teng et al., 2002 Genome Biol. 2REVIEWS:3012; Simons et al., 2001 Cell Signal 13:855; Furie et al., 2001 Thromb. Haemost. 86:214; Gaffen, 2001 Cytokine 14:63; Dittel, 2000 Arch. Immunol. Ther. Exp. (Warsz.) 48:381; Parnes et al., 2000 Immunol. Rev. 176:75; Moretta et al., 2000 Semin. Immunol. 12:129; Ben Ze'ev, 1999 Ann. N.Y. Acad. Sci. 886:37; Marsters et al., Recent Prog. Horm. Res. 54:225).

### Nucleic Acid Molecules

The nucleic acid molecules (polynucleotides) that encode a cell surface CD83 polypeptide, cell surface anti-immune receptor antibody, and/or tumor antigen (or tumor-associated antigen) for use according to the invention may include, but are not limited to only the coding sequence for the cell surface CD83 polypeptide; cell surface anti-immune receptor antibody, and/or tumor antigen polypeptide; the coding sequence for the cell surface CD83 polypeptide, cell surface anti-immune receptor antibody, and/or tumor antigen polypeptide and additional coding sequence; the coding sequence for the cell surface CD83 polypeptide, cell surface anti-immune receptor antibody, and/or tumor antigen (and optionally additional coding sequence) and non-coding sequence, such as introns or non-coding sequences 5' and/or 3' of the coding sequence for each of the cell surface CD83 polypeptide, cell surface anti-immune receptor antibody, and/or tumor antigen. Non-coding sequences for example may further include but need not be limited to one or more regulatory polynucleotide sequences that may be a regulated or regulatable promoter, enhancer, other transcription regulatory sequence, repressor binding sequence, translation regulatory sequence, or any other regulatory nucleotide sequence. Thus, the term "nucleic acid molecule (or polynucleotide) encoding" a cell surface CD83 polypeptide, cell surface anti-immune receptor antibody, and/or tumor antigen encompasses nucleic acid molecules that may include only coding sequence for a cell surface CD83 polypeptide, cell surface anti-immune receptor antibody, and/or tumor antigen, as well as nucleic acid molecules that include additional coding and/or non-coding sequence(s).

The present invention further relates to nucleic acid molecules (polynucleotides) that hybridize to polynucleotide sequences encoding cell surface CD83 polypeptide, cell surface anti-immune receptor antibody, and/or tumor antigen (or tumor-associated antigen) as provided herein, as incorporated by reference or as will be readily apparent to those familiar with the art, if at least 70%, preferably 80-85%, more preferably at least 90%, and still more preferably at least 92%, 94%, 95%, 96%, 97%, 98% or 99% nucleotide base sequence identity exists between the sequences. The present invention particularly relates to polynucleotides that hybridize under highly stringent conditions to the cell surface CD83 polypeptide, cell surface anti-immune receptor antibody, and/or tumor antigen encoding nucleic acids referred to herein. As used herein, the term "highly stringent conditions" means hybridization will occur only if at least 95% and preferably at least 97% identity is shared between the sequences. Exemplary high stringency conditions include using a buffer containing 0.1x SSPE or SSC and 0.1% SDS, at 65 °C. The polynucleotides that hybridize to cell surface CD83 polypeptide, cell surface anti-immune receptor antibody, and/or tumor antigen encoding nucleic acids referred to herein, in preferred embodiments, encode polypeptides which retain substantially the same biological function or activity as the CD83 polypeptide, cell surface anti-immune receptor antibody, and/or tumor antigen polypeptides encoded by the polynucleotides of the references cited herein.

The present invention also relates to polynucleotides that hybridize under suitable moderately stringent conditions, which include, for example, pre-washing in a solution of 5X SSC, 0.5% SDS, 1.0 mM EDTA (pH 8.0); hybridizing at 50 °C-70°C, 5X SSC for 1-16 hours; followed by washing once or twice at 22-65 °C for 20-40 minutes with one or more each of 2X, 0.5X and 0.2X SSC containing 0.05-0.1% SDS. For additional stringency, conditions may include a wash in 0.1X SSC and 0.1 % SDS at 50-60 °C for 15 minutes. As known to those having ordinary skill in the art, variations in stringency of hybridization conditions may be achieved by altering the time, temperature, and/or concentration of the solutions used for pre-hybridization, hybridization, and wash steps. Suitable conditions may also depend in part on the particular nucleotide sequences of the probe used, and of the blotted, proband nucleic acid sample. Accordingly, it will be appreciated that suitably stringent conditions can be readily selected without undue experimentation when a desired selectivity of the probe is identified, based on its ability to hybridize to one or more certain proband sequences while not hybridizing to certain other proband sequences.

The nucleic acid molecules of the present invention may be in the form of RNA (such as mRNA or synthetic RNA) or in the form of DNA, which DNA includes cDNA, genomic DNA, and synthetic DNA. The DNA may be double-stranded or single-stranded, and if single-stranded may be the coding strand or non-coding (anti-sense) strand. A coding sequence that encodes a cell surface CD83 polypeptide, cell surface anti-immune receptor antibody, and/or tumor antigen (or tumor-associated antigen) for use according to the invention may be identical to the coding sequence known in the art for any given cell surface CD83 polypeptide, cell surface anti-immune receptor antibody, and/or tumor antigen, or may be a different coding sequence, which, as a result of the redundancy or degeneracy of the genetic code, encodes the same cell surface CD83 polypeptide, cell surface anti-immune receptor antibody, and/or tumor antigen. The degeneracy of the genetic code is a well known principle in the molecular biology arts, and persons skilled in the art can readily substitute one codon for another that encodes the same amino acid.

Polynucleotides and oligonucleotides for use as described herein can be synthesized by any method known to those of skill in this art (*see, e*.*g*., WO 93/01286, U.S. Application Serial No. 07/723,454; U.S. Patent No. 5,218,088; U.S. Patent No. 5,175,269; U.S. Patent No. 5,109,124). Identification of oligonucleotides and polynucleotide sequences for use in the compositions provided by the present invention involves methods well known in the art. For example, the desirable properties, lengths, and other characteristics of useful oligonucleotides are well known. In certain embodiments, synthetic oligonucleotides and polynucleotide sequences may be designed that resist degradation by endogenous host cell nucleolytic enzymes by containing such linkages as phosphorothioate, methylphosphonate, sulfone, sulfate, ketyl, phosphorodithioate, phosphoramidate, phosphate esters, and other such linkages that have proven useful in antisense applications (*see, e.g.,* Agrwal et al., Tetrehedron Lett. 28:3539-42 (1987); Miller et al., J. Am. Chem. Soc. 93:6657-65 (1971); Stec et al., Tetrehedron Lett. 26:2191-94 (1985); Moody et al., Nucleic Acids Res. 12:4769-4782 (1989); Uznanski et al., Nucleic Acids Res. (1989); Letsinger et al., Tetrahedron 40:137-43 (1984); Eckstein, Annu. Rev. Biochem. 54:367-402 (1985); Eckstein, Trends Biol. Sci. 14:97-100 (1989); Stein In: Oligodeoxynucleotides. Antisense Inhibitors of Gene Expression, Cohen, Ed, Macmillan Press, London, pp. 97-117 (1989); Jager et al., Biochemistry 27:7237-46 (1988)).

A truncated CD83 polypeptide may be any CD83 polypeptide molecule that comprises less than a full length version of the CD83 polypeptide. Similarly, a truncated anti-immune receptor antibody or a truncated tumor antigen (or tumor-associated antigen) comprises lesser amino acids than the full length molecules. Truncated molecules provided by the present invention may include truncated biological polymers, and in preferred embodiments of the invention such truncated molecules may be truncated nucleic acid molecules or truncated polypeptides. Truncated nucleic acid molecules have less than the full length nucleotide sequence of a known or described nucleic acid molecule, wherein such a known or described nucleic acid molecule may be a naturally occurring, a synthetic or a recombinant nucleic acid molecule, so long as one skilled in the art would regard it as a full length molecule. Thus, for example, truncated nucleic acid molecules that correspond to a gene sequence contain less than the full length gene where the gene comprises coding and non-coding sequences, promoters, enhancers and other regulatory sequences, flanking sequences and the like, and other functional and non-functional sequences that are recognized as part of the gene. In another example, truncated nucleic acid molecules that correspond to a mRNA sequence contain less than the full length mRNA transcript, which may include various translated and non-translated regions as well as other functional and non-functional sequences. In other preferred embodiments, truncated molecules are polypeptides that comprise less than the full length amino acid sequence of a particular protein.

Truncated molecules that are linear biological polymers such as nucleic acid molecules or polypeptides may have one or more of a deletion from either terminus of the molecule or a deletion from a non-terminal region of the molecule; such deletions may be deletions of 1-1500 contiguous nucleotide or amino acid residues, preferably 1-500 contiguous nucleotide or amino acid residues, more preferably 1-300, 1-150, or 1-120 contiguous nucleotide or amino acid residues, and more preferably 1-100,1-75,1-50, or 1-25 contiguous nucleotide or amino acid residues.

The present invention further relates to variants of the herein referenced polynucleotides that encode fragments, analogs and/or derivatives of a cell surface CD83 polypeptide, cell surface anti-immune receptor antibody, or tumor antigen (or tumor-associated antigen). The variants of the nucleic acids encoding a cell surface CD83 polypeptide, cell surface anti-immune receptor antibody, or tumor antigen may be naturally occurring allelic variants of the polynucleotides or non-naturally occurring variants. As is known in the art, an allelic variant is an alternate form of a nucleic acid sequence that may have at least one of a substitution, a deletion or an addition of one or more nucleotides, any of which does not substantially alter the function of the encoded cell surface CD83 fusion polypeptide, cell surface anti-immune receptor antibody, or tumor antigen.

Variants and derivatives of a cell surface CD83 polypeptide, cell surface anti-immune receptor antibody, or tumor antigen (or tumor-associated antigen) may be obtained by mutations of nucleotide sequences encoding such polypeptides. Alterations of the native amino acid sequences may be accomplished by any of a number of conventional methods. Mutations can be introduced at particular loci by synthesizing oligonucleotides containing a mutant sequence that is flanked by restriction sites, enabling ligation to fragments of the native sequence. Following ligation, the resulting reconstructed sequence encodes an analog having the desired amino acid insertion, substitution, or deletion.

Alternatively, oligonucleotide-directed site-specific mutagenesis procedures can be employed to provide an altered encoding polynucleotide wherein predetermined codons can be altered by substitution, deletion, or insertion. Exemplary methods of making such alterations are disclosed by Walder et al. (Gene 42:133 (1986); Bauer et al. (Gene 37:73 (1985); Craik (BioTechniques, January 1985, 12-19); Smith et al. (Genetic Engineering: Principles and Methods BioTechniques, January 1985, 12-19); Smith et al. (Genetic Engineering: Principles and Methods, Plenum Press,1981); Kunkel (Proc. Natl. Acad. Sci. USA 82:488, 1985); Kunkel et al. (Methods Enzymol. 154:367, 1987); and U.S. Patent Nos. 4,518,584 and 4,737,462.

As an example, modification of DNA may be performed by site-directed mutagenesis of DNA encoding the polypeptide of interest combined with the use of DNA amplification methods using primers to introduce and amplify alterations in the DNA template, such as PCR splicing by overlap extension (SOE). Site-directed mutagenesis is typically effected using a phage vector that has single- and double-stranded forms, such as M13 phage vectors, which are well-known and commercially available. Other suitable vectors that contain a single-stranded phage origin of replication may be used (*see, e.g.,* Veira et al., Methods Enzymol. 15:3 (1987)). In general, site-directed mutagenesis is performed by preparing a single-stranded vector that encodes the protein of interest (*e.g*., a cell surface CD83 polypeptide, cell surface anti-immune receptor antibody, or tumor antigen). An oligonucleotide primer that contains the desired mutation within a region of homology to the DNA in the single-stranded vector is annealed to the vector followed by addition of a DNA polymerase, such as *E. coli* DNA polymerase I (Klenow fragment), which uses the double stranded region as a primer to produce a heteroduplex in which one strand encodes the altered sequence and the other the original sequence. The heteroduplex is introduced into appropriate bacterial cells and clones that include the desired mutation are selected. The resulting altered DNA molecules may be expressed recombinantly in appropriate host cells to produce the modified protein.

The described mutagenesis methods and other methods known in the art such as alanine scanning mutagenesis, error prone polymerase chain reaction mutagenesis, and oligonucleotide-directed mutagenesis, some of which generate tens of thousands of mutants, are well known and have been used extensively in the art (*see, e.g.,* Huse et al., Intern. Rev. Immunol. 10:129-37 (1993); Dickinson et al., Proc. Natl. Acad. Sci. USA 93:14379-84 (1996); Goettlinger et al., Int. Conf. AIDS 8:Mo5 (1992); Vajdos et al., J. Mol. Biol. 320:415-28 (2002), which are enclosed for the Examiner's convenience; *see generally* Sambrook et al. Molecular Cloning: A Laboratory Manual, 3rd ed., Cold Spring Harbor Laboratory Press, NY (2001)). These methods may be used to identify which amino acids in a given polypeptide sequence are important to a particular function of the polypeptide and, consequently, also identify amino acids at particular positions that are not important or required for a particular function.

While persons skilled in the art may appreciate that a single amino acid change may diminish, substantially impair, or abrogate a particular function, persons skilled in the art also understand that many amino acid residues can be substituted without any loss of function, and in some cases, an amino acid deletion, insertion, or substitution increases function. Using the mutagenesis methods known in the art, functions ofproteins can be mapped to specific structural domains, undesirable activities of enzymes can be eliminated, and their desirable catalytic and physical properties can be enhanced. (*See* Larsson et al., Protein Eng. Des. Sel. 17:49-55 (2004); Huse et al., Intern. Rev. Immunol. 10:129-37 (1993); Yelton et al., J. Immunol. 155:1994-2004 (1995); Barbas et al., Trends Biotechnol. 14:230-24 (1996); *see generally* Woolfson, Curr. Opin. Struct. Biol. 11:464-71 (2001); James D. Watson et al., ed., The Benjamin/Cummings Publishing Co., (Menlo Park, CA) (4^{th} ed. 1987)). To determine whether perturbations in protein structure caused by a substitution, deletion, or insertion of an amino acid affect the structure of the polypeptide such that one or more functions of the polypeptide are affected, persons skilled in the art can use assays for assessing folding of the protein of interest *(see, e.g.,* Sambrook et al., *supra*). Such assays commonly include, for example, the ability of the protein to react with mono- or polyclonal antibodies that are specific for native or unfolded epitopes, the proper movement and posttranslational modification of the protein within a cell, the retention of catalytic or ligand-binding functions, the sensitivity or resistance of the mutant protein to digestion with proteases, and other functional assays that characterize a particular polypeptide *(see* Sambrook et al., *supra*).

Equivalent DNA constructs that encode various additions or substitutions of amino acid residues or sequences, or deletions of terminal or internal residues or sequences not needed for biological activity are also encompassed by the invention. For example, sequences encoding Cys residues that are not essential for biological activity can be altered to cause the Cys residues to be deleted or replaced with other amino acids, preventing formation of incorrect intramolecular disulfide bridges upon renaturation.

### Recombinant Expression Constructs

The present invention also relates to vectors and to constructs prepared from known vectors that include polynucleotides of the present invention, and in particular to "recombinant expression constructs" that include any polynucleotide encoding a cell surface CD83 polypeptide, cell surface anti-immune receptor antibody, and/or tumor antigen polypeptide (or tumor-associated antigen) according to the invention as provided above; to host cells that are genetically engineered with vectors and/or constructs of the invention, and to methods of administering compositions comprising polynucleotide sequences encoding such a cell surface CD83 polypeptide, cell surface anti-immune receptor antibody, and/or tumor antigen polypeptide of the invention, or fragments, derivatives, or variants thereof, by recombinant techniques. The present invention provides polynucleotides encoding polypeptides that comprise, for example, a coding sequence of CD83, or a coding sequence of a cell surface anti-immune cell receptor antibody fused to coding sequences for a polypeptide tail that comprises a transmembrane and/or cytoplasmic domains. A cell surface CD83 polypeptide, cell surface anti-immune receptor antibody, and/or tumor antigen can be expressed in virtually any host cell under the control of appropriate promoters, depending on the nature of the construct (*e.g*., type of promoter, as described above), and on the nature of the desired host cell (*e.g*., whether post-mitotic terminally differentiated or actively dividing; *e.g*., whether the expression construct occurs in host cell as an episome or is integrated into host cell genome). Appropriate cloning and expression vectors for use with prokaryotic and eukaryotic hosts are described by Sambrook, et al., Molecular Cloning: A Laboratory Manual, Third Edition, Cold Spring Harbor, NY (2001)).

Typically, the constructs are derived from plasmid vectors. A preferred construct is a modified pNASS vector (Clontech, Palo Alto, CA), which has nucleic acid sequences encoding an ampicillin resistance gene, a polyadenylation signal, and a T7 promoter site. Other suitable mammalian expression vectors are well known (*see, e.g.,* Ausubel et al., 1995; Sambrook et al., *supra*; *see also, e.g.,* catalogues from Invitrogen Life Technologies, San Diego, CA; Novagen, Madison, WI; Pharmacia, Piscataway, NJ; and others). Presently preferred constructs are prepared from a pLNCX plasmid (BD Biosciences Clontech, Palo Alto, CA).

Generally, recombinant expression vectors will include origins of replication and selectable markers permitting transformation of the host cell, and a promoter derived from a highly-expressed gene to direct transcription of a downstream structural sequence, as described above. The heterologous structural sequence is assembled in appropriate phase with translation initiation and termination sequences. Thus, for example, the cell surface CD83 polypeptide, cell surface anti-immune receptor antibody, and/or tumor antigen (or tumor-associated antigen) encoding nucleic acids as provided herein may be included in any one of a variety of expression vector constructs as a recombinant expression construct for expressing a cell surface CD83 polypeptide, cell surface anti-immune receptor antibody, and/or tumor antigen in a host cell. In preferred embodiments the constructs are included in compositions that are administered in vivo. Such vectors and constructs include chromosomal; nonchromosomal; and synthetic DNA sequences, *e.g*., derivatives of SV40; bacterial plasmids; phage DNA; yeast plasmids; vectors derived from combinations of plasmids; and phage DNA; viral DNA, such as vaccinia, adenovirus, fowl pox virus, and pseudorabies; or replication deficient retroviruses as described below. However, any other vector may be used for preparation of a recombinant expression construct, and in preferred embodiments such a vector will be replicable and viable in the host.

The appropriate polynucleotide sequence(s) may be inserted into the vector by a variety of procedures. In general, the polynucleotide DNA sequence is inserted into an appropriate restriction endonuclease site(s) by procedures known in the art. Standard techniques for cloning, DNA isolation, amplification, and purification, for enzymatic reactions involving DNA ligase, DNA polymerase, restriction endonucleases and the like, and various separation techniques are those known and commonly employed by those skilled in the art. A number of standard techniques are described, for example, in Ausubel et al. (Current Protocols in Molecular Biology, Greene Publ. Assoc. Inc. & John Wiley & Sons, Inc., Boston, MA (1993); Sambrook et al. 2001, *supra*; Sambrook et al., Molecular Cloning, Second Ed., (Cold Spring Harbor Laboratory, Plainview, NY 1989); Maniatis et al., Molecular Cloning, Cold Spring Harbor Laboratory, Plainview, NY 1982); Glover ed., DNA Cloning Vol. I and II, IRL Press, Oxford, UK 1985); Hames and Higgins eds. (Nucleic Acid Hybridization, IRL Press, Oxford, UK 1985); and elsewhere.

The DNA sequence in the expression vector is operatively linked to at least one appropriate expression control sequences (*e.g*., a constitutive promoter or a regulated promoter) to direct mRNA synthesis. Representative examples of such expression control sequences include promoters of eukaryotic cells or their viruses, as described above. Promoter regions can be selected from any desired gene using CAT (chloramphenicol transferase) vectors or other vectors with selectable markers. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art, and preparation of certain particularly preferred recombinant expression constructs comprising at least one promoter or regulated promoter operatively linked to a nucleic acid encoding a cell surface CD83 polypeptide, cell surface anti-immune receptor antibody, and/or tumor antigen polypeptide is described herein.

Transcription of the DNA encoding the polypeptides of the present invention by higher eukaryotes may be increased by inserting an enhancer sequence into the vector. Enhancers are *cis*-acting elements of DNA, usually about from 10 to 300 base pairs that act on a promoter to increase its transcription. Examples including the SV40 enhancer on the late side of the replication origin base pairs 100 to 270, a cytomegalovirus early promoter enhancer, the polyoma enhancer on the late side of the replication origin, and adenovirus enhancers.

For purposes of mutagenesis or manipulation of the encoding polynucleotides or for isolation of the encoding polynucleotides of interest, nucleic acid molecules may be introduced into a host organism. Also for purposes of expressing a cell surface CD83 polypeptide, cell surface anti-immune receptor antibody, and/or tumor antigen (or tumor-associated antigen) for use in the compositions and methods of the present invention, the polynucleotide encoding sequences are introduced into a ho st organism. Host organisms include those organisms in which recombinant production of a cell surface CD83 polypeptide, cell surface anti-immune receptor antibody, and/or tumor antigen products encoded by the recombinant constructs of the present invention compositions may occur, such as bacteria (for example, *E. coli*), yeast (for example, *Saccharomyces cerevisiae* and *Pichia pastoris*), insect cells, and mammals, including *in vitro* and *in vivo* expression. Host organisms thus may include organisms for the construction, propagation, expression or other steps in the production of the compositions provided herein. Hosts also include subjects in which an immune response is induced, as described above. Presently preferred host organisms are *E. coli* bacterial strains; inbred murine strains and murine cell lines; and human cells, subjects, and cell lines.

The DNA construct encoding the desired cell surface CD83 polypeptide, cell surface anti-immune receptor antibody, and/or tumor antigen (or tumor-associated antigen) is introduced into a plasmid for expression in an appropriate host. In certain embodiments, the host is a bacterial host. The sequence encoding the polypeptide of interest is preferably codon-optimized for expression in the particular host. Thus, for example, if a human cell surface CD83 polypeptide, cell surface anti-immune receptor antibody, and/or tumor antigen is expressed in bacteria, the codons would be optimized for bacterial usage. For small coding regions, the gene can be synthesized as a single oligonucleotide. For larger proteins, splicing of multiple oligonucleotides, mutagenesis, or other techniques known to those in the art may be used. The sequences of nucleotides in the plasmids that are regulatory regions, such as promoters and operators, are operationally associated with one another for transcription. The sequence of nucleotides encoding a cell surface CD83 polypeptide or an cell surface anti-immune receptor antibody (fusion polypeptide), and/or tumor antigen that is a fusion polypeptide may also include DNA encoding a secretion signal, whereby the resulting polypeptide is a precursor protein. The resulting processed protein may be recovered from the periplasmic space or the fermentation medium. A polypeptide expressed in bacteria may also be recovered from inclusion bodies that form within the bacteria according to methods known in the art. The inclusion bodies can be isolated and then the polypeptide solubilized, often by denaturation techniques, followed by renaturation and recovery of the polypeptide of interest.

In preferred embodiments, the DNA plasmids also include a transcription terminator sequence. As used herein, a "transcription terminator region" is a sequence that signals transcription termination. The entire transcription terminator may be obtained from a protein-encoding gene, which may be the same or different from the inserted cell surface CD83 polypeptide, cell surface anti-immune receptor antibody, and/or tumor antigen (or tumor-associated antigen) encoding gene or the source of the promoter. Transcription terminators are optional components of the expression systems herein, but are employed in preferred embodiments.

The plasmids used herein include a promoter in operative association with the DNA encoding the protein or polypeptide of interest and are designed for expression of proteins in a suitable host as described above (*e.g*., bacteria, yeast, mouse, or human) depending upon the desired use of the plasmid (*e.g*., administration of a composition containing cell surface CD83 polypeptide, cell surface anti-immune receptor antibody, and/or tumor antigen encoding sequences, or of a composition containing expressed cell surface CD83 polypeptide, cell surface anti-immune receptor antibody, and/or tumor antigen products, for example tumor cells expressing such polypeptides). Suitable promoters for expression ofproteins and polypeptides herein are widely available and are well known in the art (including a human CD83 promoter, *see* GenBank Accession No. AJ427415). Inducible promoters or constitutive promoters that are linked to regulatory regions are preferred. Such promoters include, but are not limited to, the T7 phage promoter and other T7-like phage promoters, such as the T3, T5 and SP6 promoters, the trp, lpp, and lac promoters, such as the lacUV5, from *E. coli*; the P10 or polyhedrin gene promoter of baculovirus/insect cell expression systems (*see, e.g.,* U.S. Patent Nos. 5,243,041, 5,242,687, 5,266,317, 4,745,051, and 5,169,784) and inducible promoters from other eukaryotic expression systems. For expression of the proteins such promoters are inserted in a plasmid in operative linkage with a control region such as the lac operon.

Promoter regions include those that are inducible and functional in E. *coli.* Examples of suitable inducible promoters and promoter regions include, but are not limited to the *E. coli* lac operator responsive to isopropyl β-D-thiogalactopyranoside (IPTG; *see* Nakamura et al., Cell 18:1109-1117 (1979); the metallothionein promoter metal-regulatory-elements responsive to heavy-metal (e.g., zinc) induction (*see*, *e.g*., U.S. Patent No. 4,870,009 to Evans et al.); the phage T71ac promoter responsive to IPTG (*see, e.g*., U.S. Patent No. 4,952,496; and Studier et al., Methods Enzymol. 185:60-89 (1990)); and the TAC promoter.

The plasmids may optionally include a selectable marker gene or genes that are functional in the host. A selectable marker gene includes any gene that confers a phenotype on bacteria that allows transformed bacterial cells to be identified and selectively grown from among a vast majority of untransformed cells. Suitable selectable marker genes for bacterial hosts, for example, include the ampicillin resistance gene (Amp^{r}), tetracycline resistance gene (Tc^{r}) and the kanamycin resistance gene (Kan^{r}). The kanamycin resistance gene is presently preferred.

The plasmids may also include DNA encoding a signal for secretion of the operatively linked protein. Secretion signals suitable for use are widely available and are well known in the art. Prokaryotic and eukaryotic secretion signals functional in *E. coli* may be employed. The presently preferred secretion signals include, but are not limited to, those encoded by the following *E. coli* genes: *ompA*, *ompT*, *ompF*, *ompC*, *beta-lactamase*, and *alkaline phosphatase,* and the like (von Heijne, J. Mol. Biol. 184:99-105, 1985). In addition, the bacterial pelB gene secretion signal (Lei et al., J. Bacteriol. 169:4379, 1987), the phoA secretion signal, and the cek2 functional in insect cells may be employed. The most preferred secretion signal is the *E. coli* ompA secretion signal. Other prokaryotic and eukaryotic secretion signals known to those of skill in the art may also be employed *(see, e.g*., von Heijne, J. Mol. Biol. 184:99-105, 1985). Using the methods described herein, one of skill in the art can substitute secretion signals that are functional in yeast, insect, or mammalian cells to secrete proteins from those cells, respectively.

Preferred plasmids for transformation of *E. coli* cells include the pET expression vectors (*e.g*., pET-11a, pET-12a-c, pET-15b; *see* U.S. Patent No. 4,952,496; available from Novagen, Madison, WI). Other preferred plasmids include the pKK plasmids, particularly pKK 223-3, which contain the tac promoter (Brosius et al., Proc. Natl. Acad. Sci. 81:6929, 1984; Ausubel et al., Current Protocols in Molecular Biology*;* U.S. Patent Nos. 5,122,463, 5,173,403, 5,187,153, 5,204,254, 5,212,058, 5,212,286, 5,215,907, 5,220,013, 5,223,483, and 5,229,279). Plasmid pKK has been modified by replacement of the ampicillin resistance gene with a kanamycin resistance gene. (Available from Pharmacia; obtained from pUC4K, *see, e.g.,* Vieira et al. (Gene 19:259-68, 1982; and U.S. Patent No. 4,719,179.) Baculovirus vectors, such as pBlueBac (also called pJVETL and derivatives thereof), particularly pBlueBac III (*see, e.g.,* U.S. Patent Nos. 5,278,050, 5,244,805, 5,243,041, 5,242,687, 5,266,317, 4,745,051, and 5,169,784; available from Invitrogen Life Technologies, San Diego) may also be used for expression of the polypeptides in insect cells. Other plasmids include the pIN-IIIompA plasmids, such as pIN-IIIompA2 *(see* U.S. Patent No. 4,575,013; *see also* Duffaud et al., Methods Enzymol. 153:492-507, 1987).

Preferably, the DNA molecule is replicated in bacterial cells, preferably in *E. coli.* The preferred DNA molecule also includes a bacterial origin of replication, to ensure the maintenance of the DNA molecule from generation to generation of the bacteria. In this way, large quantities of the DNA molecule can be produced by replication in bacteria. Preferred bacterial origins of replication include, but are not limited to, the f1 -ori and col E1 origins of replication. Preferred hosts contain chromosomal copies of DNA encoding T7 RNA polymerase operably linked to an inducible promoter, such as the lacUV promoter (*see* U.S. Patent No. 4,952,496). Such hosts include, but are not limited to, lysogens *E. coli* strains HMS174(DE3)pLysS, BL21(DE3)pLysS, HMS174(DE3) and BL21 (DE3). Strain BL21 (DE3) is preferred. The pLys strains provide low levels of T7 lysozyme, a natural inhibitor of T7 RNA polymerase.

The nucleic acid including DNA molecules provided may also contain a gene coding for a repressor protein. The repressor protein is capable of repressing the transcription of a promoter that contains sequences of nucleotides to which the repressor protein binds. The promoter can be de-repressed by altering the physiological conditions of the cell. For example, the alteration can be accomplished by adding to the growth medium a molecule that inhibits the ability to interact with the operator or with regulatory proteins or other regions of the DNA or by altering the temperature of the growth media. Preferred repressor proteins include, but are not limited to the *E. coli* lacI repressor responsive to IPTG induction, the temperature sensitive λ cI857 repressor, and the like. The *E. coli* lacI repressor is preferred.

In general, recombinant constructs of the subject invention compositions will also contain elements necessary for transcription and translation. In particular, such elements are preferred when the composition comprises a recombinant expression construct containing polynucleotide sequences encoding a cell surface CD83 polypeptide, cell surface anti-immune receptor antibody, and/or tumor antigen (or tumor-associated antigen) for expression in the host in which an immune response is desired. In certain embodiments of the present invention, cell type preferred or cell type specific expression of a cell surface CD83 polypeptide, cell surface anti-immune receptor antibody, and/or tumor antigen encoding sequence may be achieved by placing the polynucleotide sequence under regulation of a promoter. The choice of the promoter will depend upon the cell type to be transformed, transfected or transduced, and the degree or type of control desired. Promoters can be constitutive or active and may further be cell type specific, tissue specific, individual cell specific, event specific, temporally specific, or inducible. Cell-type specific promoters and event type specific promoters are preferred. Examples of constitutive or nonspecific promoters include the SV40 early promoter (U.S. Patent No. 5,118,627), the SV40 late promoter (U.S. Patent No. 5,118,627), CMV early gene promoter (U.S. Patent No. 5,168,062), and adenovirus promoter. In addition to viral promoters, cellular promoters are also amenable within the context of this invention. In particular, cellular promoters for the so-called housekeeping genes are useful. Viral promoters are preferred, because generally they are stronger promoters than cellular promoters. Promoter regions have been identified in the genes of many eukaryotes including higher eukaryotes, such that suitable promoters for use in a particular host can be readily selected by those skilled in the art.

Inducible promoters may also be used. These promoters include MMTV LTR (PCT WO 91/13160), inducible by dexamethasone; metallothionein promoter, inducible by heavy metals; and promoters with cAMP response elements, inducible by cAMP. By using an inducible promoter, the nucleic acid sequence encoding a cell surface CD83 polypeptide, cell surface anti-immune receptor antibody, and/or tumor antigen may be delivered to a cell by the subject invention compositions and will remain quiescent until the addition of the inducer. This allows further control on the timing of production of the gene product.

Event-type specific promoters are active or up-regulated only upon the occurrence of an event, such as tumorigenicity or viral infection. The HIV LTR is a well known example of an event-specific promoter. The promoter is inactive unless the *tat* gene product is present, which occurs upon viral infection. Some event-type promoters are also tissue-specific.

Additionally, promoters that are coordinately regulated with a particular cellular gene may be used. For example, promoters of genes that are coordinately expressed when a particular cell surface CD83 polypeptide, cell surface anti-immune receptor antibody, and/or tumor antigen (or tumor-associated antigen) gene is expressed may be used *(see, e.g.,* human CD83 promoter, GenBank Accession No. AF427415). This type of promoter is especially useful when one knows the pattern of gene expression relevant to induction of an immune response in a particular tissue of the immune system, so that specific immunocompetent cells within that tissue may be activated or otherwise recruited to participate in an immune response.

In addition to the promoter, repressor sequences, negative regulators, or tissue-specific silencers may be inserted to reduce non-specific expression of a cell surface CD83 polypeptide, cell surface anti-immune receptor antibody, and/or tumor antigen (or tumor-associated antigen) in certain situations, such as, for example, a host that is transiently immunocompromised as part of a therapeutic strategy. Multiple repressor elements may be inserted in the promoter region. Repression of transcription is independent on the orientation of repressor elements or distance from the promoter. One type of repressor sequence is an insulator sequence. Such sequences inhibit transcription (Dunaway et al., Mol. Cell Biol. 17:182-89 (1997); Gdula et al., Proc. Natl. Acad. Sci. USA 93:9378-83 (1996), Chan et al., J. Virol. 70: 5312-28, 1996; Scott and Geyer, EMBO J. 14:6258-67 (1995); Kalos and Fournier, Mol. Cell Biol. 15:198-207 (1995); Chung et al., Cell 74: 505-14 (1993)) and will silence background transcription.

Repressor elements have also been identified in the promoter regions of the genes for type II (cartilage) collagen, choline acetyltransferase, albumin (Hu et al., J. Cell Growth Differ. 3:577-88 (1992); phosphoglycerate kinase (PGK-2) (Misuno et al., Gene 119:293-97,1992), and in the 6-phosphofructo-2-kinase/fructose-2, 6-bisphosphatase gene (Lemaigre et al., Mol. Cell Biol. 11:1099-1106)). Furthermore, the negative regulatory element Tse-1 has been identified in a number of liver specific genes and has been shown to block cAMP response element- (CRE) mediated induction of gene activation in hepatocytes (Boshart et al., Cell 61:905-16, (1990)).

In preferred embodiments, elements that increase the expression of the desired product are incorporated into the construct. Such elements include internal ribosome binding sites (IRES), which increase translation efficiency (Wang and Siddiqui, Curr. Top. Microbiol. Immunol. 203:99, 1995; Ehrenfeld and Semler, Curr. Top. Microbiol Immunol. 203:65 (1995); Rees et al., Biotechniques 20:102 (1996); Sugimoto et al., Biotechnology 12:694 (1994)). Other sequences may also enhance expression. For some genes, sequences especially at the 5' end inhibit transcription and/or translation. These sequences are usually palindromes that can form hairpin structures. Any such sequences in the nucleic acid to be delivered are generally deleted. Expression levels of the transcript or translated product are assayed to confirm or ascertain which sequences affect expression. Transcript levels may be assayed by any known method, including Northern blot hybridization, RNase probe protection and the like. Protein levels may be assayed by any known method, including ELISA, western blot, immunocytochemistry, or other well known techniques.

Other elements may be incorporated into the constructs of the present invention. In preferred embodiments, the construct includes a transcription terminator sequence, including a polyadenylation sequence, splice donor and acceptor sites, and an enhancer. Other elements useful for expression and maintenance of the construct in mammalian cells or other eukaryotic cells may also be incorporated (e.g., origin of replication). Because the constructs are conveniently produced in bacterial cells, elements that are necessary for, or that enhance, propagation in bacteria are incorporated. Such elements include an origin of replication, a selectable marker and the like.

As provided herein, an additional level of controlling the expression of polynucleotides encoding a cell surface CD83 polypeptide, cell surface anti-immune receptor antibody, and/or tumor antigen (or tumor-associated antigen) delivered to cells using the constructs of the invention compositions may be provided by simultaneously delivering two or more differentially regulated nucleic acid constructs. The use of such a multiple nucleic acid construct approach may permit coordinated regulation of an immune response such as, for example, spatiotemporal coordination that depends on the cell type and/or presence of another expressed encoded component. Those familiar with the art will appreciate that multiple levels of regulated gene expression may be achieved in a similar manner by selection of suitable regulatory sequences, including but not limited to promoters, enhancers and other well known gene regulatory elements.

As provided herein, in certain embodiments the vector may be a viral vector such as a retroviral vector (Miller et al., BioTechniques 7:980 (1989); Coffin and Varmus, Retroviruses, Cold Spring Harbor Laboratory Press, NY 1996). For example, retroviruses from which the retroviral plasmid vectors may be derived include, but are not limited to, Moloney murine leukemia virus, spleen necrosis virus, retroviruses such as Rous sarcoma virus, Harvey sarcoma virus, avian leukosis virus, gibbon ape leukemia virus, human immunodeficiency virus, adenovirus, myeloproliferative sarcoma Virus, and mammary tumor virus.

Retroviruses are RNA viruses that can replicate and integrate into the genome of a host cell via a DNA intermediate. This DNA intermediate, or provirus, may be stably integrated into the host cell DNA. According to certain embodiments of the present invention, a composition may comprise a retrovirus into which a foreign polynucleotide sequence that encodes a foreign protein is incorporated in place of normal retroviral RNA. When retroviral RNA enters a host cell coincident with infection, the foreign gene is also introduced into the cell, and may then be integrated into host cell DNA as if it were part of the retroviral genome. Expression of this foreign gene within the host results in expression of the foreign protein.

Most retroviral vector systems that have been developed for gene therapy are based on murine retroviruses. Such retroviruses exist in two forms, as free viral particles referred to as virions, or as proviruses integrated into host cell DNA. The virion form of the virus contains the structural and enzymatic proteins of the retrovirus (including the enzyme reverse transcriptase), two RNA copies of the viral genome, and portions of the source cell plasma membrane containing viral envelope glycoprotein. The retroviral genome is organized into four main regions: the Long Terminal Repeat (LTR), which contains *cis*-acting elements necessary for the initiation and termination of transcription and is situated both 5' and 3' of the coding genes, and the three coding genes *gag, pol,* and env. These three genes *gag, pol,* and *env* encode, respectively, internal viral structures, enzymatic proteins (such as integrase), and the envelope glycoprotein (designated gp70 and p15e) that confers infectivity and host range specificity of the virus, as well as the "R" peptide of undetermined function.

Separate packaging cell lines and vector producing cell lines have been developed because of safety concerns regarding the uses of retroviruses, including their use in compositions as provided by the present invention. Briefly, this methodology employs the use of two components, a retroviral vector and a packaging cell line (PCL). The retroviral vector contains long terminal repeats (LTRs), the foreign DNA to be transferred, and a packaging sequence (y). This retroviral vector will not reproduce by itself because the genes that encode structural and envelope proteins are not included within the vector genome. The PCL contains genes encoding the *gag, pol,* and *env* proteins, but does not contain the packaging signal "y". Thus, a PCL can only form empty virion particles by itself. Within this general method, the retroviral vector is introduced into the PCL, thereby creating a vector-producing cell line (VCL). This VCL manufactures virion particles containing only the retroviral vector's (foreign) genome, and therefore has previously been considered to be a safe retrovirus vector for therapeutic use.

"Retroviral vector construct" refers to an assembly that is, within preferred embodiments of the invention, capable of directing the expression of a sequence(s) or gene(s) of interest, such as cell surface CD83 polypeptide, cell surface anti-immune receptor antibody, or tumor antigen (or tumor-associated antigen) encoding nucleic acid sequences. Briefly, the retroviral vector construct must include a 5' LTR, a tRNA binding site, a packaging signal, an origin of second strand DNA synthesis, and a 3'LTR. A wide variety of heterologous sequences may be included within the vector construct, including for example, sequences which encode a protein (*e.g*., cytotoxic protein, disease-associated antigen, immune accessory molecule, or replacement gene), or which are useful as a molecule itself (*e.g*., as a ribozyme, antisense sequence, or RNAi).

Retroviral vector constructs of the present invention may be readily constructed from a wide variety of retroviruses, including for example, B, C, and D type retroviruses as well as spumaviruses and lentiviruses (*see, e.g.,* RNA Tumor Viruses, Second Edition, Cold Spring Harbor Laboratory, 1985). Such retroviruses may be readily obtained from depositories or collections such as the American Type Culture Collection (ATCC; Rockville, Maryland), or isolated from known sources using commonly available techniques. Any of the above retroviruses may be readily utilized in order to assemble or construct retroviral vector constructs, packaging cells, or producer cells of the present invention given the disclosure provided herein, and standard recombinant techniques (*e.g*., Sambrook et al., *supra*).

Suitable promoters for use in viral vectors generally may include, but are not limited to, the retroviral LTR; the SV40 promoter; and the human cytomegalovirus (CMV) promoter described in Miller, et al., Biotechniques 7:980-990 (1989), or any other promoter (*e.g*., cellular promoters such as eukaryotic cellular promoters including, but not limited to, the histone, pol III, and β-actin promoters). Other viral promoters that may be employed include, but are not limited to, adenovirus promoters, thymidine kinase (TK) promoters, and B19 parvovirus promoters. The selection of a suitable promoter will be apparent to those skilled in the art from the teachings contained herein, and may be from among either regulated promoters or promoters as described above.

As described above, the retroviral plasmid vector is employed to transduce packaging cell lines to form producer cell lines. Examples of packaging cells that may be transfected include, but are not limited to, the PE501, PA317, ψ-2, ψ-AM, PA12, T19-14X, VT-19-17-H2, ψCRE, ψCRIP, GP+E-86, GP+envAm12, and DAN cell lines as described in Miller, Human Gene Therapy, 1:5-14 (1990). The vector may transduce the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line generates infectious retroviral vector particles that include the nucleic acid sequence(s) encoding the cell surface CD83 polypeptide, cell surface anti-immune receptor antibody, or tumor antigen (or tumor-associated antigen) polypeptides. Such retroviral vector particles then may be employed, to transduce eukaryotic cells, either *in vitro* or *in vivo.* The transduced eukaryotic cells will express the nucleic acid sequence(s) encoding the cell surface CD83 polypeptide, cell surface anti-immune receptor antibody, or tumor antigen. Eukaryotic cells that may be transduced include, but are not limited to, tumor cells, embryonic stem cells, as well as hematopoietic stem cells, hepatocytes, fibroblasts, circulating peripheral blood mononuclear and polymorphonuclear cells including myelomonocytic cells, lymphocytes, myoblasts, tissue macrophages, dendritic cells, Kupffer cells, lymphoid and reticuloendothelia cells of the lymph nodes and spleen, keratinocytes, endothelial cells, and bronchial epithelial cells.

As another example of an embodiment of the invention in which a viral vector is used to prepare the recombinant cell surface CD83 polypeptide, cell surface anti-immune receptor antibody, or tumor antigen (or tumor-associated antigen) expression constructs, host cells transduced by a recombinant viral construct directing the expression of a cell surface CD83 polypeptide, cell surface anti-immune receptor antibody, or tumor antigen may produce viral particles containing expressed cell surface CD83 polypeptide, anti-immune receptor antibody, or tumor antigen that are derived from portions of a host cell membrane incorporated by the viral particles during viral budding.

In another aspect, the present invention relates to host cells, such as tumor cells, containing the above described recombinant cell surface CD83 polypeptide, anti- cell surface immune receptor antibody, or tumor antigen (or tumor-associated antigen) expression constructs. Host cells are genetically engineered (transduced, transformed, or transfected) with the vectors and/or expression constructs of this invention that may be, for example, a cloning vector, a shuttle vector, or an expression construct. The vector or construct may be, for example, in the form of a plasmid, a viral particle, a phage, etc. The engineered host cells can be cultured in conventional nutrient media modified as appropriate for activating promoters, selecting transformants or amplifying particular genes such as genes encoding a cell surface CD83 polypeptide, cell surface anti-immune receptor antibody, or tumor antigen. The culture conditions for particular host cells selected for expression, such as temperature, pH and the like, will be readily apparent to the ordinarily skilled artisan.

The host cell can be a higher eukaryotic cell, such as a mammalian cell (including a tumor cell), or a lower eukaryotic cell, such as a yeast cell, or the host cell can be a prokaryotic cell, such as a bacterial cell. Representative examples of appropriate host cells according to the present invention include, but need not be limited to, bacterial cells, such as *E. coli, Streptomyces, Salmonella tvphimurium*; fungal cells, such as yeast; insect cells, such as *Drosophila S2* and *Spodoptera Sf9*; animal cells, such as CHO, COS or 293 cells; adenoviruses; plant cells, or any suitable cell already adapted to *in vitro* propagation or so established *de novo.* The selection of an appropriate host is deemed to be within the scope of those skilled in the art from the teachings herein.

Various mammalian cell culture systems can also be employed to express recombinant protein. Examples of mammalian expression systems include the COS-7 lines of monkey kidney fibroblasts, described by Gluzman, Cell 23:175 (1981), and other cell lines capable of expressing a compatible vector, for example, the C127, 3T3, CHO, HeLa, and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences, for example as described herein regarding the preparation of cell surface CD83 polypeptide, cell surface anti-immune receptor antibody, or tumor antigen expression constructs. DNA sequences derived from the SV40 splice, and polyadenylation sites may be used to provide the required nontranscribed genetic elements. Introduction of the construct into the host cell can be effected by a variety of methods with which those skilled in the art will be familiar, including but not limited to, for example, calcium phosphate transfection, DEAE-Dextran mediated transfection, or electroporation (Davis et al., 1986 Basic Methods in Molecular Biology).

### Methods of Treatment

As described herein, methods and compositions are provided for inducing anti-tumor immunity in a host such as a subject or biological source, preferably in a mammal such as a human patient. In certain embodiments, the use of a composition in the manfacture of a medicament for treating a subject having a malignant condition using the presently disclosed compositions to induce anti-tumor immunity are provided. Compositions of the present invention may be preferably administered to a subject in combination with a pharmaceutically acceptable carrier, incipient, or vehicle, as described herein.

In one embodiment, the use of a composition in the manufacture of a medicament for inducing anti-tumor immunity comprises a composition that comprises a recombinant expression construct that has at least one promoter operatively linked to a polynucleotide encoding a cell surface CD83 polypeptide. In another embodiment, the the use of a composition in the manufacture of a medicament further comprises a second recombinant expression construct comprising at least one second promoter operatively linked to a polynucleotide encoding at least one tumor antigen or tumor associated antigen, which may enhance anti-tumor immunity by further stimulating a T cell response and/or by inducing a humoral response.

According to the present invention, anti-tumor immunity also may be induced by using a recombinant expression construct comprising at least one first promoter operatively linked to a polynucleotide encoding a cell surface CD83 polypeptide and at least one second promoter operatively linked to a polynucleotide that encodes a cell surface form of an antibody, or antigen-binding fragment thereof, that binds specifically to an immune cell receptor, for example, an antibody that binds to a CD137 polypeptide in the manufacture of a medicament. Alternatively, the composition may comprise a first recombinant expression construct that comprises at least one first promoter operatively linked to a polynucleotide encoding a cell surface CD83 polypeptide and a second recombinant expression construct comprising at least one second promoter operatively linked to a polynucleotide encoding a cell surface form of an antibody, or antigen-binding fragment thereof, that binds specifically to an immune receptor such as CD137 polypeptide. Anti-tumor immunity may be further improved or enhanced as described herein by including in the composition a recombinant expression construct having an additional promoter that is operatively linked to a polynucleotide encoding at least one tumor antigen or tumor-associated antigen, or fragment or derivative thereof. A recombinant expression construct having a polynucleotide encoding at least one tumor antigen or tumor-associated antigen is understood to mean that the expression construct may encode more than one tumor antigen (or tumor-associated antigen) and may encode at least two, three, four, or five tumor antigens (or tumor-associated antigens), derivatives, or fragments thereof. Alternatively, a composition for inducing tumor immunity may comprise two or more recombinant expression constructs that each have a promoter that is operatively linked to a polynucleotide encoding at least one tumor antigen (or tumor-associated antigens), or fragment or derivative thereof.

The cell surface CD83 polypeptide expressed by the recombinant expression constructs preferably comprises a CD83 polypeptide or a portion thereof. A composition for inducing anti-tumor immunity in a human subject or host or for treating a subject having a malignant condition, preferably, the cell surface CD83 polypeptide comprises a human CD83 polypeptide or portion thereof. In particular embodiments, the cell surface-expressed (cell surface form) anti-immune cell receptor antibody, such as an anti-CD137 antibody, is a single chain Fv antibody that is fused to a polypeptide for cell surface expression, for example, such a polypeptide comprises a transmembrane domain and a cytoplasmic domain, such as the domains derived from a CD80 polypeptide. For treatment of a human subject, the anti-CD 137 antibody specifically binds to human CD137, and the transmembrane and cytoplasmic portions are also derived from a human polypeptide.

In another embodiment, the invention provides a method for inducing anti-tumor immunity in a subject or host by administering a composition that comprises at least one tumor cell that expresses a cell surface CD83 polypeptide, or by administering a composition that comprises at least one tumor cell that expresses a cell surface CD83 polypeptide and that expresses a cell surface expressed antibody or antigen-binding fragment thereof that specifically binds to an immune cell receptor such as CD137. In certain embodiments, the composition includes at least one tumor cell that expresses a cell surface CD83 polypeptide and at least one second tumor cell that expresses on its surface an antibody or antigen-binding fragment thereof that specifically binds to an immune cell receptor such as CD137. As described herein, the cell surface-expressed anti-CD137 antibody may be a single chain Fv antibody that is fused to a polypeptide comprising a transmembrane domain and a cytoplasmic domain. The composition for treatment of a human subject comprises preferably, the cell surface CD83 polypeptide a human CD83 polypeptide or portion thereof, and the cell surface form of the anti-CD 137 antibody specifically binds to a human CD137 polypeptide.

In addition to direct in vivo procedures, ex vivo procedures may be used in which cells are removed from a host, modified, and placed into the same or another host animal. It will be evident that one can use any of the compositions described herein for introduction of cell surface CD83 polypeptide-, cell surface anti-immune cell receptor antibody-, and/or tumor antigen (or tumor associated antigen)-encoding nucleic acid molecules into cells in an ex vivo context. Protocols for viral, physical, and chemical methods of uptake are described herein and are well known in the art.

The at least one tumor cell may be isolated from a biological sample obtained from a biological source or a subject having a malignant condition. Methods for isolating a tumor cell are described herein and known in the art, and recombinant expression constructs encoding cell surface CD83 and/or cell surface anti-immune cell receptor antibody may be introduced into the tumor cells according to methods described herein and known in the art. For inducing anti-tumor immunity in a subject, the tumor cells that are isolated from the subject and manipulated to express a cell surface CD83 polypeptide and/or a cell surface anti-CD 137 antibody fusion polypeptide on the tumor cell surface may be delivered back into the same subject. In alternative embodiments, the tumor cell so constructed may be delivered into a different subject having a malignant condition.

In either instance, the tumor cell has preferably been modified, manipulated, or treated such that the ability of the tumor cell to divide has been inhibited, abrogated, or substantially impaired, thus minimizing or abrogating the ability of the tumor cell to propagate or grow in the subject. Methods for inhibiting or substantially impairing the ability of a tumor cell to divide include treating the cell with one or more agents that affect mitosis, transcription, or translation, synthesis of cellular molecules or assembly of organelles, and the like. For example, a cell may be modified by treatment with mitomycin C (MMC), which inhibits DNA synthesis, or may be treated by irradiation (*see*, *e*.*g*., Baureus-Koch et al., Br. J. Cancer 90:48-54 (2004)).

Alternatively, the tumor cells may be transfected with a polynucleotide that encodes a polypeptide, which when expressed in the tumor cell, confers susceptibility of the tumor cell to an agent that substantially impairs the ability of the tumor cell to divide. For example, a tumor cell may be transfected with a recombinant expression construct that contains a polynucleotide that encodes thymidine kinase. When such transfected tumor cells are exposed to acylclovir, for example, the cell s that express thymidine kinase are killed. Additionally or alternatively, one or more chemotherapeutic drugs that inhibit or impair the ability of cells to divide may be administered in an appropriate manner for the particular drug, according to procedures with which the skilled artisan will be familiar.

The ability of a tumor cell to divide in a host or subject is substantially impaired, for instance, by employing any of the above methods for abrogating cell growth, when cells are rendered incapable of growing or dividing such that greater than 50% of the cells are unable to divide, preferably when greater than 60% or 70% are unable to divide, more preferably when 80%, 85%, or 90% are unable to divide, and even more preferably when 95%, 98%, or 100% of the tumor cells are unable to divide in the host or subject. The modified tumor cell may be administered to the subject along with a pharmaceutically acceptable carrier according to administration methods known in the art and described herein.

The therapeutic compositions that each contain a separate recombinant expression construct capable of directing the expression of a cell surface CD83 polypeptide, a cell surface anti-immune receptor antibody (*e*.*g*., anti-CD137 scFv), or at least one tumor antigen or tumor associated antigen, may be administered together or separately, for example, at different times. Similarly, compositions comprising a tumor cell that expresses a cell surface CD83 polypeptide and/or a cell surface expressed anti-CD 137 antibody may be administered to a subject together or separately at the same time or at different times (*e*.*g*., different days or weeks). Preferably compositions of the present invention comprising a cell surface CD83 polypeptide and a cell surface expressed anti-CD137 antibody are administered at the same time, that is, on the same day. The appropriate timing and dose for administration of the subject invention compositions will be understood to persons skilled in the medical arts according to their understanding of the compositions, the subject and any and all underlying medical conditions, including host immune status, which may be monitored according to any of a number of criteria described herein and known in the art. A method for inducing an anti-tumor response may include administration of (or immunization with) any one of the subject invention compositions at one or a plurality of points in time.

As noted herein, the presently disclosed compositions for inducing anti-tumor immunity in a host or subject may be administered to a subject having a malignant condition. Although preferably anti-tumor immunity according to the present invention is induced when the tumor load in a subject has been diminished or reduced by conventional methods, such as surgery, chemotherapy, and/or radiation, these compositions may be administered at any stage of the disease, that is, when a malignant condition is detectable according to the presence of a visible or identifiable tumor mass, or when the condition is detectable by diagnostic methods that indicate a subject is in the early stage of a malignant condition and may not yet have a detectable tumor mass. The compositions of the present invention may be useful for inducing anti-tumor immunity that will result in a reduction, total or partial, of the tumor mass. The compositions may also be useful for inhibiting, preventing, or slowing the growth of a tumor, whether the tumor is yet macroscopically visible (with or without inspection by surgical methods) or is not yet detectable by methods known to an artisan skilled in the medical arts. The subject invention compositions and methods may be considered effective after administration to a subject if the subject manifests any clinical benefit, such as a reduction in the severity of symptomatology of the malignant condition, or if the progression of the malignant condition is slowed, inhibited, or abrogated, or if the subject is considered to be in remission or cured according to standards and indicators known in the art.

As described herein, compositions are provided that are capable of delivering nucleic acid molecules encoding a cell surface CD83 polypeptide, a cell surface anti-immune cell receptor antibody (such as a cell surface anti-CD 137 scFv) and/or at least one tumor antigen (or tumor-associated antigen). Such compositions may include, without limitation, recombinant viral vectors (*e*.*g*., retroviruses *(see* WO 90/07936, WO 91/02805, WO 93/25234, WO 93/25698, and WO 94/03622), adenovirus *(see* Berkner, Biotechniques 6:616-27 (1988); Li et al., Hum. Gene Ther. 4:403-409 (1993); Vincent et al., Nat. Genet. 5:130-34 (1993); and Kolls et al., Proc. Natl. Acad. Sci. USA 91:215-19 (1994)); pox virus (*see* U.S. Patent No. 4,769,330; U.S. Patent No. 5,017,487; and WO 89/01973)); recombinant expression construct nucleic acid molecules complexed to a polycationic molecule (*see* WO 93/03709); and nucleic acids associated with liposomes (*see* Wang et al., Proc. Natl. Acad. Sci. USA 84:7851 1987)). In certain embodiments, the nucleic acid (*e.g.,* DNA) may be linked to a killed or inactivated adenovirus *(see* Curiel et al., Hum. Gene Ther. 3:147-154 (1992); Cotton et al., Proc. Natl. Acad. Sci. USA 89:6094 (1992)). Other suitable compositions include DNA-ligand (*see* Wu et al., J. Biol. Chem. 264:16985-87 (1989)) and lipid-DNA combinations (*see* Felgner et al., Proc. Natl. Acad. Sci. USA 84:7413-17 (1989)).

Transfection techniques for delivering the presently disclosed compositions to a cell, including delivery of polypeptides and nucleic acid molecules, may include the use of cationic liposomes (*e*.*g*., Felgner et al., Proc. Natl. Acad. Sci. USA 84:7413-17 (1987)); poly (ortho ester) microspheres (see, *e*.*g*., U.S. Patent No. 5,939,453) calcium phosphate coprecipitation (*e*.*g*., Graham et al., Virology 52:456-67 (1973)); electroporation (*e*.*g*., Neumann et al., EMBO J. 7:841-45 (1982)); microinjection (*e*.*g*., Capecchi, Cell 22:479-88 (1980)); viral vectors (*e*.*g*., Cepko et al., Cell 37:1053-62 (1984)) and the like, or any other suitable transfection methodology. In certain preferred embodiments, a cell surface CD83 polypeptide, cell surface anti-immune receptor antibody, or tumor antigen (or tumor-associated antigen) may be delivered to cells as an intact polypeptide. For example, methods to deliver proteins to cells may include the use of protein transduction domains as peptide carriers, for example, HIV-1 TAT, *Drosophila* Antennapedia homeotic transcription factor, and herpes simplex virus-1 DNA binding protein VP22 (Schwarze et al., Trends Cell Biol. 10:290-95 (2000). To deliver an active protein, correct renaturation is required upon internalization of the protein in the cell. Such delivery systems may require that the polypeptide of interest be covalently linked to the delivery molecule and/or chemical modification of the polypeptide. Another deliver system provides an amphipathic peptide carrier that can form a non-covalent complex with the peptide/polypeptide to be delivered to the cell (*see* Chariot^{™}, Active Motif®, Carlsbad, CA; Morris et al., J. Biol. Chem. 274:24941-46 1999); Morris et al., Nature Biotechnol. 19:1173-76 (2110)).

In certain embodiments, the compositions described herein may be provided to a host or subject in conjunction with at least one agent that decreases, relieves, alleviates, or otherwise counteracts immunological suppression (*e*.*g*., anti-immunosuppressive effect or anti-immunosuppression) of a host immune response. Naturally occurring suppression of immune responses in a host or subject is mediated by antigen-specific T regulatory cells, also described in the art as T suppressor cells. Such immunological suppression could, according to non-limiting theory, potentially diminish the ability of a cell surface CD83 polypeptide and an anti-immune cell receptor antibody (and/or an at least one tumor antigen) to induce an anti-tumor immune response. Accordingly, such potentially undesirable immunological suppression may be diminished, abrogated, or inhibited by counteracting the effects of, for example, Fas ligand expression, CTLA4-CD80/CD86 interaction, TGF-beta expression, and/or prostaglandin activity. Generally, T regulatory cells divide more quickly (that is, have a shorter life span) than T cells that are immunologically reactive against a tumor cell (*e*.*g*., cytotoxic T lymphocytes); therefore, administering a chemotherapeutic agent such as cyclophosphamide or the like at the appropriate time to interfere with immune suppression may significantly diminish (*e*.*g*., decrease in a statistically significant manner) the immunosuppressive effect mediated by T regulatory cells without significantly diminishing the anti-tumor immune response. Alternatively, an agent capable of partially or completely inhibiting T suppressor cell activity, such as a cytokine, a suitable small molecule, an appropriately selected antisense nucleic acid or specific RNAi nucleic acid, or an antibody (or antigen-binding fragment thereof) that specifically binds to a functional immunosuppressive molecule may be useful for diminishing or counteracting the immune suppression. Non-limiting examples of such agents include antagonists of (*e*.*g*., blocking antibodies specific for) TGF-beta, glucocorticoid-induced tumor necrosis factor (GITR), and CTLA4.

These agents may be made according to methods described herein and described in the art. Antibodies that specifically bind to TGF-beta, GITR, or to CTLA4 may be made according to methods described herein and known in the art as exemplified by U.S. Patent No. 5,772,998 and 6,407,218 (TGF-beta); U.S. Patent Application No. 2003/0133936 (GITR); and U.S. Patent Nos. 5,968,510 and 5,977,318 (CTLA4). Antisense molecules may be made according to methods with which a skilled artisan will be familiar and which are described in, for example, U.S. Patent Nos. 5,190,931; 5,135,917; 5,087,617; and 5,168,053 (*see also* Schiavone et al., Curr. Opin. Mol. Ther. 5:217-24 (2003)). In addition, antisense nucleic acids that specifically interfere with transcription and/or translation of nucleic acids encoding GITR and TGF-beta are described in Nocentini et al., Proc. Natl. Acad. Sci. USA 94:6216-21 (1997) and in U.S. Patent No. 6,355,689, respectively. Methods for making specific RNAi (RNA interference) nucleic acids are also described in the art (*see*, *e*.*g*., U.S. Patent No. 6,506,559; WO 01/75164; WO 99/32619; Elbashir et al., Nature 411:494-98 (2001); Zhang et al., Curr. Pharm. Biotech. 5:1-7 (2004); Paddison et al., Curr. Opin. Mol. Ther. 5:217-24 (2003); *see also* U.S. Patent Application No. 2003/0157030 describing RNAi molecules specific for TGF-beta). Other agents, such as small molecules that interfere with transcription or translation of TGF-beta, GITR, or CTLA-4, or agents such as peptides that specifically bind to and interfere with the function of these polypeptides, may be prepared according to methods described in, for example, U.S. Patent No. 6,136,779, U.S. Patent No. 6,503,713, and U.S. Patent No. 6,207,156.

Anti-tumor immunity induced according to methods described herein may also be further enhanced by administering certain appropriately selected cytokines or growth factors. For example, granulocyte-macrophage colony-stimulating factor (GMSCF) may augment antigen presenting cell function in concert with a CD83-CD83 ligand interaction that is promoted by administering to a host one of the CD83/anti-CD137 compositions described herein.

### Pharmaceutical Compositions

The present invention compositions for inducing anti-tumor immunity may be formulated into pharmaceutical compositions for administration according to well known methodologies. Pharmaceutical compositions generally comprise cells expressing one or more immune system stimulating molecules on the cell surface, one or more recombinant expression constructs, and/or expression products of such constructs, in combination with a pharmaceutically acceptable carrier, excipient or diluent. Such carriers will be nontoxic to recipients at the dosages and concentration employed. For nucleic acid-based compositions (*e*.*g*., vaccines), or for compositions comprising expression products of the subject invention recombinant constructs, about 0.01 µg/kg to about 100 mg/kg body weight will be adminstered, typically by the intradermal, subcutaneous, intramuscular, or intravenous route, or by other routes. A preferred dosage is about 1 µg/kg to about 1 mg/kg, with about 5 µg/kg to about 200 µg/kg particularly preferred. As will be evident to those skilled in the art, the number and frequency of administration will be dependent upon the response of the subject or host. "Pharmaceutically acceptable carriers" for therapeutic use are well known in the pharmaceutical art and are described, for example, in Remington's Pharmaceutical Sciences, Mack Publishing Co. (A.R. Gennaro ed. 1985). For example, sterile saline and phosphate-buffered saline at physiological pH may be used. Preservatives, stabilizers, dyes and even flavoring agents may be provided in the pharmaceutical composition. For example, sodium benzoate, sorbic acid and esters of p-hydroxybenzoic acid may be added as preservatives. *Id*. at 1449. In addition, antioxidants and suspending agents may be used. *Id*.

"Pharmaceutically acceptable salt" refers to salts of the compounds of the present invention derived from the combination of such compounds and an organic or inorganic acid (acid addition salts) or an organic or inorganic base (base addition salts). The compounds of the present invention may be used in either the free base or salt forms, with both forms being considered as being within the scope of the present invention.

The pharmaceutical compositions that contain one or more CD83 polypeptide and/or cell surface anti-immune receptor antibody (such as anti-CD137 scFv fusion polypeptide) and/or tumor antigen or tumor-associated antigen encoding constructs (or their expressed products) may be in any form that allows for the composition to be administered to a patient. For example, the composition may be in the form of a solid, liquid or gas (aerosol). Typical routes of administration include, without limitation, oral, topical, parenteral (*e*.*g*., sublingually or buccally), sublingual, rectal, vaginal, and intranasal. The term parenteral as used herein includes subcutaneous injections, intravenous, intramuscular, intrasternal, intracavernous, intrathecal, intrameatal, intraurethral injection or infusion techniques. The pharmaceutical composition is formulated so as to allow the active ingredients contained therein to be bioavailable upon administration of the composition to a patient. Compositions that will be administered to a patient take the form of one or more dosage units, where for example, a tablet may be a single dosage unit, and a container of one or more compounds of the invention in aerosol form may hold a plurality of dosage units.

For oral administration, an excipient and/or binder may be present. Examples are sucrose, kaolin, glycerin, starch dextrins, sodium alginate, carboxymethylcellulose and ethyl cellulose. Coloring and/or flavoring agents may be present. A coating shell may be employed.

The composition may be in the form of a liquid, *e*.*g*., an elixir, syrup, solution, emulsion or suspension. The liquid may be for oral administration or for delivery by injection, as two examples. When intended for oral administration, preferred compositions contain, in addition to one or more CD83 polypeptide and/or anti-CD137 scFv and/or tumor antigen or tumor-associated antigen constructs or expressed products, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

A liquid pharmaceutical composition as used herein, whether in the form of a solution, suspension or other like form, may include one or more of the following adjuvants: sterile diluents such as water for injection, saline solution, preferably physiological saline, Ringer's solution, isotonic sodium chloride, fixed oils such as synthetic mono or digylcerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable pharmaceutical composition is preferably sterile.

Inclusion of other components in the therapeutic composition may be desirable, such as delivery vehicles including but not limited to aluminum salts, water-in-oil emulsions, biodegradable oil vehicles, oil-in-water emulsions, biodegradable microcapsules, and liposomes. Examples of immunostimulatory substances (adjuvants) for use in such vehicles include N-acetylmuramyl-L-alanine-D-isoglutamine (MDP), lipopolysaccharides (LPS), glucan, IL-12, GM-CSF, gamma interferon, and IL-15.

While any suitable carrier known to those of ordinary skill in the art may be employed in the pharmaceutical compositions of this invention, the type of carrier will vary depending on the mode of administration and whether a sustained release is desired. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e*.*g*., polylactic galactide) may also be employed as carriers for the pharmaceutical compositions of this invention. Suitable biodegradable microspheres are disclosed, for example, in U.S. Patent Nos. 4,897,268 and 5,075,109. In this regard, it is preferable that the microsphere be larger than approximately 25 microns.

Pharmaceutical compositions (including recombinant expression constructs, cells containing the constructs, or vaccines) may also contain diluents such as buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents. Preferably, product is formulated as a lyophilizate using appropriate excipient solutions (*e*.*g*., sucrose) as diluents.

As described above, the subject invention includes compositions capable of delivering nucleic acid molecules encoding cell surface CD83 polypeptide and/or cell surface anti-immune receptor antibody (*e*.*g*., anti-CD137 scFv fusion polypeptide) and/or a tumor antigen (or tumor-associated antigen). Such compositions include recombinant viral vectors (*e*.*g*., retroviruses (*see* WO 90/07936, WO 91/02805, WO 93/25234, WO 93/25698, and WO 94/03622); adenovirus *(see* Berkner, Biotechniques 6:616-27, 1988; Li et al., Hum. Gene Ther. 4:403-409, (1993); Vincent et al., Nat. Genet. 5:130-34, (1993); and Kolls et al., Proc. Natl. Acad. Sci. USA 91:215-19, (1994), pox virus (*see* U.S. Patent No. 4,769,330; U.S. Patent No. 5,017,487; and WO 89/01973)), recombinant expression construct nucleic acid molecules complexed to a polycationic molecule *(see* WO 93/03709), and nucleic acids associated with liposomes *(see* Wang et al., Proc. Natl. Acad. Sci. USA 84:7851,1987). In certain embodiments, the DNA may be linked to killed or inactivated adenovirus *(see* Curiel et al., Hum. Gene Ther. 3:147-154, 1992; Cotton et al., Proc. Natl. Acad. Sci. USA 89:6094,1992). Other suitable compositions include DNA-ligand *(see* Wu et al., J. Biol. Chem. 264:16985-16987,1989) and lipid-DNA combinations *(see* Felgner et al., Proc. Natl. Acad. Sci. USA 84:7413-7417, 1989).

In addition to direct *in vivo* procedures, *ex vivo* procedures may be used in which cells are removed from a host, modified, and placed into the same or another host animal. It will be evident that one can use any of the compositions noted above for introduction of cell surface CD83 polypeptide and/or cell surface anti-immune receptor antibody (such as anti-CD137 scFv) encoding nucleic acid molecules into tumor cells in an *ex vivo* context. Protocols for viral, physical, and chemical methods of uptake are well known in the art.

Accordingly, the present invention is useful for enhancing, stimulating, or eliciting, in a host, a subject or patient or in cell culture, an anti-tumor immune response that may include a T cell response and a humoral immune response. As used herein, the term "patient" refers to any warm-blooded animal, preferably a human. A patient may have or be afflicted with a malignant condition such as cancer, or may be normal (*i*.*e*., free of detectable disease and infection).

A liquid composition intended for either parenteral or oral administration should contain an amount of one or more recombinant expression constructs described herein such that a suitable dosage will be obtained. Typically, this amount is at least 0.01 wt% of a construct. When intended for oral administration, this amount may be varied to be between 0.1 and about 70% of the weight of the composition. Preferred oral compositions contain between about 4% and about 50% of a construct(s). Preferred compositions and preparations are prepared so that a parenteral dosage unit contains between 0.01 to 1% by weight of active compound.

The pharmaceutical composition may be intended for topical administration, in which case the carrier may suitably comprise a solution, emulsion, ointment, or gel base. The base, for example, may comprise one or more of the following: petrolatum, lanolin, polyethylene glycols, beeswax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device. Topical formulations may contain a concentration of the compositions comprising a tumor cell or one or more constructs of from about 0.1 to about 10% w/v (weight per unit volume).

The composition may be intended for rectal administration, in the form, *e*.*g*., of a suppository that will melt in the rectum and release the drug. The composition for rectal administration may contain an oleaginous base as a suitable nonirritating excipient. Such bases include, without limitation, lanolin, cocoa butter and polyethylene glycol.

The compositions including recombinant expression constructs may be administered through use ofinsert(s), bead(s), timed-release formulation(s), patch(es) or fast-release formulation(s).

The following examples are included for illustrative purposes only and are not intended to limit the scope of the invention.

### EXAMPLE 1

### TUMOR CELL LINES, VECTORS, TRANSFECTION METHODS, ANTIBODIES, AND ANIMAL STUDY DESIGN

The following reagents and methods were used for experiments described in the Examples presented herein.

**Tumor lines.** K1735 is a melanoma ofC3H/HeN origin from which clones M2 and SW1 were isolated (Fidler et al., Cancer Res. 41:3266-67 (1981); Price et al., Cancer Res. 48:2258-64 (1988)). SW1-P2 was derived by transfecting SW1 cells to express a 50 amino acid peptide derived from the NH2 terminal domain of the transcription factor ATF2 (Bhoumik et al., J. Clin. Invest. 110:643-650 (2002)). Expression of ATF2, which belongs to the ATF/GREB family, within the nucleus rather than the cytoplasm is associated with poor prognosis of melanoma (Berger et al., Cancer Res. 63:8301-307 (2003)). SW1-P2 cells express less ATF2 in the nucleus and more in the cytoplasm than SW1-C cells, and this is correlated with decreased malignancy and increased ability to undergo apoptosis upon exposure to radiation or certain chemicals (Bhoumik et al., Clin. Cancer Res. 7:331-42 (2001)).

The SW1-C and SW1-P2 cell lines were chosen because initial tests revealed that they were not rejected by immunocompetent mice after transfection to express anti-CD137 scFv derived from hybridoma 1 D8 (data not shown), a procedure that makes cells from the M2 clone highly immunogenic (Ye et al., Nat. Med. 8:343-48 (2002)). Ag104 is a spontaneous fibrosarcoma of C3H/HeN originally obtained from Dr H. Schreiber (University of Chicago, Chicago, IL) and has low immunogenicity (Ward et al., J. Exp. Med. 170:217-32 (1989)). Tumor cells transplanted to mice were derived from *in vitro* cultures. Cell suspensions comprising greater than 90% live tumor cells were prepared by exposing cultures to 0.01 % versene for 5 minutes.

**Vectors and transfection of cells.** The mouse CD83 (mCD83) gene was amplified from anti-CD3 monoclonal antibody-activated mouse spleen cells using primer GTGTCGCAGCGCTCCAGCC [SEQ ID NO:_] and primer GGCATTCAGGCACACTGATC [SEQ ID NO:_] (Twist et al., Immunogenetics 48:383-93 (1998)). An amplified cDNA fragment was first cloned into pGEM-T easy vector (Promega, Madison, WI) and verified by DNA sequencing, after which the mouse CD83 gene was cloned into pLNCX2 vector (BD Biosciences Clontech, Palo Alto, CA) and pLenti6/V5 vector (Invitrogen^{™} Life Technologies, Carlsbad, CA). Transfection of a packaging cell line and infection of target cell lines (including cells from the M2 clone of K1735 cells) were performed according to the manufacturer's recommendations.

To produce mCD83 Ig fusion protein, the mouse CD83 extracellular domain (ECD) [SEQ ID NO:_] (positions 1-134 of SEQ ID NO: 6) was amplified from the mouse CD83 gene using primer AAGCTTCCAGCCATGTCGCAAGGCCTC [SEQ ID NO:_] and GGATCCGCCCTGTAGTTCCTG [SEQ ID NO:_]. The amplified fragment was first cloned into pCR-topo vector (Invitrogen Life Technologies), and the nucleotide sequence was verified by standard DNA sequencing technique. Mouse CD83 ECD was cloned into pD18-mIgG (IgG₂ₐ isotype) vector and was transfected into COS-7 cells to produce mCD83-ECD-mIgG fusion protein, which was subsequently purified using Protein A Sepharose 4B (Sigma Aldrich, St. Louis, MO).

A CD83-human Ig fusion protein (CD83-hIg) was generated by cloning a human IgG1 constant region (Lenschow et al., Science 257:789-92 (1992)) instead of the murine constant region, as described by Scholler et al. (J. Immunol. 168:2599-2602 (2002)). The fusion protein was used for screening hybridomas for production of anti-CD83 antibody and for FACS analysis with FITC-labeled goat anti-human immunoglobulin that was used as a second reagent.

Target cells transfected with mCD83 gene were detected with rat anti-mCD83 monoclonal antibody 7A1 (obtained as described below) and PE-labeled goat anti-rat immunoglobulin. Transfected cells are referred to by the name of the respective clone or subline followed by the transfected gene. For example, SW1-P2-CD83 cells were derived from SW1-P2 and stably expressed CD83 at their surface. As one control, M2 cells were transfected with an irrelevant gene (mouse anti-human CD28 scFv), which is referred to as M2-control (Ye et al., Nat. Med. 8:343-48 (2002)). M2-1D8 cells that express anti-CD137 scFv from hybridoma 1D8 were constructed as previously described (*id*.).

For most experiments, tumor cells were sterilized by in vitro exposure to Mitomycin C (MMC) (Sigma). Tumor cells were washed once with PBS and then incubated with 50 µg MMC /10⁷ cells for one hour at 37 °C (Hara et al., Cancer Gene Ther. 7:83-90 (2000)). The cells were then washed four times with PBS prior to use in vivo (as vaccines) or in vitro (to stimulate T cell responses).

**Antibodies.** For *in vitro* studies, R-phycoerythrin (PE)-, FITC-, or biotin-conjugated anti-mouse CD4 MAb GK1.5, similarly conjugated anti-mouse CD8 MAb 53-6.7, PE-conjugated anti-mouse CD19, and anti-mouse CD11c, as well as PC5-conjugated anti-mouse CD45, and rabbit anti-mouse NK antisera, were purchased from BD Biosciences Pharmingen (San Diego, CA). PE-conjugated goat F(ab')₂ anti-human IgG was purchased from Biosource International (Camarillo, CA). For in vivo studies, the following antibodies were used: monoclonal antibody 169-4 (anti-CD8) produced by a rat hybridoma (Dr R. Mittler, Emory University, Atlanta, GA); anti-CD4 monoclonal antibody GK1.5 produced by a rat hybridoma (ATCC, Manassas, VA); rabbit anti-asialo GM1 antibodies (Wako Pure Chemical Industries, Richmond,VA); and purified rat IgG (Sigma and Rockland, Gilbertsville, PA).

Anti-CD83 monoclonal antibodies were generated by first immunizing a Lewis rat by intramuscular injection of 200 µg mCD83-ECD-mIgG fusion protein mixed with TiterMax (CytRx, Norcross, GA). The rat was then injected three times subcutaneously with 150 µg mCD83-ECD-mIgG every second week. The rat was boosted two weeks after the last immunization by an injection intrperitoneally 10 days before sacrifice and were then injected intravenously 3 days before sacrifice. Spleen cells were fused with mouse myeloma cells P3-X63-AG8.653 according to standard procedures (Yeh et al., Proc. Natl. Acad. Sci. U S A 76:2927-31 (1979)). Hybridomas were screened for binding to mCD83-ECD-hIgG fusion protein, and 6 high-producing hybridomas were cloned. The highest producer, 7A1, was cultured in protein-free hybridoma medium, PFHM-II (Invitrogen Life Technologies). The monoclonal antibody was purified from the supernatant by Protein G Sepharose 4B chromatography (Sigma).

**Animal Studies.** Six to eight week old female C3H/HeN mice were purchased from Taconic (Charles River Labs, Wilmington, MA). Experiments were carried out in ALAC approved facilities under protocols approved by the Pacific Northwest Research Institute (PNRI) Animal Committee. Mice, 5 per group unless otherwise stated, were immunized by transplantation, subcutaneously on one side of the back, with 2×10⁶ live or MMC-treated tumor cells and challenged with 10⁶ or 2×10⁶ live WT cells on the other side of the back. Tumor size was assessed by measuring the two largest perpendicular diameters with calipers and reported as average tumor area (mm²) ±SD.

To investigate therapeutic activity, mice with subcutaneous WT tumors on one side of the back (approximately 20 mm² surface area) were immunized by subcutaneously injection of live or MMC-treated tumor cells (2×10⁶/mouse) on the contralateral side of the back. Mice were monitored with respect to extent of tumor growth and survival. Mice whose tumors had regressed were observed for a minimum of 120 days and checked for evidence of autoimmunity, including weight loss and depigmentation.

### EXAMPLE 2

### TUMOR CELL IMMUNITY INDUCED BY CELL SURFACE EXPRESSION OF CD83

**Transfected Cells Express CD83 at Their Surface.** Expression of CD83 on M2-CD83, SW1-P2-CD83, and SW1-C-CD83 cells as shown in Figures 1A, 1B, and 1C, respectively, was determined by flow cytometry analyses (EPICS XL, Coulter) performed as described (Weston et al., J. Immunol. Methods 133:87-97 (1990)). The corresponding WT cell lines lacked expression of CD83 on the cell surface. A similar experiment demonstrated that CD83 was expressed on Ag104-CD83 cells.

**Role of CD4+ T cells, CD8+ T cells, and NK cells in Regression of M2-CD83 Tumors.** M2-CD83 cells (2×10⁶ cells/mouse) were regularly rejected when transplanted in syngeneic immunocompetent mice. To investigate the roles of CD4+ and CD8+ T cells as well as NK cells in the regression of M2-CD83 tumors, naive mice were injected with antibodies to remove each respective cell population. T cells were depleted as described (Chen et al., Cell 71:1093-102 (1992)) by injecting mice intraperitoneally three times with a monoclonal antibody specific for CD4 (GK1.5, rat IgG2b) or CD8 (169-4, rat IgG 2a), or with a mixture of the two, at 0.5 mg/mouse for 3 consecutive days. The mice were then injected with 0.5 mg of each monoclonal antibody every third day. NK cells were depleted by injection of anti-asialo GM1 antibodies at 30 µl/mouse intraperitoneally every fourth day. On day 12, spleen cells from each group were analyzed by FACS to verify that the depletions were efficient. On day 13, mice (5/group) were transplanted subcutaneously with M2-CD83 cells (2×10⁶/mouse) and followed for tumor outgrowth. Control mice were injected with rat IgG.

When flow cytometry analysis of spleen cells isolated from similarly injected mice showed that the targeted cell populations were depleted (twelve days after injection with the antibodies), 2×10⁶ M2-CD83 cells were transplanted subcutaneously. M2-CD83 cells were rejected by mice injected with rat IgG with similar growth kinetics as in untreated mice. Tumors were removed 4-8 days after transplantation of 2×10⁶ tumor cells for studies by standard immunohistochemistry methods. Frozen sections were cut at 10 µm and stained using a Vector ABC kit (Vector Laboratories, Burlingame, CA) according to the manufacturer's protocol. The cells were then stained to detect CD4+, CD8+ T cells, and NK cells. The sections were counterstained with H-E. Figure 1D shows that M2-CD83 cells grew progressively in all mice injected with the monoclonal antibodies specific for CD8+, CD4+, and NK cells. The cells grew more slowly in mice in which the NK cells had been depleted than in mice lacking CD4+ and/or CD8+ T cells. These findings are different from those obtained in similar experiments with M2-1D8 cells for which CD8+ T cells are not needed for tumor rejection (Ye et al., Nat. Med. 8:343-48 (2002)).

To determine whether an interaction between CD83 and its ligand(s) was responsible for the regression of M2-CD83 in immunocompetent mice, experiments were performed to assess whether regression could be prevented by injection of soluble mCD83 immunoglobulin. Mice were transplanted subcutaneously with 2×10⁶/mouse M2-CD83 cells, and 100 µg/mouse mCD83mIg fusion protein was injected intraperitoneally on the day of tumor transplantation, followed by the same dose, every third day for a total of four injections. In a parallel experiment the mice were, instead, injected intraperitoneally with 100 µg/mouse of rat anti-mCD83 monoclonal antibody (rat immunoglobulin was used as control) on the day of transplantation of M2-CD83 cells (2×10⁶/mouse). As shown in Figure 1E, 2×10⁶ M2-CD83 cells grew in mice that had been repeatedly injected with mCD83Ig and did not grow in the controls. An experiment was also performed in which the experimental mice were injected with rat anti-mCD83 monoclonal antibody 7A1, and the control mice were injected with rat IgG. M2-CD83 cells grew in all of 5 mice receiving anti-mCD83 MAb and were rejected in the control group.

**Systemic Anti-Tumor Immunity in Mice That Rejected M2-CD83 Cells.** Figure 1F shows that mice which first rejected M2-CD83 also rejected a challenge dose of 2×10⁶ M2-WT cells. In contrast, M2-WT cells grew in and killed all naive mice. Outgrowth of syngeneic Ag104 sarcoma cells was retarded in animals that were previously injected with and had rejected the M2-CD83 cells (Figure 1G). All five mice, whose M2-CD83 tumors had regressed, survived without evidence of recurrence of the WT tumors (Figure 1H), as did two of five immunized mice challenged with Ag104 cells (Figure 1I). This experiment was repeated twice with similar results. The observed rejection of Ag104 by some of the mice immunized against M2-CD83 cells is in contrast to findings obtained with mice that had been immunized against M2-1D8 expressing anti-CD 137 scFv in which a challenge dose of WT cells from Ag104 grows progressively while M2 cells are rejected (Ye et al., Nat. Med. 8:343-48 (2002)). No evidence of toxicity in mice immunized against M2-CD83 cells was observed, including no depigmentation of the skin as observed in approximately one third of the mice that had been repeatedly immunized against M2-1D8 cells (*id*.).

### EXAMPLE 3

### EFFECT OF CELL SURFACE-EXPRESSED CD83 ON GROWTH OF ESTABLISHED TUMORS

**Therapeutic Efficacy Against M2-WT Tumors.** Mice were injected with M2-WT cells on one side of the back. Immunization with live M2-CD83 cells (that is, not treated with MMC or a similar agent to prevent the cells from growing in the animal) was started when the tumors had an approximate 20 mm² surface area. Immunization was repeated three times as indicated by arrows in Figure 2. Figure 2A shows that live M2-CD83 cells can be used as a therapeutic vaccine against established subcutaneous tumors. In contrast, M2-WT tumors grew as well in mice immunized with irradiated (12,000 rads) or MMC-treated M2-WT cells as in mice injected with PBS. Figure 2B shows that tumors regressed in four of five mice vaccinated by transplantation of M2-CD83 cells. The mice remained tumor-free during an observation period of 120 days. No indication of toxicity or depigmentation was observed. The experiment was repeated with similar results.

The immunogenicity of M2-CD83 cells that had been sterilized by in vitro treatment with MMC (see Example 1) or irradiation (12,000 rads) was then studied. With both types of treated cells, the sterilized tumor cells prevented outgrowth of a challenge dose of M2-WT cells. Experiments were then performed in which mice with M2-WT tumors were repeatedly immunized with MMC-treated M2-CD83 cells. Immunizations were begun when the tumors had approximately 20 mm² surface area. While the WT tumors grew in all controls, the tumors were rejected in all of five immunized mice (Figure 2B). The mice survived tumor free and without signs of toxicity over an observation period of 120 days. The experiment was repeated with similar results.

### EXAMPLE 4

### EFFECT ON TUMOR CELL GROWTH BY IMMUNIZATION WITH CELLS EXPRESSING CD83 ON THE CELL SURFACE AND WITH CELLS EXPRESSING ANTI-CD137 ANTIBODY ON THE CELL SURFACE

**Transfection of SW1-C Cells or SW1-P2 cells to Express CD83 (or 1D8) Does Not Make The Cells Immunogenic.** Experiments were performed that were similar to experiments using M2-WT cells but instead cells from the SW1 clone were used (Price et al., Cancer Res. 48:2258-64 (1988)), herein referred to as SW1-C. Like M2, the SW1 clone is derived from K1735 cells. A SW1-P2 line that expresses an ATF2-derived peptide was also used (Bhoumik et al., J. Clin. Invest. 110:643-650 (2002)) (see Example 1). SW1-C-CD3, SW1-C-1D8, and SW1-C cells were transplanted into naive mice (10⁶ cells/mouse). In contrast to growth of M2-CD83 cells in naive mice, both SW1-C cells transfected with mCD83Ig and SW1-C transfected with 1D8 (anti-CD137 scFv) cells grew progressively in naïve syngeneic mice as indicated in Figure 3A. SW1-P2 cells transfected to express either CD83 or anti-CD137 scFv also grew progressively in naïve syngeneic mice.

Outgrowth of SW1-C cells was delayed, but not prevented, in mice immunized four times with MMC-treated M2-CD83 cells or MMC-treated M2-1 D8 cells (2 x 10⁶ cells per mouse) (Figure 3B). The delay in outgrowth of the tumor was less in mice immunized 1-3 times. Outgrowth of SW1-P2 WT cells (10⁶ cells/mouse) is shown in Figure 3C. Outgrowth of SW1-P2 cells was inhibited to a greater extent than outgrowth of SWC-1 in mice that had been similarly immunized (Figure 3C and 3D). One of the five mice rejected the WT tumor cells and survived tumor-free. Immunization with M2-1D8 cells, which is effective for therapeutic vaccination against M2-WT tumors (Ye et al., Nat. Med. 8:343-48 (2002)), delayed but did not prevent outgrowth of SW1-C cells or SW1-P2 cells. Immunization with MMC-treated M2-WT, M2-control (M2-anti-human CD28 scFv), or SW1-C -WT cells had no effect on tumor growth.

**Rejection of SW1-C WT and SW1-P2 WT Cells by Mice Immunized with a Mixture of MMC-Treated M2-CD83 and M2-1D8 Cells.** To analyze whether the immunological mechanisms engaged by M2-CD83 cells and M2-1D8 cells (M2 cells transfected with an anti-CD137 scFv vector 1 D8 *(see* Ye et al., Nat. Med. 8:343-48 (2002)) were different, experiments were performed in which animals were concomitantly immunizing with a mixture of cells from the two cell lines. Mice were immunized twice, one week apart, with a mixture of M2-CD83 cells and M2-1D8 cells and then challenged with SW-1C or SW1-P2. Figure 3E shows that immunization with a mixture of M2-CD83 and M2-1 D8 cells prevented the take of SW1-C cells in three of five mice and delayed outgrowth in the two other mice in the group. Figure 3F shows that this immunization regimen prevented the take of SW1-P2 cells in all immunized mice. Other groups of mice were immunized first with M2-CD83 cells followed one week later by immunization with M2-1D8 cells and then challenged with SW1-P2 WT cells or SW1 -C WT cells. No protection was observed in mice challenged with SW1-P2 cells, and a modest effect was observed in mice challenged with SW1-C cells. Immunization with MMC-treated M2 WT cells or MMC-treated SW1-P2 WT cells was ineffective (Figure 3E and 3F, respectively). This experiment was repeated with similar findings.

### EXAMPLE 5

### ANALYSIS OF IMMUNE RESPONSE IN ANIMALS IMMUNIZED WITH CELLS EXPRESSING CD83 ON THE CELL SURFACE

**M2-CD83 Attracts and Stimulates CD4 and CD8+ T cells, NK cells, and B cells.** M2-WT cells and M2-CD83 cells (2×10⁶ cells/mouse) were transplanted in naive mice. Tumors were harvested six days later when the M2-CD83 tumors started to regress. Frozen sections were cut at 10 µm and stained using a Vector ABC kit (Vector Laboratories, Burlingame, CA) according to the manufacturer's protocol and stained to detect CD4+ and CD8+ T cells, and NK cells. The sections were counterstained with H-E. The antibodies used for detecting the different cells are described in Example 1. Figure 4A presents the M2-WT tumor cell sections, and Figure 4B presents the M2-CD83 tumor sections. An influx of both CD4+ and CD8+ T cells into the M2-CD83 tumors was observed. An increased number of infiltrating NK cells was also observed in the M2-CD83 tumors compared with the M2 WT tumors.

The types of cells present in the tumors were also analyzed by flow cytometry preformed as described in Example 2. Tumor draining lymph node (LN) and spleen cell suspensions were prepared mechanically. The spleen cell suspensions were incubated with red blood cell lysing buffer (Sigma) for 5 minutes at room temperature. To obtain tumor-infiltrating lymphoid cells (TIL), tumors were cut in 1 mm² pieces and digested for 3 hours at room temperature in 1 mM HEPES Hank's buffer containing 5 U/ml collagenase, 0.1% hyaluronidase, and 0.01% DNAase (enzymes purchased from Invitrogen^{™} Life Technologies). The suspensions were filtered, cells collected by centrifugation, and the erythrocytes were lysed. After washing with PBS, the cells were added to a mouse lymphocyte isolation buffer (Cedarlane, Ontario, Canada) and centrifuged at 200 ×g for 20 minutes, after which the lymphocyte band was collected and washed with PB S.

The production of IFNγ and TNF by spleen cells or by TIL was measuring using ELISPOT kits (Cell Sciences, Norwood, MA) according to the manufacturer's protocol. The plates were counted by an Image analyzer at Fred Hutchinson Cancer Research Center (Seattle, WA, courtesy of Dr C. Yee).

Table 1 presents a summary of the analyses of spleen cells and tumor-infiltrating lymphoid cells (TIL). The numbers of CD4+ and CD8+ T cells among tumor infiltrating lymphoid cells (TIL) from M2-CD83 was significantly greater than from M2-WT tumors. The frequency of TIL that stained for IFNγ almost doubled in the M2-CD83 tumors. Increased numbers of CD4+ and CD8+ T cells were observed in spleens from mice transplanted with M2-CD83 cells, and an increase of IFNγ positive CD4 cells was observed in spleens from the M2-CD83 group. This experiment was repeated with similar results.

**Table 1. Flow Cytometry Analyses of Spleen (Sp) cells and Tumor-Infiltrating Lymphoid Cells (TIL)**

| | | |
|---|---|---|
| CELL POPULATION | M2-WT (%) | M2-CD83 (%) |
| Sp CD4+/CD45+ | 15.1+/-0.3 | 16.9+/-0.2 |
| Sp CD8+/CD45+ | 9.6+/-0.1 | 11.3+/-0.1 |
| Sp CD4+ IFNγ | 1.0+/-0.1 | . 5.9+/-0.5 |
| TIL CD4+/CD45+ | 4.6+/-0.4 | 17.1+/-0.5 |
| TIL CD8+/CD45+ | 2.2+/-0.4 | 42.0+/-0.2 |
| TIL IFNγ | 34.2+/-2.1 | 59.2+/-2.8 |
| TIL TNF | 45.5+/-1.1 | 54.3+/-0.6 |

The percentage of cells expressing a certain marker is provided as the mean +/-standard deviation from three determinations.

In other experiments, spleens were harvested 6 days after transplantation of 2×10⁶ M2-CD83 cells and cultured for 4 days. For comparison, spleens were obtained from naive mice and, in one experiment, from a mouse transplanted 6 days previously with the same dose of M2-WT cells. Spleen cells were cultivated in vitro for four days in a 6-well plate coated with 10 µg/ml of anti-CD3 monoclonal antibody (PN IM2767, Immunotech) and 10 U/ml IL-2 (Roche, Indianapolis, IN). The cells were monitored by flow cytometry analysis for expression of certain surface markers. Antibodies used for this analysis are described in Example 1. Results from two separate experiments are presented in Table 2. A consistent increase in CD 19+ cells, which were most likely B cells, was observed in the spleens of mice that were rejecting M2-CD83 tumors. No difference in the number of CD19+ cells was indicated in mice with M2-WT tumors compared with naive mice.

**Table 2. Composition of Spleen Cell Populations after Cultivation In Vitro**

| CELLS | NAÏVE | M2-WT | M2-CD83 |
|---|---|---|---|
| CD83L | 11.5; 10.8 | NT (Not Tested) | 21.5; 16.7 |
| CD19 | 8.2; 9.3 | NT; 11.6 | 28.9; 27.9 |
| CD4 | 46.3; 46.6 | NT; 48.1 | 42.2; 43.4 |
| CD8 | NT; 34.1 | NT; 25.1 | NT; 17.7 |
| CD11b | NT; 4.2 | NT; 5.1 | NT; 6.0 |
| CD11c | 8.4; 6.0 | NT; 10.2 | 8.6; 7.8 |
| CD14 | 1.0; 0.8 | NT; 0.8 | 1.2; 0.9 |

A T cell proliferation assay was performed as described (Ye et al., Nat. Med. 8:343-48 (2002)) (*See also* Li et al. J. Immunol. 153:153:421-28 (1994)). Spleens were removed from mice that were either naïve or were immunized three times, seven days apart, with MMC-treated M2-WT or M2-CD83 cells (2 x 10⁶ cells per mouse). Spleen cells were seeded into 96-well flat-bottom plates (1×10⁵ cells/well) together with 5×10⁵ syngeneic, MMC-treated spleen cells (*i*.*e*., spleen cells from a naïve mouse were incubated with MMC-treated spleen cells from a naive mouse), or with MMC-treated tumor cells (M2-WT cells or Ag104-WT) (*see also* Li et al., J. Exp. Med. 183:639-44 (1996); Melero et al., Eur. J. Immunol. 28:1116-21 (1998)). After incubation for 72 hours, triplicate cultures were pulsed for 16-18 hrs with 1 µCi ³[H]- thymidine (Amersham Pharmacia, Biotech Piscataway, New Jersey) and its uptake measured.

Figure 5 shows that proliferation was increased when the spleen cells were combined with either MMC-treated M2-WT cells or with Ag104-WT cells. Experiments in which the spleen cells were labeled with CFDA (5-(and-6)-carboxyfluorescein diacetate, succinimidyl ester, (fixable-cell-permeant, fluorescein-based tracer for long-term cell labeling), Invitrogen Life Technologies) revealed that both CD4+ and CD8+ T cells proliferated. The number ofT cells producing IFNγ was analyzed using ELISPOT assays. A greater number of IFNγ producing T cells was identified from mice that had been immunized against M2-CD83 cells, compared with naïve mice or mice that had been immunized against M2-WT cells. This greater number of IFNγ producing T cells was observed when the T cells were combined with M2-WT or with Ag104-WT cells.

## Claims

1. A composition for inducing anti-tumor immunity comprising at least one composition that is selected from:
(a) a composition comprising a tumor cell that expresses (i) a cell surface CD83 polypeptide, or a portion thereof; and (ii) a cell surface form of an antibody, or antigen-binding fragment thereof, that binds specifically to CD137;
(b) a composition comprising (i) a first tumor cell that expresses a cell surface CD83 polypeptide, or a portion thereof; and (ii) a second tumor cell that expresses a cell surface form of an antibody, or antigen-binding fragment thereof, that binds specifically to CD137;
(c) a composition comprising (1) a recombinant expression construct comprising at least one first promoter operatively linked to a polynucleotide encoding a cell surface CD83 polypeptide and at least one second promoter operatively linked to a polynucleotide that encodes a cell surface form of an antibody, or antigen-binding fragment thereof, that binds specifically to a CD137 polypeptide or (ii) a first recombinant expression construct that comprises at least one first promoter operatively linked to a polynucleotide encoding a cell surface CD83 polypeptide and a second recombinant expression construct comprising at least one second promoter operatively linked to a polynucleotide encoding a cell surface form ofan antibody, or antigen-binding fragment thereof, that binds specifically to a CD137 polypeptide, or a portion thereof;
(d) a composition comprising (i) a first recombinant expression construct that comprises at least one promoter operatively linked to a polynucleotide encoding a cell surface CD83 polypeptide, or a portion thereof, and (ii) a second recombinant expression construct comprising at least one second promoter operatively linked to a polynucleotide encoding at least one tumor antigen.

2. The composition of claim 1(a) wherein the tumor cell is transfected with (i) a recombinant expression construct comprising at least one first promoter operatively linked to a polynucleotide encoding a cell surface CD83 polypeptide and at least one second promoter operatively linked to a polynucleotide that encodes a cell surface form of an antibody, or antigen-binding fragment thereof, that binds specifically to a CD137 polypeptide or (ii) a first recombinant expression construct that comprises at least one first promoter operatively linked to a polynucleotide encoding a cell surface CD83 polypeptide and a second recombinant expression construct comprising at least one second promoter operatively linked to a polynucleotide encoding a cell surface form of an antibody, or antigen-binding fragment thereof, that binds specifically to a CD137 polypeptide.

3. The composition of claim 1(b) wherein the first tumor cell is transfected with a first recombinant expression construct comprising at least one first promoter operatively linked to a polynucleotide encoding a cell surface CD83 polypeptide, and wherein the second tumor cell is transfected with a second recombinant expression construct comprising at least one second promoter operatively linked to a polynucleotide encoding a cell surface form of an antibody, or antigen-binding fragment thereof, that binds specifically to a CD 137 polypeptide.

4. The composition of claim 1(c) comprising a recombinant expression vector comprising at least one promoter operatively linked to a polynucleotide sequence encoding at least one tumor antigen.

5. The composition of either claim 1(d) or claim 4 wherein the tumor antigen is expressed by a tumor cell in a biological sample that is obtained from a subject having a malignant condition.

6. The composition of any one of claims 1-5 wherein the antibody that specifically binds to CD137 is a single chain Fv antibody.

7. The composition of any one of claims 1-5 wherein the antigen-binding fragment is selected from the group consisting of an Fab, an Fab', an (Fab')₂, and an Fv.

8. Use of a composition according to any one of claims 1-7 in the manufacture of a medicament for inducing anti-tumor immunity in a subject.

9. Use of a composition according to any one of claims 1-7 in the manufacture of a medicament for treating a malignant condition in a subject.

10. The use of either claim 8 or claim 9 wherein the composition comprises (i) a first tumor cell that expresses a cell surface CD83 polypeptide, or a portion thereof; and (ii) a second tumor cell that expresses a cell surface form of an antibody, or antigen-binding fragment thereof, that binds specifically to CD137, and wherein the ability of each of the first and second tumor cells to divide in the subject is inhibited or substantially impaired.

11. The use of claim 10 wherein the antibody is a single chain Fv antibody.

12. The use of claim 10 wherein the antigen-binding fragment is selected from the group consisting of an Fab, an Fab', an (Fab')₂, and an Fv.

13. The use of claim 10 wherein the first and the second tumor cells are obtained from a biological sample obtained from the subject.

14. The use of either claim 8 or claim 9 wherein the composition comprises at least one tumor cell isolated from the subject, into which isolated tumor cell a composition according to at least one of claims 1(c), 1(d) and 4 has been introduced, wherein an ability of the at least one tumor cell to divide has been inhibited or substantially impaired.

15. The use of claim 9 wherein the malignant condition is selected from the group consisting of melanoma, carcinoma, sarcoma, lymphoma, and leukemia.

16. The use of claim 9 wherein the malignant condition is melanoma.

17. The use of either claim 8 or claim 9 wherein the composition further comprises an agent that inhibits an immunosuppressive effect of an immunosuppressive molecule.

18. The use of claim 17 wherein the immunosuppressive molecule is selected from the group consisting of a TGF-beta polypeptide, a CTLA4 polypeptide, a glucocorticoid-induced tumor necrosis factor receptor and Fas ligand.

19. The use of claim 17 wherein the agent that inhibits an immunosuppressive effect of an immunosuppressive molecule is selected from the group consisting of a chemotherapeutic agent, an antibody or antigen-binding fragment thereof that specifically binds to a TGF-beta polypeptide, an antibody or antigen-binding fragment thereof that specifically binds to a CTLA4 polypeptide, and an antibody or antigen-binding fragment thereof that specifically binds to a glucocorticoid-induced tumor necrosis factor receptor.

20. A recombinant expression construct comprising at least one first promoter operatively linked to a polynucleotide encoding a cell surface CD83 polypeptide, and at least one second promoter operatively linked to a polynucleotide that encodes a cell surface form of an antibody or antigen-binding fragment thereof that binds specifically to a CD 137 polypeptide.

21. The recombinant expression construct of claim 20 wherein the antibody is a single chain Fv antibody.

22. The recombinant expression construct of claim 20 wherein the antigen-binding fragment is selected from the group consisting of an Fab, an Fab', an (Fab')₂, and an Fv.

23. A host cell comprising the recombination expression construct according to any one of claims 20-22.

## Patentansprüche

1. Zusammensetzung zum Induzieren von Antitumor-Immunität, umfassend mindestens eine Zusammensetzung, die aus
(a) einer Zusammensetzung, umfassend eine Tumorzelle, die (i) ein Zell-oberflächen-CD83-Polypeptid oder einen Anteil davon und (ii) eine Zelloberflächenform eines Antikörpers oder eines Antigen-bindenden Fragments davon, der bzw. das spezifisch an CD137 bindet, exprimiert;
(b) einer Zusammensetzung, umfassend (i) eine erste Tumorzelle, die ein Zelloberflächen-CD83-Polypeptid oder einen Anteil davon exprimiert, und (ii) eine zweite Tumorzelle, die eine Zelloberflächenform eines Antikörpers oder eines Antigen-bindenden Fragments davon, der bzw. das spezifisch an CD137 bindet, exprimiert;
(c) einer Zusammensetzung, umfassend (i) ein rekombinantes Expressionskonstrukt, umfassend mindestens einen ersten Promotor, der mit einem Polynucleotid funktionell verbunden ist, das ein Zelloberflächen-CD83-Polypeptid kodiert, und mindestens einen zweiten Promotor, der mit einem Polynucleotid funktionell verbunden ist, das eine Zelloberflächenform eines Antikörpers oder eines Antigen-bindenden Fragments davon, der bzw. das spezifisch an ein CD137-Polypeptid bindet, kodiert, oder (ii) ein erstes rekombinantes Expressionskonstrukt, das mindestens einen ersten Promotor umfasst, der mit einem Polynucleotid funktionell verbunden ist, das ein Zelloberflächen-CD83-Polypeptid kodiert, und ein zweites rekombinantes Expressionskonstrukt, umfassend mindestens einen zweiten Promotor, der mit einem Polynucleotid funktionell verbunden ist, das eine Zelloberflächenform eines Antikörpers oder eines Antigen-bindenden Fragments davon kodiert, der bzw. das spezifisch an ein CD137-Polypeptid bindet, oder ein Anteil davon;
(d) einer Zusammensetzung, umfassend (i) ein erstes rekombinantes Expressionskonstrukt, das mindestens einen Promotor umfasst, der mit einem Polynucleotid funktionell verbunden ist, das ein Zelloberflächen-CD83-Polypeptid oder einen Anteil davon kodiert, und (ii) ein zweites rekombinantes Expressionskonstrukt, umfassend mindestens einen zweiten Promotor, der mit einem Polynucleotid funktionell verbunden ist, das mindestens ein Tumorantigen kodiert,
ausgewählt ist.

2. Zusammensetzung nach Anspruch 1 (a), wobei die Tumorzelle transfiziert ist mit (i) einem rekombinanten Expressionskonstrukt, umfassend mindestens einen ersten Promotor, der mit einem Polynucleotid funktionell verbunden ist, das ein Zelloberflächen-CD83-Polypeptid kodiert, und mindestens einen zweiten Promotor, der mit einem Polynucleotid funktionell verbunden ist, das eine Zelloberflächenform eines Antikörpers oder eines Antigen-bindenden Fragments davon, der bzw. das spezifisch an ein CD137-Polypeptid bindet, kodiert, oder (ii) einem ersten rekombinanten Expressionskonstrukt, das mindestens einen ersten Promotor umfasst, der mit einem Polynucleotid funktionell verbunden ist, das ein Zelloberflächen-CD83-Polypeptid kodiert, und einem zweiten rekombinanten Expressionskonstrukt, umfassend mindestens einen zweiten Promotor, der mit einem Polynucleotid funktionell verbunden ist, das eine Zelloberflächenform eines Antikörpers oder eines Antigen-bindenden Fragments davon, der bzw. das spezifisch an ein CD137-Polypeptid bindet, kodiert.

3. Zusammensetzung nach Anspruch 1 (b), wobei die erste Tumorzelle mit einem ersten rekombinanten Expressionskonstrukt transfiziert ist, umfassend mindestens einen ersten Promotor, der mit einem Polynucleotid funktionell verbunden ist, das ein Zelloberflächen-CD83-Polypeptid kodiert, und wobei die zweite Tumorzelle mit einem zweiten rekombinanten Expressionskonstrukt transfiziert ist, umfassend mindestens einen zweiten Promotor, der mit einem Polynucleotid funktionell verbunden ist, das eine Zelloberflächenform eines Antikörpers oder eines Antigen-bindenden Fragments davon, der bzw. das spezifisch an ein CD137-Polypeptid bindet, kodiert.

4. Zusammensetzung nach Anspruch 1 (c), umfassend einen rekombinanten Expressionsvektor, der mindestens einen mit einer Polynucleotidsequenz, die mindestens ein Tumorantigen kodiert, funktionell verbundenen Promotor umfasst.

5. Zusammensetzung nach entweder Anspruch 1(d) oder Anspruch 4, wobei das Tumorantigen von einer Tumorzelle in einer biologischen Probe, die von einem Patienten (subject) mit einem malignen Leiden erhalten wird, exprimiert wird.

6. Zusammensetzung nach einem der Ansprüche 1 - 5, wobei es sich bei dem Antikörper, der spezifisch an CD137 bindet, um einen einkettigen Fv-Antikörper handelt.

7. Zusammensetzung nach einem der Ansprüche 1 - 5, wobei das Antigen-bindende Fragment aus der Gruppe, bestehend aus einem Fab, einem Fab', einem (Fab')₂ und einem Fv, ausgewählt ist.

8. Verwendung einer Zusammensetzung gemäß einem der Ansprüche 1 - 7 bei der Herstellung eines Medikaments zum Induzieren von Antitumor-Immunität bei einem Patienten.

9. Verwendung einer Zusammensetzung gemäß einem der Ansprüche 1 - 7 bei der Herstellung eines Medikaments zum Behandeln eines malignen Leidens bei einem Patienten.

10. Verwendung nach entweder Anspruch 8 oder Anspruch 9, wobei die Zusammensetzung (i) eine erste Tumorzelle, die ein Zelloberflächen-CD83-Polypeptid oder einen Anteil davon exprimiert, und (ii) eine zweite Tumorzelle, die eine Zelloberflächenform eines Antikörpers oder eines Antigen-bindenden Fragments davon exprimiert, der bzw. das spezifisch an CD137 bindet, umfasst und wobei die Fähigkeit von sowohl der ersten als auch der zweiten Tumorzelle, sich in dem Patienten zu teilen, gehemmt oder wesentlich beeinträchtigt ist.

11. Verwendung nach Anspruch 10, wobei es sich bei dem Antikörper um einen einkettigen Fv-Antikörper handelt.

12. Verwendung nach Anspruch 10, wobei das Antigen-bindende Fragment aus der Gruppe, bestehend aus einem Fab, einem Fab', einem (Fab')₂ und einem Fv, ausgewählt ist.

13. Verwendung nach Anspruch 10, wobei die erste und die zweite Tumorzelle aus einer von dem Patienten erhaltenen biologischen Probe erhalten werden.

14. Verwendung nach entweder Anspruch 8 oder Anspruch 9, wobei die Zusammensetzung mindestens eine von dem Patienten isolierte Tumorzelle, in welche eine Zusammensetzung gemäß mindestens einem der Ansprüche 1 (c), 1 (d) und 4 eingeführt worden ist, umfasst, wobei eine Fähigkeit der mindestens einen Tumorzelle, sich zu teilen, gehemmt oder wesentlich beeinträchtigt worden ist.

15. Verwendung nach Anspruch 9, wobei das maligne Leiden aus der Gruppe, bestehend aus Melanom, Karzinom, Sarkom, Lymphom und Leukämie, ausgewählt ist.

16. Verwendung nach Anspruch 9, wobei es sich bei dem malignen Leiden um ein Melanom handelt.

17. Verwendung nach entweder Anspruch 8 oder Anspruch 9, wobei die Zusammensetzung ferner ein Mittel umfasst, das eine immunsuppressive Wirkung eines immunsuppressiven Moleküls hemmt.

18. Verwendung nach Anspruch 17, wobei das immunsuppressive Molekül aus der Gruppe, bestehend aus einem TGF-beta-Polypeptid, einem CTLA4-Polypeptid, einem Glucocorticoid-induzierten Tumornekrosefaktor-Rezeptor und einem Fas-Liganden, ausgewählt ist.

19. Verwendung nach Anspruch 17, wobei das Mittel, das eine immunsuppressive Wirkung eines immunsuppressiven Moleküls hemmt, aus der Gruppe, bestehend aus einem chemotherapeutischen Mittel, einem Antikörper oder einem Antigen-bindenden Fragment davon, der bzw. das spezifisch an ein TGF-beta-Polypeptid bindet, einem Antikörper oder einem Antigen-bindenden Fragment davon, der bzw. das spezifisch an ein CTLA4-Polypeptid bindet, und einem Antikörper oder einem Antigen-bindenden Fragment davon, der bzw. das spezifisch an einen Glucocorticoid-induzierten Tumornekrosefaktor-Rezeptor bindet, ausgewählt ist.

20. Rekombinantes Expressionskonstrukt, umfassend mindestens einen ersten Promotor, der mit einem Polynucleotid funktionell verbunden ist, das ein Zelloberflächen-CD83-Polypeptid kodiert, und mindestens einen zweiten Promotor, der mit einem Polynucleotid funktionell verbunden ist, das eine Zelloberflächenform eines Antikörpers oder eines Antigen-bindenden Fragments davon, der bzw. das spezifisch an ein CD137-Polypeptid bindet, kodiert.

21. Rekombinantes Expressionskonstrukt nach Anspruch 20, wobei es sich bei dem Antikörper um einen einkettigen Fv-Antikörper handelt.

22. Rekombinantes Expressionskonstrukt nach Anspruch 20, wobei das Antigen-bindende Fragment aus der Gruppe, bestehend aus einem Fab, einem Fab', einem (Fab')₂ und einem Fv, ausgewählt ist.

23. Wirtszelle, umfassend das Rekombinations-Expressionskonstrukt gemäß einem der Ansprüche 20 - 22.

## Revendications

1. Composition pour induire une immunité anti-tumorale comprenant au moins une composition qui est choisie parmi :
(a) une composition comprenant une cellule tumorale qui exprime (i) un polypeptide CD83 de surface cellulaire, ou une partie de celui-ci ; et (ii) une forme de surface cellulaire d'un anticorps, ou d'un fragment de celui-ci se liant à un antigène, qui se lie spécifiquement à CD137 ;
(b) une composition comprenant (i) une première cellule tumorale qui exprime un polypeptide CD83 de surface cellulaire, ou une partie de celui-ci ; et (ii) une deuxième cellule tumorale qui exprime une forme de surface cellulaire d'un anticorps, ou un fragment de celui-ci se liant à un antigène, qui se lie spécifiquement à CD137 ;
(c) une composition comprenant (i) une construction d'expression recombinante comprenant au moins un premier promoteur lié opérationnellement à un polynucléotide codant un polypeptide CD83 de surface cellulaire et au moins un deuxième promoteur lié opérationnellement à un polynucléotide qui code une forme de surface cellulaire d'un anticorps, ou un fragment de celui-ci se liant à un antigène, qui se lie spécifiquement à un polypeptide CD137 ou (ii) une première construction d'expression recombinante qui comprend au moins un premier promoteur lié opérationnellement à un polynucléotide codant un polypeptide CD83 de surface cellulaire et une deuxième construction d'expression recombinante comprenant au moins un deuxième promoteur lié opérationnellement à un polynucléotide codant une forme de surface cellulaire d'un anticorps, ou un fragment de celui-ci se liant à un antigène, qui se lie spécifiquement à un polypeptide CD137, ou à une partie de celui-ci ;
(d) une composition comprenant (i) une première construction d'expression recombinante qui comprend au moins un promoteur lié opérationnellement lié à un polynucléotide codant un polypeptide CD83 de surface cellulaire, ou une partie de celui-ci ; et (ii) une deuxième construction d'expression recombinante comprenant au moins un deuxième promoteur lié opérationnellement à un polynucléotide codant au moins un antigène tumoral.

2. Composition selon la revendication 1(a), dans laquelle la cellule tumorale est transfectée avec (i) une construction d'expression recombinante comprenant au moins un premier promoteur lié opérationnellement à un polynucléotide codant un polypeptide CD83 de surface cellulaire et au moins un deuxième promoteur lié opérationnellement à un polynucléotide qui code une forme de surface cellulaire d'un anticorps, ou un fragment de celui-ci se liant à un antigène, qui se lie spécifiquement à un polypeptide CD137 ou (ii) une première construction d'expression recombinante qui comprend au moins un premier promoteur lié opérationnellement à un polynucléotide codant un polypeptide CD83 de surface cellulaire et une deuxième construction d'expression recombinante comprenant au moins un deuxième promoteur lié opérationnellement à un polynucléotide codant une forme de surface cellulaire d'un anticorps, ou un fragment de celui-ci se liant à un antigène, qui se lie spécifiquement à un polypeptide CD137.

3. Composition selon la revendication 1 (b), dans laquelle la première cellule tumorale est transfectée avec une première construction d'expression recombinante comprenant au moins un premier promoteur lié opérationnellement à un polynucléotide codant un polypeptide CD83 de surface cellulaire, et dans laquelle la deuxième cellule tumorale est transfectée avec une deuxième construction d'expression recombinante comprenant au moins un deuxième promoteur lié opérationnellement à un polynucléotide codant une forme de surface cellulaire d'un anticorps, ou un fragment de celui-ci se liant à un antigène, qui se lie spécifiquement à un polypeptide CD137.

4. Composition selon la revendication 1(c) comprenant un vecteur d'expression recombinant qui comprend au moins un promoteur lié opérationnellement à une séquence polynucléotidique codant au moins un antigène tumoral.

5. Composition selon la revendication 1 (d) ou la revendication 4 dans laquelle l'antigène tumoral est exprimé par une cellule tumorale dans un échantillon biologique qui est obtenu chez un sujet présentant un état malin.

6. Composition selon l'une quelconque des revendications 1 à 5, dans laquelle l'anticorps qui se lie spécifiquement à CD137 est un anticorps Fv monocaténaire.

7. Composition selon l'une quelconque des revendications 1 à 5, dans laquelle le fragment se liant à un antigène est choisi dans le groupe constitué par un Fab, un Fab', un (Fab')₂ et un Fv.

8. Utilisation d'une composition selon l'une quelconque des revendications 1 à 7 dans la fabrication d'un médicament pour induire une immunité anti-tumorale chez un sujet.

9. Utilisation d'une composition selon l'une quelconque des revendications 1 à 7 dans la fabrication d'un médicament pour traiter un état malin chez un sujet.

10. Utilisation selon la revendication 8 ou la revendication 9, dans laquelle la composition comprend (i) une première cellule tumorale qui exprime un polypeptide CD83 de surface cellulaire, ou une partie de celui-ci; et (ii) une deuxième cellule tumorale qui exprime une forme de surface cellulaire d'un anticorps, ou un fragment de celui-ci se liant à un antigène, qui se lie spécifiquement à CD137, et dans laquelle la capacité de chacune des premières et deuxièmes cellules tumorales à se diviser chez le sujet est inhibée ou fortement diminuée.

11. Utilisation selon la revendication 10, dans laquelle l'anticorps est un anticorps Fv monocaténaire.

12. Utilisation selon la revendication 10, dans laquelle le fragment se liant à un antigène est choisi dans le groupe constitué par un Fab, un Fab', un (Fab')₂ et un Fv.

13. Utilisation selon la revendication 10, dans laquelle les premières et deuxièmes cellules tumorales proviennent d'un échantillon biologique obtenu du sujet.

14. Utilisation selon la revendication 8 ou la revendication 9, dans laquelle la composition comprend au moins une cellule tumorale isolée du sujet, dans laquelle cellule tumorale isolée une composition selon au moins une des revendications 1 (c), 1 (d) et 4 a été introduite, où une capacité de la au moins une cellule tumorale à se diviser a été inhibée ou fortement diminuée.

15. Utilisation selon la revendication 9, dans laquelle l'état malin est choisi dans le groupe constitué par les mélanomes, les carcinomes, les sarcomes, les lymphomes et la leucémie.

16. Utilisation selon la revendication 9, dans laquelle l'état malin est un mélanome.

17. Utilisation selon la revendication 8 ou la revendication 9, dans laquelle la composition comprend en outre un agent qui inhibe un effet immunosuppresseur d'une molécule immunosuppressive.

18. Utilisation selon la revendication 17, dans laquelle la molécule immunosuppressive est choisie dans le groupe constitué par un polypeptide TGF-béta, un polypeptide CTLA4, un récepteur de facteur de nécrose tumorale induite par un glucocorticoïde et le ligand Fas.

19. Utilisation selon la revendication 17, dans laquelle l'agent qui inhibe un effet immunosuppresseur d'une molécule immunosuppressive est choisi dans le groupe constitué par un agent chimio-thérapeutique, un anticorps ou un fragment de celui-ci se liant à un antigène, qui se lie spécifiquement à un polypeptide TGF-béta, un anticorps ou un fragment de celui-ci se liant à un antigène qui se lie spécifiquement à un polypeptide CTLA4, et un anticorps ou un fragment de celui-ci se liant à un antigène qui se lie spécifiquement à un récepteur du facteur de nécrose tumorale induite par un glucocorticoïde.

20. Construction d'expression recombinante comprenant au moins un premier promoteur lié opérationnellement à un polynucléotide codant un polypeptide CD83 de surface cellulaire et au moins un deuxième promoteur lié opérationnellement à un polynucléotide qui code une forme de surface cellulaire d'un anticorps ou un fragment de celui-ci se liant à un antigène qui se lie spécifiquement à un polypeptide CD137.

21. Construction d'expression recombinante selon la revendication 20, dans laquelle l'anticorps est un anticorps Fv monocaténaire.

22. Construction d'expression recombinante selon la revendication 20, dans laquelle le fragment se liant à un antigène est choisi dans le groupe constitué par un Fab, un Fab', un (Fab')₂ et un Fv.

23. Cellule hôte comprenant la construction d'expression de recombinaison selon l'une quelconque des revendications 20 à 22.
